# EUROPEAN PATENT APPLICATION

(11) **EP 4 364 796 A2**
(43) Date of publication of application: **08.05.2024**
(21) Application number: 24158250.1
(22) Date of filing: 14.03.2014
(51) Int. Cl.: A61P 3/10

(54) **USE OF LINAGLIPTIN IN CARDIO- AND RENOPROTECTIVE ANTIDIABETIC THERAPY**

(30) Priority: 15.03.2013 EP 13159624; 19.07.2013 EP 13177311
(62) Divisional of application: 20177745.5
(71) Applicant: Boehringer Ingelheim International GmbH, 55216 Ingelheim am Rhein (DE)
(72) Inventor: JOHANSEN, Odd-Erik, 55216 INGELHEIM AM RHEIN (DE); VON EYNATTEN, Maximilian, 55216 INGELHEIM AM RHEIN (DE); KLEIN, Thomas, 55216 INGELHEIM AM RHEIN (DE); WOERLE, Hans-Juergen, 55216 INGELHEIM AM RHEIN (DE)
(74) Representative: Lutze, Oliver

(57) **Abstract**

The present invention relates to a certain DPP-4 inhibitor for use in cardio- and/or renoprotective therapy, including in patients at high vascular risk.

## Description

### Field of the Invention

The present invention relates to a certain DPP-4 inhibitor (preferably linagliptin, optionally in combination with one or more other active agents) for use in therapy of diabetic (preferably type 2 diabetes) or non-diabetic patients (preferably for use in cardio- and/or renoprotective therapy of human type 2 diabetes patients), including patients with or at-risk of (micro- and/or macro-)vascular diseases, such as e.g. patients having or being at-risk of cardiovascular and/or (renal) microvascular diseases, such as e.g. patients at high vascular risk, as well as to pharmaceutical compositions and combinations comprising such active components, and to certain therapeutic uses thereof.

### Background of the Invention

Type 2 diabetes mellitus is a common chronic and progressive disease arising from a complex pathophysiology involving the dual endocrine effects of insulin resistance and impaired insulin secretion with the consequence not meeting the required demands to maintain plasma glucose levels in the normal range. This leads to chronic hyperglycaemia and its associated micro- and macrovascular complications or chronic damages, such as e.g. diabetic nephropathy, retinopathy or neuropathy, or macrovascular (e.g. cardio- or cerebrovascular) complications, and/or related cognitive function impairment. The vascular disease component plays a significant role, but is not the only factor in the spectrum of diabetes associated disorders. The high frequency of complications leads to a significant reduction of life expectancy. Diabetes is currently the most frequent cause of adult-onset loss of vision, renal failure, and amputation in the Industrialised World because of diabetes induced complications and is associated with a two to five fold increase in cardiovascular disease risk. The elevated risk for macrovascular disease is primarily related to increased risk for athero-thrombosis that leads to increased morbidity and premature mortality from cardiovascular (CV) disease and an important predictor for CV diseases is renal impairment, nephropathy and/or chronic kidney disease (CKD) which often co-exists.

The treatment of type 2 diabetes typically begins with diet and exercise, followed by oral antidiabetic monotherapy, and although conventional monotherapy may initially control blood glucose in some patients, it is however associated with a high secondary failure rate. The limitations of single-agent therapy for maintaining glycemic control may be overcome, at least in some patients, and for a limited period of time by combining multiple drugs to achieve reductions in blood glucose that cannot be sustained during long-term therapy with single agents. Available data support the conclusion that in most patients with type 2 diabetes current monotherapy will fail and treatment with multiple drugs will be required.

But, because type 2 diabetes is a progressive disease, even patients with good initial responses to conventional combination therapy will eventually require an increase of the dosage or further treatment with an additional oral or non-oral antidiabetic drug (often finally with insulin therapy) because the blood glucose level is very difficult to maintain stable for a long period of time. Although existing combination therapy has the potential to enhance glycemic control, it is not without limitations (especially with regard to long term efficacy). Further, traditional therapies may show an increased risk for side effects, such as hypoglycemia or weight gain, which may compromise their efficacy and acceptability.

Thus, for many patients, these existing drug therapies result in progressive deterioration in metabolic control despite treatment and do not sufficiently control metabolic status especially over long-term and thus fail to achieve and to maintain glycemic control in advanced, progressed or late stage type 2 diabetes, including diabetes with inadequate glycemic control despite conventional oral and/or non-oral antidiabetic medication.

Therefore, although intensive treatment of hyperglycemia can reduce the incidence of chronic damages, many patients with diabetes remain inadequately treated, partly because of limitations in long term efficacy, safety/tolerability and dosing inconvenience of conventional antihyperglycemic therapies.

In addition, obesity, overweight or weight gain (e.g. as side or adverse effect of some conventional antidiabetic medications) further complicates the treatment of diabetes and its microvascular or macrovascular, and/or related cognitive, complications.

This high incidence of therapeutic failure is a major contributor to the high rate of long-term hyperglycemia-associated complications or chronic damages (including micro- and makrovascular complications such as e.g. diabetic nephrophathy, retinopathy or neuropathy, or cerebro- or cardiovascular complications such as e.g. myocardial infarction, stroke or vascular mortality or morbidity) in patients with diabetes.

Oral antidiabetic drugs conventionally used in therapy (such as e.g. first-, second- or third-line, and/or mono- or (initial or add-on) combination therapy) may include, without being restricted thereto, metformin, sulphonylureas, thiazolidinediones, glinides and α-glucosidase inhibitors.

Non-oral (typically injected) antidiabetic drugs conventionally used in therapy (such as e.g. first-, second- or third-line, and/or mono- or (initial or add-on) combination therapy) may include, without being restricted thereto, GLP-1 or GLP-1 analogues, and insulin or insulin analogues.

However, the use of these conventional antidiabetic or antihyperglycemic agents can be associated with various adverse effects. For example, metformin can be associated with lactic acidosis or gastrointestinal side effects; sulfonylureas, glinides and insulin or insulin analogues can be associated with hypoglycemia and weight gain; thiazolidinediones can be associated with edema, bone fracture, weight gain and heart failure/cardiac effects; and alpha-glucosidase blockers and GLP-1 or GLP-1 analogues can be associated with gastrointestinal adverse effects (e.g. dyspepsia, flatulence or diarrhea, or nausea or vomiting).

In addition to morbidity associated with each of these side effects, they could also have adverse cardiovascular implications. For example, hypoglycaemia and weight gain are postulated as contributors to adverse CV mortality outcomes.

Hypoglycemic episodes have also been identified detrimental to cognitive skills and are associated with a greater risk of cognitive impairment or dementia. The risk of hypoglycemia is further increased in the elderly with comorbidities and multiple medication use.

Therefore, it remains a need in the art to provide efficacious, safe and tolerable antidiabetic therapies.

Further, within the therapy of type 2 diabetes, it is a need for treating the condition effectively, avoiding the (micro- and/or macrovascular) complications inherent to the condition, and delaying or modifying disease progression, e.g. in order to achieve a long-lasting therapeutic benefit.

Furthermore, it remains a need that antidiabetic treatments not only prevent and/or treat the long-term complications often found in advanced stages of diabetes disease, but also are a therapeutic option in those diabetes patients who have developed or are at-risk of developing such complications (e.g. renal impairment).

In particular, there is a need that antidiabetic treatments prevent and/or treat preferably both microvascular (renal) complications and macrovascular (CV) complications together, preferably within one therapy.

Further in particular, there is also a need to provide a therapeutic option in those diabetes patients who have developed or are at-risk of developing both microvascular (renal) complications and macrovascular (CV) complications.

Also, there is a need that antidiabetic treatments prevent and/or treat accelerated cognitive decline (which may be associated with micro- and/or macrovascular complications), preferably together with both microvascular (renal) complications and macrovascular (CV) complications, preferably within one therapy.

Moreover, it remains a need to provide prevention or reduction of risk for adverse effects associated with conventional antidiabetic therapies.

### Summary of the Invention

The present invention relates to a certain DPP-4 inhibitor (preferably linagliptin, optionally in combination with one or more other active agents) for use in therapy of diabetic (preferably type 2 diabetes) or non-diabetic patients (such as e.g. for decreasing, preventing, protecting against, delaying the onset, slowing progression and/or reducing the risk of cardiovascular and/or renal morbidity and/or mortality in human, diabetic or non-diabetic patients, preferably for use in cardio- and/or renoprotective therapy of human type 2 diabetes patients), e.g. including in patients with or at-risk of (micro- and/or macro-)vascular diseases, such as e.g. patients having or being at-risk of cardiovascular and/or (renal) microvascular diseases, such as e.g. patients at high vascular risk, as well as to pharmaceutical compositions and combinations comprising such active components, and to certain therapeutic uses thereof.

Such therapy according to this invention (e.g. as described hereinabove or hereinbelow in further detail) may include treatment with such certain DPP-4 inhibitor (preferably linagliptin, optionally in combination with one or more other active agents) over a lengthy period, such as described in more detail (duration of treatment) hereinbelow.

The present invention relates to a certain DPP-4 inhibitor (preferably linagliptin, optionally in combination with one or more other active agents) for use in therapy of diabetic (preferably type 2 diabetes) or non-diabetic patients (preferably for use in cardioprotective as well as renoprotective therapy of human type 2 diabetes patients, preferably beyond and/or independently from improving glycemic control), including patients with or at-risk of (microand/or macro-)vascular diseases, such as e.g. patients having or being at-risk of cardiovascular and/or renal microvascular diseases, such as e.g. patients at high vascular risk, as well as to pharmaceutical compositions and combinations comprising such active components, and to certain therapeutic uses thereof.

The present invention relates to a certain DPP-4 inhibitor (preferably linagliptin, optionally in combination with one or more other active agents) for use in patients (preferably diabetic, particularly type 2 diabetes patients) with or at-risk of cardiovascular and/or renal microvascular diseases, such as e.g. patients at high vascular risk, e.g. as described herein; such as e.g. in patients according to at least one of the embodiments 1 to 6 or 1 to 7 as described herein below.

The present invention relates to a certain DPP-4 inhibitor (preferably linagliptin, optionally in combination with one or more other active agents) for use in decreasing, preventing, protecting against, delaying and/or reducing the risk of morbidity and /or premature mortality from vascular disease, such as e.g. cardiovascular (CV) and/or renal microvascular disease (in an embodiment, such vascular disease may further include cognitive decline or impairment), preferably in diabetic (particularly type 2 diabetes patients), such as e.g. in patients having or being at high risk of cardiovascular and/or renal microvascular disease, such as e.g. in patients at high vascular risk (such as e.g. those patients described herein); such as e.g. in those patients according to at least one of the embodiments 1 to 6 or 1 to 7 as described herein below.

Accordingly, the present invention also relates to a certain DPP-4 inhibitor (preferably linagliptin, optionally in combination with one or more other active agents) for use in decreasing, preventing, protecting against, delaying (e.g. occurrence or progression) and/or reducing the risk of any or all of the following: CV morbidity, premature CV mortality, renal morbidity and/or premature renal mortality; preferably in diabetic (particularly type 2 diabetes patients), such as e.g. in patients having or being at high risk of cardiovascular and/or renal microvascular disease, such as e.g. in patients at high vascular risk (such as e.g. those patients described herein); such as e.g. in those patients according to at least one of the embodiments 1 to 6 or 1 to 7 as described herein below.

In certain embodiments, the therapies according to the present invention (e.g. such as described hereinabove or hereinbelow, e.g. the cardio- and/or renoprotective therapy of human type 2 diabetes patients, such as e.g. patients having or being at high risk of cardiovascular and/or renal microvascular disease, such as e.g. patients at high vascular risk (such as e.g. those patients described herein); such as e.g. those patients according to at least one of the embodiments 1 to 6 or 1 to 7 as described herein below) may include duration of treatment with a certain DPP-4 inhibitor, particularly linagliptin (preferably 5 mg per day, administered orally, optionally in combination with one or more other active substances, e.g. such as those described herein) over a lengthy period, such as e.g. at least 1-6 years, >/= 2 years, or 3-7 years such as 3-4 years, 3-5 years, 3-6 years, 4-5 years, 4-6 years, 5-6 years or 5-7 years, preferably at least 48 months, more preferably at least 3 years.

For example, duration of treatment with a certain DPP-4 inhibitor, particularly linagliptin (preferably 5 mg per day, administered orally, optionally in combination with one or more other active substances, e.g. such as those described herein) may be over a lengthy period, such as e.g. at least 1-6 years, >/= 2 years, or 3-7 years such as 3-4 years, 3-5 years, 3-6 years, 4-5 years, 4-6 years, 5-6 years or 5-7 years, preferably at least 48 months, more preferably at least 3 years, such as to optimize cardio- and/or renoprotective therapy and/or to improve cardiovascular and/or renal morbidity and/or mortality in a human patient, particularly human type 2 diabetes patient, such as e.g. a patient having or being at high risk of cardiovascular and/or renal microvascular disease, such as e.g. a patient at high vascular risk (such as described herein); such as e.g. a patient according to at least one of the embodiments 1 to 6 or 1 to 7 as described herein below.

For more detailed example, duration of treatment with a certain DPP-4 inhibitor, particularly linagliptin (preferably 5 mg per day, administered orally, optionally in combination with one or more other active substances, e.g. such as those described herein) may be over a lengthy period, preferably at least 48 months, more preferably at least 3 years (such as e.g. at least 3-4 years, or at least 5-6 years), such as e.g. in a patient (such as diabetes, particularly type 2 diabetes patient) having or being at high risk of cardiovascular and/or renal microvascular disease, such as e.g. a patient at high vascular risk (such as described herein); such as e.g. a patient according to at least one of the embodiments 1 to 6 or 1 to 7 as described herein below.

In an embodiment, the present invention relates to a certain DPP-4 inhibitor (preferably linagliptin, optionally in combination with one or more other active agents) for use in decreasing, preventing, protecting against, delaying (e.g. occurrence or progression) and/or reducing the risk of: CV morbidity and/or (premature) CV mortality, such as e.g. one or more selected from: CV death (e.g. fatal myocardial infarction, fatal stroke, fatal heart failure, cardiogenic shock, or sudden cardiac death), non-fatal myocardial infarction, non-fatal stroke (e.g. (intracranial) hemorrhagic or non-hemorrhagic stroke, and/or silent or non-silent) and/or unstable angina pectoris (e.g. hospitalization for unstable angina pectoris), and/or, optionally, stable angina pectoris, transient ischemic attack, congestive heart failure, peripheral revascularization procedures and/or coronary revascularization procedures (such as e.g. hospitalization for any of such morbidity); preferably in diabetic (particularly type 2 diabetes patients), such as e.g. in patients having or being at high risk of cardiovascular and/or renal microvascular disease, such as e.g. in patients at high vascular risk (such as e.g. those patients described herein); such as e.g. in those patients according to at least one of the embodiments 1 to 7 as described herein below.

In an embodiment, the present invention relates to a certain DPP-4 inhibitor (preferably linagliptin, optionally in combination with one or more other active agents) for use in decreasing, preventing, protecting against, delaying (e.g. occurrence or progression) and/or reducing the risk of: morbidity (such as e.g. renal morbidity) and/or (premature) renal mortality, such as e.g. one or more selected from: retinopathy, nephropathy, neuropathy, cognitive decline, dementia, depressive, mood or anxiety disorders, microalbuminuria, macroalbuminuria, chronic kidney disease (CKD), renal impairment, renal death, end-stage renal disease and/or loss in estimated glomerular filtration rate (e.g. eGFR ≥ 50% from baseline); preferably in diabetic (particularly type 2 diabetes patients), such as e.g. in patients having or being at high risk of cardiovascular and/or renal microvascular disease, such as e.g. in patients at high vascular risk (such as e.g. those patients described herein); such as e.g. in those patients according to at least one of the embodiments 1 to 7 as described herein below.

In an embodiment, the present invention relates to a certain DPP-4 inhibitor (preferably linagliptin, optionally in combination with one or more other active agents)
for use in decreasing, preventing, protecting against, delaying (e.g. occurrence or progression) and/or reducing the risk of: CV morbidity and/or (premature) CV mortality, such as e.g. one or more selected from: CV death (e.g. fatal myocardial infarction, fatal stroke, fatal heart failure, cardiogenic shock, or sudden cardiac death), non-fatal myocardial infarction, non-fatal stroke (e.g. (intracranial) hemorrhagic or non-hemorrhagic stroke, and/or silent or non-silent) and/or unstable angina pectoris (e.g. hospitalization for unstable angina pectoris), and/or, optionally, stable angina pectoris, transient ischemic attack, congestive heart failure, peripheral revascularization procedures and/or coronary revascularization procedures (such as e.g. hospitalization for any of such morbidity);
   and/or
for use in decreasing, preventing, protecting against, delaying (e.g. occurrence or progression) and/or reducing the risk of: morbidity (such as e.g. renal morbidity) and/or (premature) renal mortality, such as e.g. one or more selected from: retinopathy, nephropathy, neuropathy, cognitive decline, dementia, depressive, mood or anxiety disorders, microalbuminuria, macroalbuminuria, chronic kidney disease (CKD), renal impairment, renal death, end-stage renal disease and/or loss in estimated glomerular filtration rate (e.g. eGFR ≥ 50% from baseline);
preferably in diabetic (particularly type 2 diabetes patients), such as e.g. in patients having or being at high risk of cardiovascular and/or renal microvascular disease, such as e.g. in patients at high vascular risk (such as e.g. those patients described herein); such as e.g. in those patients according to at least one of the embodiments 1 to 7 as described herein below.

The present invention also relates to a certain DPP-4 inhibitor (preferably linagliptin, optionally in combination with one or more other active agents) for use in decreasing, preventing, protecting against, delaying (e.g. occurrence or progression) and/or reducing the risk of at least one (preferably at least two, more preferably at least three, even more preferably at least four) selected from: CV morbidity, premature CV mortality, renal morbidity and premature renal mortality; preferably in diabetic (particularly type 2 diabetes patients), such as e.g. in patients having or being at high risk of cardiovascular and/or renal microvascular disease, such as e.g. in patients at high vascular risk (such as e.g. those patients described herein); such as e.g. in those patients according to at least one of the embodiments 1 to 6 or 1 to 7 as described herein below.

The present invention further relates to a certain DPP-4 inhibitor (preferably linagliptin, optionally in combination with one or more other active agents) for use in treating, protecting against, preventing, reducing the risk of and/or delaying the onset or progression of both macrovascular (such as cardiovascular (CV)) complications and renal microvascular complications (such as nephropathy), preferably in diabetic (particularly type 2 diabetes patients), such as e.g. in patients having or being at high risk of cardiovascular and/or renal microvascular disease, such as e.g. in patients at high vascular risk (such as e.g. those patients described herein); such as e.g. in those patients according to at least one of the embodiments 1 to 6 or 1 to 7 as described herein below.

The present invention further relates to a certain DPP-4 inhibitor (preferably linagliptin, optionally in combination with one or more other active agents) for use in:
treating, protecting against, preventing, reducing the risk of and/or delaying the onset or progression of a macrovascular (such as cardiovascular (CV)) complication
   and/or
treating, protecting against, preventing, reducing the risk of and/or delaying the onset or progression of a renal microvascular complication (such as nephropathy);
preferably in diabetic (particularly type 2 diabetes patients), such as e.g. in patients having or being at high risk of cardiovascular and/or renal microvascular disease, such as e.g. in patients at high vascular risk (such as e.g. those patients described herein); such as e.g. in those patients according to at least one of the embodiments 1 to 6 or 1 to 7 as described herein below.

The present invention further relates to a certain DPP-4 inhibitor (preferably linagliptin, optionally in combination with one or more other active agents) for use in a method of preventing, protecting against, reducing the risk of and/or delaying the occurrence of:
a cardio- or cerebrovascular disease or event, such as e.g. selected from cardiovascular (CV) death (including fatal stroke, fatal myocardial infarction and sudden death), non-fatal stroke and non-fatal myocardial infarction (MI) (with or without silent MI) and, optionally, hospitalisation (e.g. for unstable angina pectoris, coronary revascularization procedures, peripheral revascularization or congestive heart failure),
   and/or
preventing, protecting against, reducing the risk of, delaying the progression of and/or
delaying the occurrence of:
   a (renal) microvascular disease, such as e.g. selected from retinopathy, albuminuria (micro or macro), chronic kidney disease (CKD), renal impairment, renal death, end-stage renal disease and loss in estimated glomerular filtration rate (e.g. eGFR ≥ 50% from baseline);
   preferably in a human diabetic (particularly type 2 diabetes patient);
   such as e.g. in a patient having or being at high risk of a cardiovascular event and/or renal microvascular disease, such as e.g. in a patient at high vascular risk (e.g. at high risk of CV events), such as e.g. a patient as described herein); such as e.g. in a patient according to at least one of the embodiments 1 to 6 or 1 to 7 as described herein below.

The present invention further relates to a certain DPP-4 inhibitor (preferably linagliptin, optionally in combination with one or more other active agents) for use in a method of preventing, reducing the risk of and/or delaying the occurrence of:
a cardio- or cerebrovascular disease or (severe) event, such as e.g. selected from cardiovascular (CV) death (including fatal stroke, fatal myocardial infarction and sudden death), non-fatal stroke, non-fatal myocardial infarction (MI) (silent MI may be excluded) and hospitalisation (optional) for unstable angina pectoris,
   and/or
a renal microvascular disease or event, such as e.g. selected from renal death, end-stage renal disease and loss in estimated glomerular filtration rate (e.g. eGFR ≥ 50% from baseline);
preferably in a human diabetic (particularly type 2 diabetes patient); such as e.g. in a patient having or being at high risk of a cardiovascular event and/or renal microvascular disease, such as e.g. in a patient at high vascular risk (e.g. at high risk of CV events), such as e.g. a patient as described herein); such as e.g. in a patient according to at least one of the embodiments 1 to 6 or 1 to 7 as described herein below.

The present invention further relates to a certain DPP-4 inhibitor (preferably linagliptin, optionally in combination with one or more other active agents) for use in a (combined) method of:
preventing, reducing the risk of and/or delaying the occurrence of:
a cardio- or cerebrovascular disease or (severe) event, such as e.g. selected from cardiovascular (CV) death (including fatal stroke, fatal myocardial infarction and sudden death), non-fatal stroke, non-fatal myocardial infarction (MI) (silent MI may be excluded) and hospitalisation (optional) for unstable angina pectoris,
   and
preventing, reducing the risk of and/or delaying the occurrence of:
   a renal microvascular disease or event, such as e.g. selected from renal death, end-stage renal disease and loss in estimated glomerular filtration rate (e.g. eGFR ≥ 50% from baseline);
   preferably in a human diabetic (particularly type 2 diabetes patient); such as e.g. in a patient having or being at high risk of a cardiovascular event and/or renal microvascular disease, such as e.g. in a patient at high vascular risk (e.g. at high risk of CV events), such as e.g. a patient as described herein); such as e.g. in a patient according to at least one of the embodiments 1 to 6 or 1 to 7 as described herein below.

The present invention further relates to a certain DPP-4 inhibitor (preferably linagliptin, optionally in combination with one or more other active agents) for use in a (combined) method of:
preventing a cardio- or cerebrovascular disease or (severe) event, such as e.g. selected from cardiovascular (CV) death (including fatal stroke, fatal myocardial infarction and sudden death), non-fatal stroke, non-fatal myocardial infarction (MI) (silent MI may be excluded) and hospitalisation (optional) for unstable angina pectoris,
   and
preventing a renal microvascular disease or event, such as e.g. selected from renal death, end-stage renal disease and loss in estimated glomerular filtration rate (e.g. eGFR ≥ 50% from baseline);
preferably in a human diabetic (particularly type 2 diabetes patient); such as e.g. in a patient having or being at high risk of a cardiovascular event and/or renal microvascular disease, such as e.g. in a patient at high vascular risk (e.g. at high risk of CV events), such as e.g. a patient as described herein); such as e.g. in a patient according to at least one of the embodiments 1 to 6 or 1 to 7 as described herein below.

Further, the present invention relates to a certain DPP-4 inhibitor (preferably linagliptin, optionally in combination with one or more other active agents) for use in decreasing, preventing, protecting against, delaying (e.g. occurrence or progression) and/or reducing the risk of (accelerated) cognitive decline or impairment or dementia; preferably in diabetic (particularly type 2 diabetes patients), such as e.g. in patients having or being at high risk of cardiovascular and/or renal microvascular disease, such as e.g. in patients at high vascular risk (such as e.g. those patients described herein); such as e.g. in those patients according to at least one of the embodiments 1 to 6 or 1 to 7 as described herein below.

Such a patient having or being at-risk of a cardio- or cerebrovascular and/or renal (micro)vascular disease, event, complication or condition, such as e.g. a patient at high vascular risk (e.g. at high risk of CV events), of this invention, who may be amenable to the therapy (treatment and/or prevention) of this invention, may be or include a patient (preferably diabetic, particularly type 2 diabetes patients) as described herein below (such as e.g. in the following embodiments 1 to 6 or 1 to 7).

For example (embodiment 1), such a patient having or being at-risk of a cardio- or cerebrovascular and/or renal (micro)vascular disease, event, complication or condition according to the present invention may be a patient (preferably diabetic, particularly type 2 diabetes patients) having a microvascular disease (e.g. retinopathy, neuropathy, or renal microvascular disease such as nephropathy, albuminuria (e.g. micro- or macro-albuminuria), proteinuria, chronic kidney disease (e.g. CKD stage 1, 2, 3, 4 or 5) and/or renal impairment (e.g. mild, moderate or severe renal impairment or end-stage renal disease (ESRD)), and/or a (previous) macrovascular (e.g. cardio- or cerebrovascular) disease (such as e.g. myocardial infarction, coronary artery disease, (ischemic or haemorrhagic) stroke, carotid artery disease and/or peripheral artery disease).

For further example (embodiment 2), such a patient having or being at-risk of a cardio- or cerebrovascular and/or (renal) (micro)vascular disease, event, complication or condition according to the present invention may be a patient (preferably diabetic, particularly type 2 diabetes patients) having nephropathy or chronic kidney disease (e.g. CKD stage 1, 2, 3, 4 or 5, particularly CKD 3 to 5), including e.g. albuminuria (e.g. micro- or macro-albuminuria, proteinuria, particularly with macro-albuminuria) and/or renal impairment (e.g. mild, moderate or severe renal impairment or end-stage renal disease (ESRD), particularly of moderate, severe or ESRD stage and/or particularly with albuminuria, more particularly with macroalbuminuria); with or without a (previous) macrovascular (e.g. cardio- or cerebrovascular) disease (such as e.g. myocardial infarction, coronary artery disease, (ischemic or haemorrhagic) stroke, carotid artery disease and/or peripheral artery disease).

For particular example (embodiment 3), such a patient having or being at high risk of a cardiovascular and/or renal microvascular disease (e.g. such a patient at high vascular risk, e.g. at high risk of CV events) amenable to the therapy (e.g. treatment and/or prevention and/or protection) of this invention may be a patient (preferably diabetic, particularly type 2 diabetes patients) having:
both
albuminuria (e.g. micro- or macro-albuminuria)
   and
previous macrovascular (e.g. cardio- or cerebrovascular) disease (such as e.g. myocardial infarction, coronary artery disease, (ischemic or haemorrhagic) stroke, carotid artery disease and/or peripheral artery disease),
   and/or
either
(mild or moderate) renal impairment (e.g. CKD stage 1, 2 or 3, such as CKD stage 1, 2 (mild) or 3a (mild-moderate), preferably eGFR ≥ 45-75 mL/min/1.73 m²) with macro-albuminuria, or
(moderate or severe) renal impairment (e.g. CKD stage 3 or 4, such as CKD stage 3b (moderate-severe) or 4 (severe), preferably eGFR 15-45 mL/min/1.73 m²), with or without any albuminuria (such as e.g. with or without micro- or macro-albuminuria).

In more detail (embodiment 4), such a patient at high vascular risk (e.g. at high risk of CV events) may be a patient (preferably diabetic, particularly type 2 diabetes patients) as follows:
with
albuminuria (such as e.g. urine albumin creatinine ratio (UACR) ≥ 30 mg/g creatinine or ≥ 30 mg/l (milligram albumin per liter of urine) or ≥ 30 µg/min (microgram albumin per minute) or ≥ 30 mg/24 h (milligram albumin per 24 hours)) and
previous macrovascular disease, such as e.g. defined as one or more of a) to f):
   a) previous myocardial infarction,
   b) advanced coronary artery disease,
   c) high-risk single-vessel coronary artery disease,
   d) previous ischemic or haemorrhagic stroke,
   e) presence of carotid artery disease,
   f) presence of peripheral artery disease,
   and/or
   with
impaired renal function (e.g. with or without CV co-morbidities), such as e.g. defined by:
   - impaired renal function (e.g. as defined by MDRD formula) with an eGFR 15-45 mL/min/1.73 m² with any urine albumin creatinine ratio (UACR), and/or
   - impaired renal function (e.g. as defined by MDRD formula) with an eGFR ≥ 45-75 mL/min/1.73 m² with an urine albumin creatinine ratio (UACR) > 200 mg/g creatinine or > 200 mg/l (milligram albumin per liter of urine) or > 200 µg/min (microgram albumin per minute) or > 200 mg/24 h (milligram albumin per 24 hours).

In further more detail, such a patient at high vascular risk (e.g. at high risk of CV events) may be a patient (preferably diabetic, particularly type 2 diabetes patients) with the Condition I (embodiment 5) and/or with the Condition II (embodiment 6), each as defined hereinbelow.

In another embodiment (embodiment 7), such a patient at high vascular risk (e.g. at high risk of CV events) may be a patient (preferably diabetic, particularly type 2 diabetes patients) with one or more of the following CV risk factors A), B), C) and/or D):
A) Previous vascular disease, such as e.g.:
   - myocardial infarction (e.g. within previous 6 weeks),
   - coronary artery disease (e.g. >=50% luminal diameter narrowing of left main coronary artery or in at least two major coronary arteries in angiogram),
   - percutaneous coronary intervention (e.g. within previous 6 weeks),
   - coronary artery by-pass grafting (e.g. within previous 4 years or with recurrent angina following surgery),
   - ischemic or hemorrhagic stroke (e.g. within previous 3 months),
   - peripheral occlusive arterial disease (e.g. previous limb bypass surgery or percutaneous transluminal angioplasty; previous limb or foot amputation due to circulatory insufficiency, angiographic or ultrasound detected significant vessel stenosis (>50%) of major limb arteries (common iliac artery, internal iliac artery, external iliac artery, femoral artery, popliteal artery), history of intermittent claudication, with an ankle:arm blood pressure ratio <0.90 on at least one side),
B) Vascular related end-organ damage, such as e.g.:
   - impaired renal function (e.g. moderately impaired renal function e.g. as defined by MDRD formula, with eGFRF 30-59 mL/min/1.73m2),
   - micro- or macroalbuminuria (e.g. microalbuminuria, or random spot urinary albumin:creatinine ratio >/= 30 µg/mg),
   - retinopathy (e.g. proliferative retinopathy, or retinal neovascularisation or previous retinal laser coagulation therapy),
C) Elderly (e.g. age >/= 70 years),
D) At least two of the following CV risk factors:
   - advanced type 2 diabetes mellitus (e.g. > 10 years duration),
   - hypertension (e.g. systolic blood pressure >140 mmHg or on at least one blood pressure lowering treatment),
   - current daily cigarette smoking,
   - (atherogenic) dyslipidemia or high LDL cholesterol blood levels (e.g. LDL cholesterol >/=135 mg/dL) or on at least one treatment for lipid abnormality.

The present invention further relates to a certain DPP-4 inhibitor (preferably linagliptin, optionally in combination with one or more other active agents) for use in a method of preventing, protecting against, reducing the risk of and/or delaying the occurrence of:
a cardio- or cerebrovascular disease or event, such as e.g. selected from cardiovascular (CV) death (including fatal stroke, fatal myocardial infarction and sudden death), non-fatal stroke, non-fatal myocardial infarction (MI) (silent MI may be excluded) and hospitalisation for unstable angina pectoris,
   and
a renal microvascular disease, such as e.g. selected from renal death, end-stage renal disease and loss in estimated glomerular filtration rate (e.g. eGFR ≥ 50% from baseline); preferably in a human diabetic (particularly type 2 diabetes patient); such as e.g. in a patient having or being at high-risk of a cardiovascular event and/or renal microvascular disease, such as e.g. in a patient at high vascular risk (e.g. at high risk of CV events), such as in a patient with:
   both
   albuminuria (e.g. micro- or macro-albuminuria)
      and
   previous macrovascular (e.g. cardio- or cerebrovascular) disease (such as e.g. myocardial infarction, coronary artery disease, (ischemic or haemorrhagic) stroke, carotid artery disease and/or peripheral artery disease),
      and/or
      either
   (mild or moderate) renal impairment (e.g. CKD stage 2 or 3, preferably eGFR ≥ 45-75 mL/min/1.73 m²) with macro-albuminuria
      or
   (moderate or severe) renal impairment (e.g. CKD stage 3 or 4, preferably eGFR 15-45 mL/min/1.73 m²) (with or without any albuminuria).

The present invention further relates to a certain DPP-4 inhibitor (preferably linagliptin, optionally in combination with one or more other active agents) for use in a method of preventing, reducing the risk of and/or delaying the occurrence of:
a cardio- or cerebrovascular disease or event, such as e.g. selected from cardiovascular (CV) death (including fatal stroke, fatal myocardial infarction and sudden death), non-fatal stroke, non-fatal myocardial infarction (MI) (silent MI may be excluded) and hospitalisation for unstable angina pectoris,
   and
a renal microvascular disease, such as e.g. selected from renal death, end-stage renal disease and loss in estimated glomerular filtration rate (e.g. eGFR ≥ 50% from baseline); preferably in a human diabetic (particularly type 2 diabetes patient), such as e.g. in a patient with or at high risk of a cardiovascular event and/or renal microvascular disease, such as e.g. in a patient at high vascular risk (e.g. at high risk of CV events), such as in a patient having: albuminuria (such as e.g. urine albumin creatinine ratio (UACR) ≥ 30 mg/g creatinine or ≥ 30 mg/l (milligram albumin per liter of urine) or ≥ 30 µg/min (microgram albumin per minute) or ≥ 30 mg/24 h (milligram albumin per 24 hours)) and
previous macrovascular disease, such as e.g. defined as one or more of a) to f):
   a) previous myocardial infarction,
   b) advanced coronary artery disease,
   c) high-risk single-vessel coronary artery disease,
   d) previous ischemic or haemorrhagic stroke,
   e) presence of carotid artery disease,
   f) presence of peripheral artery disease,
   and/or
impaired renal function (e.g. with or without CV co-morbidities), such as e.g. defined by:
   - impaired renal function (e.g. as defined by MDRD formula) with an eGFR 15-45 mL/min/1.73 m² with any urine albumin creatinine ratio (UACR), and/or
   - impaired renal function (e.g. as defined by MDRD formula) with an eGFR ≥ 45-75 mL/min/1.73 m² with an urine albumin creatinine ratio (UACR) > 200 mg/g creatinine or > 200 mg/l (milligram albumin per liter of urine) or > 200 µg/min (microgram albumin per minute) or > 200 mg/24 h (milligram albumin per 24 hours).

Accordingly, the present invention also relates to a certain DPP-4 inhibitor (preferably linagliptin, optionally in combination with one or more other active agents) for use in a method of
preventing, reducing the risk of and/or delaying the occurrence of:
a cardio- or cerebrovascular disease or event, such as e.g. selected from cardiovascular (CV) death (including fatal stroke, fatal myocardial infarction and sudden death), non-fatal stroke, non-fatal myocardial infarction (MI) (silent MI may be excluded) and hospitalisation for unstable angina pectoris,
   and
a renal microvascular disease, such as e.g. selected from renal death, end-stage renal disease and loss in estimated glomerular filtration rate (e.g. eGFR ≥ 50% from baseline); preferably in a human diabetic (particularly type 2 diabetes patient), such as e.g. in a patient having or being at high-risk of a cardiovascular event and/or renal microvascular disease, such as e.g. in a patient at high vascular risk (e.g. at high risk of CV events), such as in a patient with the Condition I and/or II as defined as follows:
   Condition I:
      albuminuria (such as e.g. urine albumin creatinine ratio (UACR) ≥ 30 mg/g creatinine or ≥ 30 mg/l (milligram albumin per liter of urine) or ≥ 30 µg/min (microgram albumin per minute) or ≥ 30 mg/24 h (milligram albumin per 24 hours)) and
      previous macrovascular disease, such as e.g. defined as one or more of a) to f):
         a) previous myocardial infarction (e.g. > 2 months),
         b) advanced coronary artery disease, such as e.g. defined by any one of the following:
            - ≥ 50% narrowing of the luminal diameter in 2 or more major coronary arteries (e.g. LAD, CX or RCA) by coronary angiography or CT angiography,
            - left main stem coronary artery with ≥ 50% narrowing of the luminal diameter,
            - prior percutaneous or surgical revascularization of ≥ 2 major coronary arteries (e.g. ≥ 2 months),
            - combination of prior percutaneous or surgical revascularization, such as e.g. of 1 major coronary artery (e.g. ≥ 2 months) and ≥ 50% narrowing of the luminal diameter by coronary angiography or CT angiography of at least 1 additional major coronary artery,
         c) high-risk single-vessel coronary artery disease, such as e.g. defined as the presence of ≥ 50% narrowing of the luminal diameter of one major coronary artery (e.g. by coronary angiography or CT angiography in patients not revascularised) and at least one of the following:
            - a positive non invasive stress test, such as e.g. confirmed by either:
               - a positive ECG exercise tolerance test in patients without left bundle branch block, Wolff-Parkinson-White syndrome, left ventricular hypertrophy with repolarization abnormality, or paced ventricular rhythm, atrial fibrillation in case of abnormal ST-T segments,
               - a positive stress echocardiogram showing induced regional systolic wall motion abnormalities,
               - a positive nuclear myocardial perfusion imaging stress test showing stress induced reversible perfusion abnormality,
               - patient discharged from hospital with a documented diagnosis of unstable angina pectoris (e.g. ≥ 2 - 12 months),
         d) previous ischemic or haemorrhagic stroke (e.g. > 3 months),
         e) presence of carotid artery disease (e.g. symptomatic or not), such as e.g. documented by either:
            - imaging techniques with at least one lesion estimated to be ≥50% narrowing of the luminal diameter,
            - prior percutaneous or surgical carotid revascularization,
         f) presence of peripheral artery disease, such as e.g. documented by either:
            - previous limb angioplasty, stenting or bypass surgery,
            - previous limb or foot amputation due to macrocirculatory insufficiency,
            - angiographic evidence of peripheral artery stenosis ≥ 50% narrowing of the luminal diameter in at least one limb (e.g. definition of peripheral artery: common iliac artery, internal iliac artery, external iliac artery, femoral artery, popliteal artery),
   Condition II:
      impaired renal function (e.g. with or without CV co-morbidities), such as e.g. defined by:
      - impaired renal function (e.g. as defined by MDRD formula) with an eGFR 15-45 mL/min/1.73 m² with any UACR, and/or
      - impaired renal function (e.g. as defined by MDRD formula) with an eGFR ≥ 45-75 mL/min/1.73 m² with an urine albumin creatinine ratio (UACR) > 200 mg/g creatinine or > 200 mg/l (milligram albumin per liter of urine) or > 200 µg/min (microgram albumin per minute) or > 200 mg/24 h (milligram albumin per 24 hours).

Furthermore, the present invention relates to a certain DPP-4 inhibitor (preferably linagliptin, optionally in combination with one or more other active agents) for use in:
treating, preventing, protecting against, reducing the risk of, delaying the occurrence of and/or delaying the progression of:
a cardio- or cerebrovascular disease, event (e.g. major cardiovascular event) or complication, such as e.g. cardiovascular death, (fatal or non-fatal) myocardial infarction (e.g. silent or non-silent MI), (fatal or non-fatal) stroke, sudden death, heart failure, and/or
hospitalisation (e.g. for acute coronary syndrome, leg amputation, coronary revascularization procedures, peripheral revascularization, heart failure or for unstable angina pectoris),
and/or, such as e.g. in combination with or together with (preferably by or within one and the same therapy or medication),
treating, preventing, protecting against, reducing the risk of, delaying the occurrence of and/or delaying the progression of:
   a (non-renal or, preferably, renal) microvascular disease or complication, such as e.g. retinopathy, cognitive decline, nephropathy, (micro- or macro-)albuminuria, chronic kidney disease (CKD), end-stage renal disease, renal impairment, acute or chronic kidney failure, renal death, and/or loss in estimated glomerular filtration rate (e.g. eGFR ≥ 50% from baseline);
   preferably in a human diabetic (particularly type 2 diabetes patient), such as e.g. in a patient suffering therefrom or at high risk thereof, such as e.g. in a patient having or being at high risk of such a cardiovascular and/or renal microvascular disease, such as e.g. in a patient at high vascular risk (e.g. as defined herein; such as e.g. a patient according to at least one of the embodiments 1 to 6 or 1 to 7 as described herein above);
   such as e.g. in a patient having:
      both
      albuminuria (e.g. micro- or macro-albuminuria)
         and
      previous macrovascular (e.g. cardio- or cerebrovascular) disease (such as e.g. myocardial infarction, coronary artery disease, (ischemic or haemorrhagic) stroke, carotid artery disease and/or peripheral artery disease),
         and/or
      either
         (mild or moderate) renal impairment (e.g. CKD stage 2 or 3, preferably eGFR ≥ 45-75 mL/min/1.73 m²) with macro-albuminuria
         or
      (moderate or severe) renal impairment (e.g. CKD stage 3 or 4, preferably eGFR 15-45 mL/min/1.73 m²) (with or without any albuminuria).

Further on, the present invention relates to a certain DPP-4 inhibitor (preferably linagliptin, optionally in combination with one or more other active agents) for treating and/or preventing metabolic diseases, such as diabetes, especially type 2 diabetes mellitus and/or diseases or conditions related thereto (e.g. diabetic complications), in a patient (particularly human patient) having or being at high risk of a cardiovascular and/or renal microvascular disease (such as e.g. a patient at high vascular risk e.g. as defined herein; such as e.g. a patient according to at least one of the embodiments 1 to 6 or 1 to 7 as described herein above).

Further, the present invention relates to a certain DPP-4 inhibitor (preferably linagliptin, optionally in combination with one or more other active agents) for treating and/or preventing diabetes, especially type 2 diabetes mellitus and/or diseases or conditions related thereto (e.g. diabetic complications, such as diabetic nephropathy and/or (micro- or macro)albuminuria), in a patient (particularly human patient) in need thereof, wherein the patient has or is at high risk of a cardiovascular and/or renal microvascular disease (such as e.g. a patient at high vascular risk e.g. as defined herein; such as e.g. a patient according to at least one of the embodiments 1 to 6 or 1 to 7 as described herein above).

Examples of metabolic disorders or diseases amenable by the therapy of this invention may include, without being limited to, type 1 diabetes, type 2 diabetes, impaired glucose tolerance (IGT), impaired fasting blood glucose (IFG), hyperglycemia, postprandial hyperglycemia, postabsorptive hyperglycemia, latent autoimmune diabetes in adults (LADA), overweight, obesity, dyslipidemia, hyperlipidemia, hypercholesterolemia, hypertriglyceridemia, hyperNEFA-emia, fasting or postprandial hyperlipidemia such as postprandial lipemia (e.g. postprandial hypertriglyceridemia), hypertension, atherosclerosis, endothelial dysfunction, osteoporosis, chronic systemic inflammation, non alcoholic fatty liver disease (NAFLD), retinopathy, neuropathy, nephropathy, nephrotic syndrome, polycystic ovarian syndrome, and/or metabolic syndrome.

The present invention further relates to a certain DPP-4 inhibitor (preferably linagliptin, optionally in combination with one or more other active agents) for use in at least one of the following methods:
- preventing, slowing the progression of, delaying the onset of or treating a metabolic disorder or disease, such as e.g. type 1 diabetes mellitus, type 2 diabetes mellitus, impaired glucose tolerance (IGT), impaired fasting blood glucose (IFG), hyperglycemia, postprandial hyperglycemia, postabsorptive hyperglycemia, latent autoimmune diabetes in adults (LADA), overweight, obesity, dyslipidemia, hyperlipidemia, hypercholesterolemia, hypertriglyceridemia, hyperNEFA-emia, postprandial lipemia, hypertension, atherosclerosis, endothelial dysfunction, osteoporosis, chronic systemic inflammation, non alcoholic fatty liver disease (NAFLD), retinopathy, neuropathy, nephropathy, nephrotic syndrome, polycystic ovarian syndrome, and/or metabolic syndrome;
- improving and/or maintaining glycemic control and/or for reducing of fasting plasma glucose, of postprandial plasma glucose, of postabsorptive plasma glucose and/or of glycosylated hemoglobin HbA1c, or preventing, reducing the risk of, slowing the progression of, delaying the onset of or treating worsening or deterioration of glycemic control, need for insulin therapy or elevated HbA1c despite treatment;
- preventing, slowing, delaying or reversing progression from pre-diabetes, impaired glucose tolerance (IGT), impaired fasting blood glucose (IFG), insulin resistance and/or from metabolic syndrome to type 2 diabetes mellitus;
- preventing, reducing the risk of, slowing the progression of, delaying the onset of or treating complications of diabetes mellitus such as micro- and macrovascular diseases, such as nephropathy, micro- or macroalbuminuria, proteinuria, nephrotic syndrome, retinopathy, cataracts, neuropathy, learning or memory impairment, neurodegenerative or cognitive disorders, dementia, cardio- or cerebrovascular diseases, tissue ischaemia, diabetic foot or ulcus, atherosclerosis, hypertension, endothelial dysfunction, myocardial infarction, acute coronary syndrome, unstable angina pectoris, stable angina pectoris, peripheral arterial occlusive disease, cardiomyopathy, heart failure, heart rhythm disorders, vascular restenosis, and/or stroke;
- reducing body weight and/or body fat and/or liver fat and/or intra-myocellular fat or preventing an increase in body weight and/or body fat and/or liver fat and/or intra-myocellular fat or facilitating a reduction in body weight and/or body fat and/or liver fat and/or intra-myocellular fat;
- preventing, slowing, delaying the onset of or treating the degeneration of pancreatic beta cells and/or the decline of the functionality of pancreatic beta cells and/or for improving, preserving and/or restoring the functionality of pancreatic beta cells and/or stimulating and/or restoring or protecting the functionality of pancreatic insulin secretion;
- preventing, slowing, delaying the onset of or treating non alcoholic fatty liver disease (NAFLD) including hepatic steatosis, non-alcoholic steatohepatitis (NASH) and/or liver fibrosis (such as e.g. preventing, slowing the progression, delaying, attenuating, treating or reversing hepatic steatosis, (hepatic) inflammation and/or an abnormal accumulation of liver fat);
- preventing, slowing the progression of, delaying the onset of or treating type 2 diabetes with failure to conventional antidiabetic mono- or combination therapy;
- achieving a reduction in the dose of conventional antidiabetic medication (e.g. of a sulphonylurea or an insulin) required for adequate therapeutic effect;
- reducing the risk for adverse effects associated with conventional antidiabetic medication (e.g. hypoglycemia or weight gain, such as associated with e.g. insulin or sulphonylurea medication); and/or
- maintaining and/or improving the insulin sensitivity and/or for treating or preventing hyperinsulinemia and/or insulin resistance;

in a patient in need thereof (such as e.g. a patient as described herein, for example a human patient having diabetes, especially type 2 diabetes), and/or
particularly in a patient (particularly human patient) having or being at high risk of a cardiovascular and/or renal microvascular disease or complication, such as e.g. in a patient at high vascular risk (e.g. as defined herein; such as e.g. a patient according to at least one of the embodiments 1 to 6 or 1 to 7 as described herein above);
such as, e.g., in a patient having:
   both
   albuminuria (e.g. micro- or macro-albuminuria)
      and
   previous macrovascular (e.g. cardio- or cerebrovascular) disease (such as e.g. myocardial infarction, coronary artery disease, (ischemic or haemorrhagic) stroke, carotid artery disease and/or peripheral artery disease),
      and/or
   either
      (mild or moderate) renal impairment (e.g. CKD stage 2 or 3, preferably eGFR ≥ 45-75 mL/min/1.73 m²) with macro-albuminuria
      or
   (moderate or severe) renal impairment (e.g. CKD stage 3 or 4, preferably eGFR 15-45 mL/min/1.73 m²) (with or without any albuminuria).

Further, the present invention relates to a method of treating, preventing, delaying the occurrence, delaying the progression, protecting against and/or reducing the likelihood or risk of: atherosclerosis, athero-thrombosis, endothelial dysfunction, and/or morbidity and/or premature mortality from cardiovascular (CV) and/or renal microvascular disease; such as e.g. a combined method of:
treating, preventing, delaying the occurrence, delaying the progression, protecting against and/or reducing the likelihood or the risk of:
a cardio- or cerebrovascular disease or event (such as e.g. selected from cardiovascular (CV) death (including fatal stroke, fatal myocardial infarction and sudden death), non-fatal stroke, non-fatal myocardial infarction (MI) (silent MI may be excluded) and hospitalisation for unstable angina pectoris),
   and
treating, preventing, delaying the occurrence, delaying the progression, protecting against and/or reducing the likelihood or the risk of:
   a renal microvascular disease (such as e.g. selected from renal death, end-stage renal disease and loss in estimated glomerular filtration rate);
   in a patient (particularly human patient particularly having diabetes, especially type 2 diabetes mellitus and/or conditions related thereto) in need thereof (such as e.g. a patient having or being at-risk of a cardio- or cerebrovascular and/or renal (micro)vascular disease, event, complication or condition such as described herein, such as e.g. a patient at high vascular risk e.g. as defined herein; such as e.g. a patient according to at least one of the embodiments 1 to 6 or 1 to 7 as described herein above);
   comprising administering an effective amount of a certain DPP-4 inhibitor (preferably linagliptin), optionally in combination with one or more other active agents, to the patient.

Further, the present invention further relates to a certain DPP-4 inhibitor (preferably linagliptin, optionally in combination with one or more other active agents, such as e.g. optionally in combination with one or more antidiabetics, and/or optionally in combination with one or more anti-hypertensive agents such as an ACE inhibitor and/or an ARB) for use in cardio- and renoprotection and/or for preventing, delaying the occurrence, protecting against, delaying the progression, or reducing the risk of:
a cardio- or cerebrovascular disease or event (such as e.g. selected from cardiovascular (CV) death (including fatal stroke, fatal myocardial infarction and sudden death), non-fatal stroke, non-fatal myocardial infarction (MI) (silent MI may be excluded) and hospitalisation for unstable angina pectoris), and
a renal microvascular disease (such as e.g. selected from renal death, end-stage renal disease and loss in estimated glomerular filtration rate);
in a patient (particularly human patient particularly having diabetes, especially type 2 diabetes mellitus, and/or conditions related thereto, such as diabetic nephropathy),
such as e.g. a patient having or being at-risk of a cardio- or cerebrovascular and/or renal (micro)vascular disease, event, complication or condition such as described herein (such as e.g. a patient at high vascular risk e.g. as defined herein; such as e.g. a patient according to at least one of the embodiments 1 to 6 or 1 to 7 as described herein above);
such as e.g. a patient having:
   both
   albuminuria (e.g. micro- or macro-albuminuria)
      and
   previous macrovascular (e.g. cardio- or cerebrovascular) disease (such as e.g. myocardial infarction, coronary artery disease, (ischemic or haemorrhagic) stroke, carotid artery disease and/or peripheral artery disease),
      and/or
   either
      (mild or moderate) renal impairment (e.g. CKD stage 2 or 3, preferably eGFR ≥ 45-75 mL/min/1.73 m²) with macro-albuminuria
      or
   (moderate or severe) renal impairment (e.g. CKD stage 3 or 4, preferably eGFR 15-45 mL/min/1.73 m²) (with or without any albuminuria).

In certain embodiments, the therapy according to the present invention (e.g. such as described hereinabove and hereinbelow) may include duration of treatment with a certain DPP-4 inhibitor, particularly linagliptin (preferably 5 mg per day, administered orally, optionally in combination with one or more other active substances, e.g. such as those described herein) over a lengthy period (such as e.g. at least 1-6 years, >/= 2 years, or 3-7 years such as 3-4 years, 3-5 years, 3-6 years, 4-5 years, 4-6 years, 5-6 years or 5-7 years, preferably at least 48 months, more preferably at least 3 years); such as e.g. to provide a long term effect on cardiovascular and/or renal (microvascular) safety, morbidity and/or mortality (e.g. including effect on cognitive impairment) according to the present invention; such as e.g. in patients (e.g. diabetic patients, especially type 2 diabetes patients) having or being at high risk of cardiovascular and/or renal microvascular disease, such as e.g. in patients at high vascular risk (such as e.g. those patients described herein); such as e.g. in those patients according to at least one of the embodiments 1 to 7 as described herein.

For example, the therapy according to the present invention (e.g. such as described hereinabove and hereinbelow) may include duration of treatment with a certain DPP-4 inhibitor, particularly linagliptin (preferably 5 mg per day, administered orally, optionally in combination with one or more other active substances, e.g. such as those described herein) over a lengthy period, preferably at least 48 months, more preferably at least 3 years (such as e.g. at least 3-4 years, or at least 5-6 years).

Other aspects of the present invention become apparent to the skilled person from the foregoing and following remarks (including the examples and claims).

### Brief Description of the Drawings

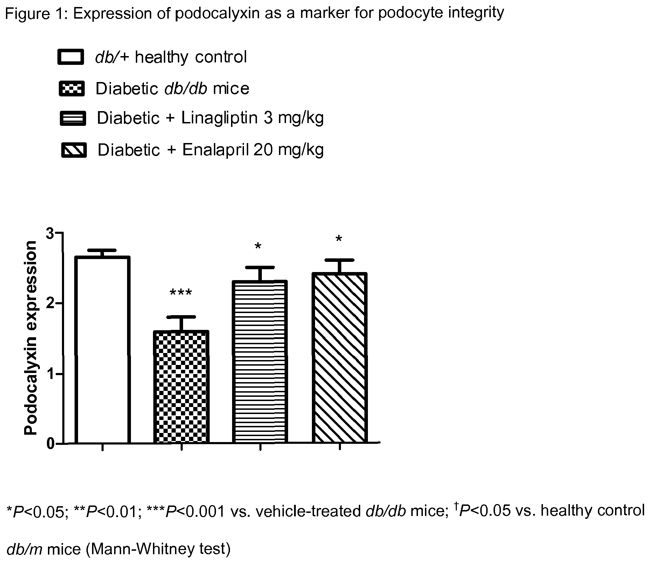

Figure 1 shows the expression of podocalyxin as a marker for podocyte integrity in linagliptin-, enalapril- or vehicle-treated diabetic db/db mice and in healthy control mice:

### Detailed Description of the Invention

Within the scope of the present invention it has now been found that a certain DPP-4 inhibitor (preferably linagliptin) as defined herein as well as pharmaceutical combinations, compositions, uses or methods according to this invention of that DPP-4 inhibitor (preferably linagliptin) optionally in combination with one or more other active agents as defined herein have properties, which make them useful for the purpose of this invention and/or for fulfilling one or more of the needs mentioned herein.

The enzyme DPP-4 (dipeptidyl peptidase IV) also known as CD26 is a serine protease known to lead to the cleavage of a dipeptide from the N-terminal end of a number of proteins having at their N-terminal end a prolin or alanin residue. Due to this property DPP-4 inhibitors interfere with the plasma level of bioactive peptides including the peptide GLP-1 and are considered to be promising drugs for the treatment of diabetes mellitus.

For example, DPP-4 inhibitors and their uses are disclosed in WO 2002/068420, WO 2004/018467, WO 2004/018468, WO 2004/018469, WO 2004/041820, WO 2004/046148, WO 2005/051950, WO 2005/082906, WO 2005/063750, WO 2005/085246, WO 2006/027204, WO 2006/029769, WO2007/014886; WO 2004/050658, WO 2004/111051, WO 2005/058901, WO 2005/097798; WO 2006/068163, WO 2007/071738, WO 2008/017670; WO 2007/128721, WO 2007/128724, WO 2007/128761, or WO 2009/121945.

DPP-4 is analogous to CD26 a T-cell antigene which plays a role in T-cell activation and immuno-modulation. Further, some substrates of DPP-4 (beyond incretins) may have potential cardio-renal effects.

Furthermore, linagliptin, a selective DPP-4 inhibitor may qualify for the instant purposes with certain anti-oxidative and/or anti-inflammatory features.

Linagliptin may further have a direct impact on the integrity of the endothelium and podocytes of the glomerula and the proximal tubular cells of the kidney as well as on endothelial function and linagliptin has a relatively high tissue distribution, including in the kidney.

Further, samples from human kidneys indicate that proteinuric human diseases (such as e.g. diabetic nephropathy or nephrotic syndrome) seem to be characterized by an upregulation of glomerular DPP-4.

Moreover, linigliptin may further qualify for the instant purposes by antidiabetic and antialbuminuric effects/usability preferably in type 2 diabetes patients, with micro- or macroalbuminuria (e.g. 30-3000 mg/g creatinine), preferably on top of current standard treatment for diabetic nephropathy (e.g. ACE inhibitor or ARB).

In a further embodiment, the patient described herein is a subject having diabetes (e.g. type 1 or type 2 diabetes or LADA, particularly type 2 diabetes).

In particular, the subject within this invention may be a human, e.g. human child, a human adolescent or, particularly, a human adult.

In further particular, the subject within this invention is a human type 2 diabetes patient.

In certain embodiments, the subject within this invention is a (human) type 2 diabetes patient in early diabetes stage (in one embodiment) or in advanced diabetes stage (in another embodiment).

In a further embodiment, the subject within this invention is a (human) type 2 diabetes patient in renal disease diabetes stage (i.e. diabetes associated with renal disease).

Accordingly, in a particular embodiment, a preferred DPP-4 inhibitor within the meaning of this invention is linagliptin.

In a further preferred embodiment within this invention, linagliptin is used as both cardioprotective and renoprotective medication (particularly antidiabetic medication). Accordingly, linagliptin is for use in both cardioprotection and renoprotection. Further, linagliptin is for use in conferring cardioprotective and renoprotective effects or benefits to the patient (particularly diabetic patient, such as e.g. type 2 diabetes patient), including at-risk patient, such as a patient at high vascular risk (e.g. as defined herein; such as e.g. a patient according to at least one of the embodiments 1 to 6 as described herein above).

In a further preferred embodiment within this invention, linagliptin is useful in a method of preventing, protecting against, reducing the risk of and/or delaying the occurrence of:
a cardio- or cerebrovascular disease or event, such as e.g. selected from cardiovascular (CV) death (including fatal stroke, fatal myocardial infarction and sudden death), non-fatal stroke, non-fatal myocardial infarction (MI) (silent MI may be excluded) and hospitalisation for unstable angina pectoris,
   and/or
a renal microvascular disease, such as e.g. selected from renal death, end-stage renal disease and loss in estimated glomerular filtration rate (e.g. eGFR ≥ 50% from baseline); preferably in a human diabetic (particularly type 2 diabetes patient);
including e.g. in a patient with or at high risk of a cardiovascular event and/or renal microvascular disease, such as e.g. in a patient at high vascular risk (e.g. at high risk of CV events, e.g. as described herein; such as e.g. a patient according to at least one of the embodiments 1 to 6 as described herein above);
such as e.g. in a patient having:
   both
   albuminuria (e.g. micro- or macro-albuminuria)
      and
   previous macrovascular (e.g. cardio- or cerebrovascular) disease (such as e.g. myocardial infarction, coronary artery disease, (ischemic or haemorrhagic) stroke, carotid artery disease and/or peripheral artery disease),
      and/or
   either
      (mild or moderate) renal impairment (e.g. CKD stage 1, 2 or 3, such as CKD stage 1, 2 (mild) or 3a (mild-moderate), preferably eGFR ≥ 45-75 mL/min/1.73 m²) with macro-albuminuria,
      or
   (moderate or severe) renal impairment (e.g. CKD stage 3 or 4, such as CKD stage 3b (moderate-severe) or 4 (severe), preferably eGFR 15-45 mL/min/1.73 m²), with or without any albuminuria (such as e.g. with or without micro- or macro-albuminuria).

In a further preferred embodiment within this invention, linagliptin is useful in (cardioprotective and renoprotective) treatment of diabetes, particularly type 2 diabetes, including e.g. in a patient with or at high risk of a cardiovascular event and/or renal microvascular disease, such as e.g. in a patient at high vascular risk (e.g. at high risk of CV events, e.g. as described herein);
such as e.g. in a patient having:
both
albuminuria (e.g. micro- or macro-albuminuria)
   and
previous macrovascular (e.g. cardio- or cerebrovascular) disease (such as e.g. myocardial infarction, coronary artery disease, (ischemic or haemorrhagic) stroke, carotid artery disease and/or peripheral artery disease),
   and/or
either
   (mild or moderate) renal impairment (e.g. CKD stage 1, 2 or 3, such as CKD stage 1, 2 (mild) or 3a (mild-moderate), preferably eGFR ≥ 45-75 mL/min/1.73 m²) with macro-albuminuria,
   or
(moderate or severe) renal impairment (e.g. CKD stage 3 or 4, such as CKD stage 3b (moderate-severe) or 4 (severe), preferably eGFR 15-45 mL/min/1.73 m²), with or without any albuminuria (such as e.g. with or without micro- or macro-albuminuria).

In a particular embodiment of this invention, linagliptin is useful in the therapy of a patient (preferably diabetic, particularly type 2 diabetes patient), having:
both
albuminuria (e.g. micro- or macro-albuminuria)
   and
previous macrovascular (e.g. cardio- or cerebrovascular) disease (such as e.g. myocardial infarction, coronary artery disease, (ischemic or haemorrhagic) stroke, carotid artery disease and/or peripheral artery disease),
   and/or
either
   (mild or moderate) renal impairment (e.g. CKD stage 1, 2 or 3, such as CKD stage 1, 2 (mild) or 3a (mild-moderate), preferably eGFR ≥ 45-75 mL/min/1.73 m²) with macro-albuminuria,
   or
(moderate or severe) renal impairment (e.g. CKD stage 3 or 4, such as CKD stage 3b (moderate-severe) or 4 (severe), preferably eGFR 15-45 mL/min/1.73 m²), with or without any albuminuria (such as e.g. with or without micro- or macro-albuminuria).

In a further particular embodiment of this invention, linagliptin is useful in the therapy of a patient (preferably diabetic, particularly type 2 diabetes patient), having:
albuminuria (such as e.g. urine albumin creatinine ratio (UACR) ≥ 30 mg/g creatinine or ≥ 30 mg/l (milligram albumin per liter of urine) or ≥ 30 µg/min (microgram albumin per minute) or ≥ 30 mg/24 h (milligram albumin per 24 hours)) and
previous macrovascular disease, such as e.g. defined as one or more of a) to f):
   a) previous myocardial infarction,
   b) advanced coronary artery disease,
   c) high-risk single-vessel coronary artery disease,
   d) previous ischemic or haemorrhagic stroke,
   e) presence of carotid artery disease,
   f) presence of peripheral artery disease,
   or
impaired renal function (e.g. with or without CV co-morbidities), such as e.g. defined by:
   - impaired renal function (e.g. as defined by MDRD formula) with an eGFR 15-45 mL/min/1.73 m² with any urine albumin creatinine ratio (UACR), and/or
   - impaired renal function (e.g. as defined by MDRD formula) with an eGFR ≥ 45-75 mL/min/1.73 m² with an urine albumin creatinine ratio (UACR) > 200 mg/g creatinine or > 200 mg/l (milligram albumin per liter of urine) or > 200 µg/min (microgram albumin per minute) or > 200 mg/24 h (milligram albumin per 24 hours).

In a yet further particular embodiment of this invention, linagliptin is useful in the therapy of a patient (preferably diabetic, particularly type 2 diabetes patient) with the Condition I as defined herein.

In another yet further particular embodiment of this invention, linagliptin is useful in the therapy of a patient (preferably diabetic, particularly type 2 diabetes patient) with the Condition II as defined herein.

In another embodiment of this invention, linagliptin (optionally in combination with one or more further active agents, such as described herein) is useful in the therapy of a patient (preferably diabetic, particularly type 2 diabetes patient) with or at high risk of a cardiovascular and/or renal microvascular disease, such as e.g. a patient at high vascular risk (e.g. at high risk of CV events), e.g. as described herein; such as e.g. a patient according to at least one of the embodiments 1 to 6 or 1 to 7 as described herein; and, optionally, with inadequate control of albuminuria despite therapy with an angiotensin-converting enzyme (ACE) inhibitor and/or an angiotensin II receptor blocker (ARB).

In another embodiment of this invention, linagliptin (optionally in combination with one or more further active agents, such as described herein) is useful in the therapy of a patient (preferably diabetic, particularly type 2 diabetes patient) such as described herein, who (additionally) may have or may be at risk of cognitive impairment, cognitive decline or dementia, such as e.g. a patient who may be at higher risk of hypoglycaemia, and/or who may be an elderly patient, and/or who may have a history of one or more diabetic complications (such as e.g. retinopathy, neuropathy, nephropathy, a macrovascular (CV) complication), and/or who may have advanced diabetes (e.g. duration of diabetes > 10 years), and/or who may be experienced with one or more antidiabetic medications (such as e.g. with previous or ongoing therapy with one or more conventional antidiabetic agents, such as e.g. metformin), and/or who may be on polypharmacy (e.g. taking 5 drugs or more per day), and/or who may be a patient at high vascular risk (e.g. at high risk of CV events), e.g. as described herein, such as e.g. a patient according to at least one of the embodiments 1 to 6 or 1 to 7 as described herein.

Effects of a therapy according to this invention on cognition may include and may be assessed by basic and instrumental activities of daily living scores, nutritional assessment scores, depression scale scores, cognitive skills and/or functional performance.

Pharmaceutical compositions or combinations for use in these therapies of this invention (e.g. treatments or preventions or protections) comprising a certain DPP-4 inhibitor (preferably linagliptin) as defined herein optionally together with one or more other active agents are also contemplated.

Further, the present invention relates to a certain DPP-4 inhibitor (preferably linagliptin), optionally in combination with one, two or more further active agents, each as defined herein, for use in the therapies (e.g. treatments or preventions or protections) as described herein.

Further, the present invention relates to the use of a certain DPP-4 inhibitor (preferably linagliptin), optionally in combination with one, two or more further active agents, each as defined herein, for preparing a pharmaceutical composition which is suitable for the treatment and/or prevention and/or protection purposes of this invention.

Further, the present invention relates to the use of a certain DPP-4 inhibitor (preferably linagliptin) for preparing a pharmaceutical composition for use in a therapy as described herein.

Further, the present invention relates to a therapeutic method as described herein, said method comprising administering an effective amount of a certain DPP-4 inhibitor (preferably linagliptin) and, optionally, one or more other active or therapeutic agents to the patient in need thereof, each as described herein.

Other aspects of the present invention become apparent to the skilled person from the foregoing and following remarks (including the examples and claims).

The aspects of the present invention, in particular the pharmaceutical compounds, compositions, combinations, methods and uses, refer to a certain DPP-4 inhibitor (preferably linagliptin), optionally in combination with one or more other active agents, as defined hereinbefore and hereinafter.

In the monitoring of the treatment of diabetes mellitus the HbA1c value, the product of a nonenzymatic glycation of the haemoglobin B chain, is of exceptional importance. As its formation depends essentially on the blood sugar level and the life time of the erythrocytes the HbA1c in the sense of a "blood sugar memory" reflects the average blood sugar level of the preceding 4-12 weeks. Diabetic patients whose HbA1c level has been well controlled over a long time by more intensive diabetes treatment (i.e. < 6.5 % of the total haemoglobin in the sample) are significantly better protected from diabetic microangiopathy. The available treatments for diabetes can give the diabetic an average improvement in their HbA1c level of the order of 1.0 - 1.5 %. This reduction in the HbA1C level is not sufficient in all diabetics to bring them into the desired target range of < 7.0 %, preferably < 6.5 % and more preferably < 6 % HbA1c.

Within the meaning of this invention, inadequate or insufficient glycemic control means in particular a condition wherein patients show HbA1c values above 6.5%, in particular above 7.0%, even more preferably above 7.5%, especially above 8%. An embodiment of patients with inadequate or insufficient glycemic control include, without being limited to, patients having a HbA1c value from 6.5 to 10% (or, in another embodiment, from 7.5 to 10%; or, in another embodiment, from 7.5 to 11%, or, in another embodiment, from 6.5 to 8.5% or, in another embodiment, from 6.5 to 7.5%). A special sub-embodiment of inadequately controlled patients refers to patients with poor glycemic control including, without being limited, patients having a HbA1c value ≥ 9%.

Within glycemic control, in addition to improvement of the HbA1c level, other recommended therapeutic goals for type 2 diabetes mellitus patients are improvement of fasting plasma glucose (FPG) and of postprandial plasma glucose (PPG) levels to normal or as near normal as possible. Recommended desired target ranges of preprandial (fasting) plasma glucose are 70-130 mg/dL (or 90-130 mg/dL) or <110 mg/dL, and of two-hour postprandial plasma glucose are <180 mg/dL or <140 mg/dL.

Further, in some embodiments, the patients according to this invention (including the patients (preferably diabetic, particularly type 2 diabetes patients) having or being at-risk of a cardioor cerebrovascular and/or renal (micro)vascular disease, event, complication or condition, such as e.g. patients at high vascular risk (e.g. at high risk of CV events), e.g. such as disclosed otherwise herein, such as e.g. in a patient according to at least one of the embodiments 1 to 6 or 1 to 7 as described herein) may be further characterized as follows or may further include any of the following patients, for example.

In an embodiment, diabetes patients within the meaning of this invention may include patients who have not previously been treated with an antidiabetic drug (drug-naive patients). Thus, in an embodiment, the therapies described herein may be used in naive patients. In certain embodiments of the therapies of this invention, the DPP-4 inhibitor (preferably linagliptin) may be used alone or in combination with one or more other antidiabetics in such patients. In another embodiment, diabetes patients within the meaning of this invention may include patients pre-treated with conventional antidiabetic background medication, such as e.g. patients with advanced or late stage type 2 diabetes mellitus (including patients with failure to conventional antidiabetic therapy), such as e.g. patients with inadequate glycemic control on one, two or more conventional oral and/or non-oral antidiabetic drugs as defined herein, such as e.g. patients with insufficient glycemic control despite (mono-)therapy with metformin, a thiazolidinedione (particularly pioglitazone), a sulphonylurea, a glinide, GLP-1 or GLP-1 analogue, insulin or insulin analogue, or an α-glucosidase inhibitor, or despite dual combination therapy with metformin/sulphonylurea, metformin/thiazolidinedione (particularly pioglitazone), sulphonylurea/ α-glucosidase inhibitor, pioglitazone/sulphonylurea, metformin/insulin, pioglitazone/insulin or sulphonylurea/insulin. Thus, in an embodiment, the therapies described herein may be used in patients experienced with therapy, e.g. with conventional oral and/or non-oral antidiabetic mono- or dual or triple combination medication as mentioned herein. In certain embodiments of the therapies of this invention, in such patients the DPP-4 inhibitor (preferably linagliptin) may be used on top of or added on the existing or ongoing conventional oral and/or non-oral antidiabetic mono- or dual or triple combination medication with which such patients are pre-treated or experienced.

For example, a diabetes patient (particularly type 2 diabetes patient, with insufficient glycemic control) of this invention may be treatment-naive or pre-treated with one or more (e.g. one or two) conventional antidiabetic agents selected from metformin, thiazolidinediones (particularly pioglitazone), sulphonylureas, glinides, α-glucosidase inhibitors (e.g. acarbose, voglibose), and insulin or insulin analogues, such as e.g. pre-treated or experienced with:
metformin, α-glucosidase inhibitor, sulphonylurea or glinide monotherapy, or metformin plus α-glucosidase inhibitor, metformin plus sulphonylurea, metformin plus glinide, α-glucosidase inhibitor plus sulphonylurea, or α-glucosidase inhibitor plus glinide dual combination therapy.

In certain embodiments relating to such treatment-naive patients, the DPP-4 inhibitor (preferably linagliptin) may be used as monotherapy, or as initial combination therapy such as e.g. with metformin, a thiazolidinedione (particularly pioglitazone), a sulphonylurea, a glinide, an α-glucosidase inhibitor (e.g. acarbose, voglibose), GLP-1 or GLP-1 analogue, or insulin or insulin analogue; preferably as monotherapy.

In certain embodiments relating to such patients pre-treated or experienced with one or two conventional antidiabetic agents, the DPP-4 inhibitor (preferably linagliptin) may be used as as add-on combination therapy, i.e. added to an existing or background therapy with the one or two conventional antidiabetics in patients with insufficient glycemic control despite therapy with the one or more conventional antidiabetic agents, such as e.g. as add-on therapy to one or more (e.g. one or two) conventional antidiabetics selected from metformin, thiazolidinediones (particularly pioglitazone), sulphonylureas, glinides, α-glucosidase inhibitors (e.g. acarbose, voglibose), GLP-1 or GLP-1 analogues, and insulin or insulin analogues, such as e.g.:
as add-on therapy to metformin, to a α-glucosidase inhibitor, to a sulphonylurea or to a glinide;
or as add-on therapy to metformin plus α-glucosidase inhibitor, to metformin plus sulphonylurea, to metformin plus glinide, to α-glucosidase inhibitor plus sulphonylurea, or to α-glucosidase inhibitor plus glinide;
or as add-on therapy to an insulin, with or without metformin, a thiazolidinedione (particularly pioglitazone), a sulphonylurea, a glinide or an α-glucosidase inhibitor (e.g. acarbose, voglibose).

A further embodiment of diabetic patients within the meaning of this invention refers to patients ineligible for metformin therapy including
- patients for whom metformin therapy is contraindicated, e.g. patients having one or more contraindications against metformin therapy according to label, such as for example patients with at least one contraindication selected from:
   renal disease, renal impairment or renal dysfunction (e.g., as specified by product information of locally approved metformin),
   dehydration,
   unstable or acute congestive heart failure,
   acute or chronic metabolic acidosis, and
   hereditary galactose intolerance;
   and
- patients who suffer from one or more intolerable side effects attributed to metformin, particularly gastrointestinal side effects associated with metformin, such as for example patients suffering from at least one gastrointestinal side effect selected from:
   nausea,
   vomiting,
   diarrhoea,
   intestinal gas, and
   severe abdominal discomfort.

A further embodiment of the diabetes patients which may be amenable to the therapies of this invention may include, without being limited, those diabetes patients for whom normal metformin therapy is not appropriate, such as e.g. those diabetes patients who need reduced dose metformin therapy due to reduced tolerability, intolerability or contraindication against metformin or due to (mildly) impaired/reduced renal function (including elderly patients, such as e.g. ≥ 60-65 years).

A further embodiment of patients (e.g. which may be diabetic or non-diabetic) within the meaning of this invention may refer to patients having renal disease, renal dysfunction, or insufficiency or impairment of renal function (including mild, moderate and/or severe renal impairment), e.g. as may be suggested (if not otherwise noted) by elevated serum creatinine levels (e.g. serum creatinine levels above the upper limit of normal for their age, e.g. ≥ 130 - 150 µmol/l, or ≥ 1.5 mg/dl (≥ 136 µmol/l) in men and ≥ 1.4 mg/dl (≥ 124 µmol/l) in women) or abnormal creatinine clearance (e.g. glomerular filtration rate (GFR) ≤ 30 - 60 ml/min).

In this context, in a further embodiment, mild renal impairment may be e.g. suggested (if not otherwise noted) by a creatinine clearance of 50-80 ml/min (approximately corresponding to serum creatine levels of ≤1.7 mg/dL in men and ≤1.5 mg/dL in women); moderate renal impairment may be e.g. suggested (if not otherwise noted) by a creatinine clearance of 30-50 ml/min (approximately corresponding to serum creatinine levels of >1.7 to ≤3.0 mg/dL in men and >1.5 to ≤2.5 mg/dL in women); and severe renal impairment may be e.g. suggested (if not otherwise noted) by a creatinine clearance of < 30 ml/min (approximately corresponding to serum creatinine levels of >3.0 mg/dL in men and >2.5 mg/dL in women). Patients with end-stage renal disease require dialysis (e.g. hemodialysis or peritoneal dialysis).

In another further embodiment, patients with renal disease, renal dysfunction or renal impairment may include patients with chronic renal insufficiency or impairment, which can be stratified (if not otherwise noted) according to glomerular filtration rate (GFR, ml/min/1.73m²) into 5 disease stages: stage 1 characterized by normal GFR ≥ 90 plus either persistent albuminuria (e.g. UACR ≥30 mg/g) or known structural or hereditary renal disease; stage 2 characterized by mild reduction of GFR (GFR 60-89) describing mild renal impairment; stage 3 characterized by moderate reduction of GFR (GFR 30-59) describing moderate renal impairment; stage 4 characterized by severe reduction of GFR (GFR 15-29) describing severe renal impairment; and terminal stage 5 characterized by requiring dialysis or GFR < 15 describing established kidney failure (end-stage renal disease, ESRD).

Chronic kidney disease and its stages (CKD 1-5) can be usually characterized or classified accordingly, such as based on the presence of either kidney damage (albuminuria) or impaired estimated glomerular filtration rate (GFR <60 [ml/min/1.73m²], with or without kidney damage).

Albuminuria stages may be for example classified as disclosed herein and/or by urine albumin creatinine ratio (such as usually UACR ≥30 mg/g, in some instances ≥20 µg/min albumin excretion rate), such as e.g. microalbuminuria may be for example classified by UACR 30-300 mg/g (in some instances 20-200 µg/min) or, in another embodiment, by UACR 30-200 mg/g, and/or macroalbuminuria may be for example classified by UACR >300 mg/g (in some instances >200 µg/min), or, in another embodiment, by UACR >200 mg/g. Very high UACR ≥2000 mg/g may be classified as nephrotic.

A further embodiment of patients within the meaning of this invention may refer to diabetes patients with or at-risk of developing renal complications, such as diabetic nephropathy (including chronic and progressive renal insufficiency, albuminuria, proteinuria, fluid retention in the body (edema) and/or hypertension).

A further embodiment of patients within the meaning of this invention (which may be diabetic or non-diabetic) may refer to patients (preferably diabetic patients) with inadequate control of albuminuria despite therapy with an angiotensin-converting enzyme (ACE) inhibitor and/or an angiotensin II receptor blocker (ARB).

A further embodiment of patients within the meaning of this invention (which may be diabetic or non-diabetic) may refer to patients (preferably diabetic patients, particularly type 2 diabetes patients) having renal- and/or cardiovascular-disease history and/or medications, such as diabetic nephropathy, macrovascular disease (e.g. coronary artery disease, peripheral artery disease, cerebrovascular disease, hypertension), microvascular disease (e.g. diabetic nephropathy, neuropathy, retinopathy), coronary artery disease, cerebrovascular disease, peripheral artery disease, hypertension, ex-smoker or current smoker, and/or on acetylsalicylic acid, antihypertensive and/or lipid lowering medication, such as e.g. on (previous or ongoing) therapy with acetylsalicylic acid, an ACE inhibitor, ARB, beta-blocker, Calcium-antagonist or diuretic, or combination thereof, and/or on (previous or ongoing) therapy with a fibrate, niacin or statin, or combination thereof.

A further embodiment of patients within the meaning of this invention (which may be diabetic or non-diabetic) may refer to patients (preferably diabetic patients, particularly type 2 diabetes patients) with diabetic nephropathy (with or without additional standard background therapy such as e.g. with an ACEi or ARB), e.g. including a vulnerable diabetic nephropathy patient such as who are aged ≥ 65 years typically having longer diabetes duration (> 5 years), renal impairment (such as mild (60 to <90 eGFR ml/min/1.73 m²) or moderate (30 to <60 eGFR ml/min/1.73 m²) renal impairment) and/or higher baseline UACR (such as advanced stages of micro- or macroalbuminuria).

A further embodiment of patients within the meaning of this invention (which may be diabetic or non-diabetic) may refer to patients (preferably diabetic patients, particularly type 2 diabetes patients) with diabetic nephropathy, especially in those patients on (e.g. previous or ongoing) therapy with an angiotensin-converting enzyme (ACE) inhibitor and/or an angiotensin II receptor blocker (ARB), such as e.g. patients with inadequate control of albuminuria despite therapy with an angiotensin-converting enzyme (ACE) inhibitor and/or an angiotensin II receptor blocker (ARB).

The DPP-4 inhibitor may be administered in combination (e.g. on-top, add-on) with the background medication such as e.g. angiotensin-converting enzyme (ACE) inhibitor or the angiotensin II receptor blocker (ARB), to the patient (e.g. such as to those patients described above).

In certain embodiments, the patients which may be amenable to the therapies of this invention may have or are at-risk of one or more of the following diseases, disorders or conditions: type 1 diabetes, type 2 diabetes, impaired glucose tolerance (IGT), impaired fasting blood glucose (IFG), hyperglycemia, postprandial hyperglycemia, postabsorptive hyperglycemia, latent autoimmune diabetes in adults (LADA), overweight, obesity, dyslipidemia (including e.g. atherogenic dyslipidemia), hyperlipidemia, hypercholesterolemia, hypertriglyceridemia, hyperNEFA-emia, postprandial lipemia, hypertension, atherosclerosis, endothelial dysfunction, osteoporosis, chronic systemic inflammation, non alcoholic fatty liver disease (NAFLD), polycystic ovarian syndrome, hyperuricemia, metabolic syndrome, nephropathy, micro- or macroalbuminuria, proteinuria, nephrotic syndrome, retinopathy, cataracts, neuropathy, learning or memory impairment, neurodegenerative or cognitive disorders, dementia, cardio- or cerebrovascular diseases, tissue ischaemia, diabetic foot or ulcus, atherosclerosis, hypertension, endothelial dysfunction, myocardial infarction, acute coronary syndrome, unstable angina pectoris, stable angina pectoris, peripheral arterial occlusive disease, cardiomyopathy (including e.g. uremic cardiomyopathy), heart failure, cardiac hypertrophy, heart rhythm disorders, vascular restenosis, stroke, (renal, cardiac, cerebral or hepatic) ischemia/reperfusion injuries, (renal, cardiac, cerebral or hepatic) fibrosis, (renal, cardiac, cerebral or hepatic) vascular remodelling; a diabetic disease, e.g. type 2 diabetes mellitus being particularly worthy to be noted (e.g. as an underlying disease).

Accordingly, the present invention thus relates to a certain DPP-4 inhibitor as defined herein, preferably linagliptin (BI 1356), for use in the therapies (e.g. treatments and/or preventions and/or protections) described herein.

The present invention further relates to a certain DPP-4 inhibitor as defined herein, preferably linagliptin (BI 1356), in combination with metformin, for use in the therapies (e.g. treatments and/or preventions and/or protections) described herein.

The present invention further relates to a certain DPP-4 inhibitor as defined herein, preferably linagliptin (BI 1356), in combination with metformin and/or a sulphonylurea, for use in the therapies (e.g. treatments and/or preventions and/or protections) described herein.

The present invention further relates to a certain DPP-4 inhibitor as defined herein, preferably linagliptin (BI 1356), in combination with pioglitazone, for use in the therapies (e.g. treatments and/or preventions and/or protections) described herein.

The present invention further relates to a certain DPP-4 inhibitor as defined herein, preferably linagliptin (BI 1356), in combination with telmisartan, for use in the therapies (e.g. treatments and/or preventions and/or protections) described herein.

The present invention further relates to a certain DPP-4 inhibitor as defined herein, preferably linagliptin (BI 1356), in combination with an insulin or insulin analogue (e.g. basal insulin, such as e.g. insulin glargin, insulin detemir or insulin degludec, or NPH insulin) for use in the therapies (e.g. treatments and/or preventions and/or protections) described herein.

The present invention further relates to a certain DPP-4 inhibitor as defined herein, preferably linagliptin (BI 1356), in combination with a diuretic, an ARB and/or an ACE inhibitor for use in the therapies (e.g. treatments and/or preventions and/or protections) described herein.

The present invention further relates to a certain DPP-4 inhibitor as defined herein, preferably linagliptin (BI 1356), in combination with one or more other active agents, e.g. selected from other antidiabetic substances, active substances that lower the blood sugar level, active substances that lower the lipid level in the blood, active substances that raise the HDL level in the blood, active substances that lower blood pressure, and active substances that are indicated in the treatment of atherosclerosis or obesity, for use in the therapies (e.g. treatments and/or preventions and/or protections) described herein.

The present invention further relates to a certain DPP-4 inhibitor as defined herein, preferably linagliptin (BI 1356), in combination with one or more other antidiabetics selected from the group consisting of metformin, a sulphonylurea, nateglinide, repaglinide, a thiazolidinedione, a PPAR-gamma-agonist, an alpha-glucosidase inhibitor, insulin or an insulin analogue, and GLP-1 or a GLP-1 analogue, optionally in combination with one or more further active agents (e.g. selected from a diuretic, ACE inhibitor and/or ARB, such as e.g. telmisartan), for use in the therapies (e.g. treatments and/or preventions and/or protections) described herein.

The present invention further relates to a pharmaceutical composition comprising a certain DPP-4 inhibitor as defined herein, preferably linagliptin (BI 1356), for use in the therapies described herein.

The present invention further relates to a pharmaceutical composition comprising a certain DPP-4 inhibitor as defined herein, preferably linagliptin (BI 1356), and metformin, for use in the therapies described herein.

The present invention further relates to a pharmaceutical composition comprising a certain DPP-4 inhibitor as defined herein, preferably linagliptin (BI 1356), and pioglitazone, for use in the therapies described herein.

The present invention further relates to a combination comprising a certain DPP-4 inhibitor (particularly linagliptin) and one or more other active agents selected from those mentioned herein, e.g. selected from other antidiabetic substances, active substances that lower the blood sugar level, active substances that lower the lipid level in the blood, active substances that raise the HDL level in the blood, active substances that lower blood pressure, active substances that are indicated in the treatment of atherosclerosis or obesity, e.g. each as described herein; particularly for simultaneous, separate or sequential use in the therapies (e.g. treatments and/or preventions and/or protections) described herein.

The present invention further relates to a combination comprising a certain DPP-4 inhibitor (particularly linagliptin) and one or more other antidiabetics selected from the group consisting of metformin, a sulphonylurea, nateglinide, repaglinide, a thiazolidinedione, a PPAR-gamma-agonist, an alpha-glucosidase inhibitor, insulin or an insulin analogue, and GLP-1 or a GLP-1 analogue, particularly for simultaneous, separate or sequential use in the therapies (treatments and/or preventions and/or protections) described herein, optionally in combination with (e.g. simultaneously, separately or sequentially) with a diuretic, ACE inhibitor and/or ARB, such as e.g. telmisartan.

The present invention further relates to therapies or (therapeutic or preventive or protective) methods or uses as described herein, comprising administering (e.g. simultaneously, separately or sequentially) an effective amount of a certain DPP-4 inhibitor (particularly linagliptin) as defined herein and, optionally, one or more other active agents, such as e.g. one or more other antidiabetics selected from the group consisting of metformin, a sulphonylurea, nateglinide, repaglinide, a thiazolidinedione, a PPAR-gamma-agonist, an alpha-glucosidase inhibitor, insulin or an insulin analogue, and GLP-1 or a GLP-1 analogue, (preferably selected from the group consisting of metformin, a sulphonylurea, nateglinide, repaglinide, a thiazolidinedione, a PPAR-gamma-agonist, an alpha-glucosidase inhibitor, and insulin or an insulin analogue), optionally in combination (e.g. simultaneously, separately or sequentially) with one or more further active agents (e.g. a diuretic, ACE inhibitor and/or ARB, such as e.g. telmisartan), to the patient (particularly human patient) in need thereof, such as e.g. a patient as described herein, preferably a human diabetic (particularly type 2 diabetes patient);
including e.g. a patient having or being at high risk of a cardiovascular and/or renal microvascular disease, such as e.g. a patient at high vascular risk (e.g. at high risk of CV events), e.g. as described herein; such as e.g. a patient according to at least one of the embodiments 1 to 6 or 1 to 7 as described herein.

The present invention further relates to therapies or (therapeutic or preventive or protective) methods or uses as described herein, comprising administering (e.g. simultaneously, separately or sequentially) an effective amount of linagliptin, to the patient (particularly human patient) in need thereof, such as e.g. a patient as described herein, preferably a human diabetic (particularly type 2 diabetes patient);
including e.g. a patient having or being at high risk of a cardiovascular and/or renal microvascular disease, such as e.g. a patient at high vascular risk (e.g. at high risk of CV events), e.g. as described herein; such as e.g. a patient according to at least one of the embodiments 1 to 6 or 1 to 7 as described herein.

The present invention further relates to therapies or (therapeutic or preventive or protective) methods or uses as described herein, comprising administering (e.g. simultaneously, separately or sequentially) an effective amount of linagliptin and metformin, to the patient (particularly human patient) in need thereof, such as e.g. a patient as described herein, preferably a human diabetic (particularly type 2 diabetes patient);
including e.g. a patient having or being at high risk of a cardiovascular and/or renal microvascular disease, such as e.g. a patient at high vascular risk (e.g. at high risk of CV events), e.g. as described herein; such as e.g. a patient according to at least one of the embodiments 1 to 6 or 1 to 7 as described herein.

The present invention further relates to therapies or (therapeutic or preventive or protective) methods or uses as described herein, comprising administering (e.g. simultaneously, separately or sequentially) an effective amount of linagliptin and metformin and a sulphonylurea, to the patient (particularly human patient) in need thereof, such as e.g. a patient as described herein, preferably a human diabetic (particularly type 2 diabetes patient);
including e.g. a patient having or being at high risk of a cardiovascular and/or renal microvascular disease, such as e.g. a patient at high vascular risk (e.g. at high risk of CV events), e.g. as described herein; such as e.g. a patient according to at least one of the embodiments 1 to 6 or 1 to 7 as described herein.

The present invention further relates to therapies or (therapeutic or preventive or protective) methods or uses as described herein, comprising administering (e.g. simultaneously, separately or sequentially) an effective amount of linagliptin and pioglitazone, to the patient (particularly human patient) in need thereof, such as e.g. a patient as described herein, preferably a human diabetic (particularly type 2 diabetes patient);
including e.g. a patient having or being at high risk of a cardiovascular and/or renal microvascular disease, such as e.g. a patient at high vascular risk (e.g. at high risk of CV events), e.g. as described herein; such as e.g. a patient according to at least one of the embodiments 1 to 6 or 1 to 7 as described herein.

The present invention further relates to therapies or (therapeutic or preventive or protective) methods or uses as described herein, comprising administering (e.g. simultaneously, separately or sequentially) an effective amount of linagliptin and an insulin or insulin analogue (such as e.g. a basal insulin), to the patient (particularly human patient) in need thereof, such as e.g. a patient as described herein, preferably a human diabetic (particularly type 2 diabetes patient);
including e.g. a patient having or being at high risk of a cardiovascular and/or renal microvascular disease, such as e.g. a patient at high vascular risk (e.g. at high risk of CV events), e.g. as described herein; such as e.g. a patient according to at least one of the embodiments 1 to 6 or 1 to 7 as described herein.

The present invention further relates to therapies or (therapeutic or preventive or protective) methods or uses as described herein, comprising administering (e.g. simultaneously, separately or sequentially) an effective amount of linagliptin and telmisartan, to the patient (particularly human patient) in need thereof, such as e.g. a patient as described herein, preferably a human diabetic (particularly type 2 diabetes patient);
including e.g. a patient having or being at high risk of a cardiovascular and/or renal microvascular disease, such as e.g. a patient at high vascular risk (e.g. at high risk of CV events), e.g. as described herein; such as e.g. a patient according to at least one of the embodiments 1 to 6 or 1 to 7 as described herein.

Further, the present invention relates to a method of therapy (e.g. treatment, prevention, protection) of a patient (particularly a human patient, who may suffer from diabetes, e.g. type 1 or type 2 diabetes or LADA, particularly type 2 diabetes); such as e.g. a patient having or being at high risk of a cardiovascular and/or renal microvascular disease, such as e.g. a patient at high vascular risk (e.g. at high risk of CV events), e.g. as described herein; particularly a patient according to at least one of the embodiments 1 to 6 or 1 to 7 as described herein;
such as e.g. a patient having:
both
albuminuria (e.g. micro- or macro-albuminuria)
   and
previous macrovascular (e.g. cardio- or cerebrovascular) disease (such as e.g. myocardial infarction, coronary artery disease, (ischemic or haemorrhagic) stroke, carotid artery disease and/or peripheral artery disease),
   and/or
either
   (mild or moderate) renal impairment (e.g. CKD stage 1, 2 or 3, such as CKD stage 1, 2 (mild) or 3a (mild-moderate), preferably eGFR ≥ 45-75 mL/min/1.73 m²) with macro-albuminuria,
   or
(moderate or severe) renal impairment (e.g. CKD stage 3 or 4, such as CKD stage 3b (moderate-severe) or 4 (severe), preferably eGFR 15-45 mL/min/1.73 m²), with or without any albuminuria (such as e.g. with or without micro- or macro-albuminuria);
said method comprising administering an effective amount of linagliptin, optionally in combination with one or more other active agents (such as selected from those described hereinabove or hereinbelow; e.g. selected from other antidiabetic substances, active substances that lower the blood sugar level, active substances that lower the lipid level in the blood, active substances that raise the HDL level in the blood, active substances that lower blood pressure, active substances that are indicated in the treatment of atherosclerosis or obesity, and/or active substances which are indicated in the treatment or prevention of major CV events and/or antiplatelet agents and/or anticoagulants), to the patient.

Further, the present invention relates to a method of:
preventing, protecting against, reducing the risk of and/or delaying the occurrence of:
a cardio- or cerebrovascular disease or event, such as e.g. selected from cardiovascular (CV) death (including fatal stroke, fatal myocardial infarction and sudden death), non-fatal stroke, non-fatal myocardial infarction (MI) (silent MI may be excluded) and hospitalisation for unstable angina pectoris,
   and
preventing, protecting against, reducing the risk of and/or delaying the occurrence of:
   a renal microvascular disease, such as e.g. selected from renal death, end-stage renal disease and loss in estimated glomerular filtration rate (e.g. eGFR ≥ 50% from baseline);
   in a patient (particularly a human patient, who may suffer from diabetes, e.g. type 1 or type 2 diabetes or LADA, particularly type 2 diabetes) in need thereof;
   including e.g. a patient having or being at high risk of a cardiovascular and/or renal microvascular disease, such as e.g. a patient at high vascular risk (e.g. at high risk of CV events), e.g. as described herein; such as e.g. a patient according to at least one of the embodiments 1 to 6 or 1 to 7 as described herein;
   such as e.g. a patient having:
      both
      albuminuria (e.g. micro- or macro-albuminuria)
         and
      previous macrovascular (e.g. cardio- or cerebrovascular) disease (such as e.g. myocardial infarction, coronary artery disease, (ischemic or haemorrhagic) stroke, carotid artery disease and/or peripheral artery disease),
         and/or
      either
         (mild or moderate) renal impairment (e.g. CKD stage 1, 2 or 3, such as CKD stage 1, 2 (mild) or 3a (mild-moderate), preferably eGFR ≥ 45-75 mL/min/1.73 m²) with macro-albuminuria,
         or
      (moderate or severe) renal impairment (e.g. CKD stage 3 or 4, such as CKD stage 3b (moderate-severe) or 4 (severe), preferably eGFR 15-45 mL/min/1.73 m²), with or without any albuminuria (such as e.g. with or without micro- or macro-albuminuria);
      comprising administering an effective amount of linagliptin, optionally in combination with one or more other active agents, e.g. selected from other antidiabetic substances, active substances that lower the blood sugar level, active substances that lower the lipid level in the blood, active substances that raise the HDL level in the blood, active substances that lower blood pressure, active substances that are indicated in the treatment of atherosclerosis or obesity, and/or active substances which are indicated in the treatment or prevention of major CV events and/or antiplatelet agents and/or anticoagulants, to the patient.

Further, the present invention relates to a method of:
preventing, protecting against, reducing the risk of and/or delaying the occurrence of:
a cardio- or cerebrovascular disease or event, such as e.g. selected from cardiovascular (CV) death (including fatal stroke, fatal myocardial infarction and sudden death), non-fatal stroke, non-fatal myocardial infarction (MI) (silent MI may be excluded) and hospitalisation for unstable angina pectoris,
   and
preventing, protecting against, reducing the risk of and/or delaying the occurrence of:
   a renal microvascular disease, such as e.g. selected from renal death, end-stage renal disease and loss in estimated glomerular filtration rate (e.g. eGFR ≥ 50% from baseline);
   in a patient (particularly a human patient, who may suffer from diabetes, e.g. type 1 or type 2 diabetes or LADA, particularly type 2 diabetes) in need thereof;
   including e.g. a patient having or being at high risk of a cardiovascular and/or renal microvascular disease, such as e.g. a patient at high vascular risk (e.g. at high risk of CV events), e.g. as described herein; such as e.g. a patient according to at least one of the embodiments 1 to 6 or 1 to 7 as described herein;
   such as e.g. a patient having:
      both
      albuminuria (e.g. micro- or macro-albuminuria)
         and
      previous macrovascular (e.g. cardio- or cerebrovascular) disease (such as e.g. myocardial infarction, coronary artery disease, (ischemic or haemorrhagic) stroke, carotid artery disease and/or peripheral artery disease),
         and/or
      either
         (mild or moderate) renal impairment (e.g. CKD stage 1, 2 or 3, such as CKD stage 1, 2 (mild) or 3a (mild-moderate), preferably eGFR ≥ 45-75 mL/min/1.73 m²) with macro-albuminuria,
         or
      (moderate or severe) renal impairment (e.g. CKD stage 3 or 4, such as CKD stage 3b (moderate-severe) or 4 (severe), preferably eGFR 15-45 mL/min/1.73 m²), with or without any albuminuria (such as e.g. with or without micro- or macro-albuminuria);
      comprising administering an effective amount of linagliptin and, optionally, one or more other antidiabetics selected from the group consisting of metformin, a sulphonylurea, nateglinide, repaglinide, a thiazolidinedione, a PPAR-gamma-agonist, an alpha-glucosidase inhibitor, insulin or an insulin analogue, and GLP-1 or a GLP-1 analogue, and, optionally, in combination with one or more further active agents (e.g. a diuretic, an ACE inhibitor and/or an ARB such as e.g. telmisartan), to the patient.

Further, the present invention relates to a certain DPP-4 inhibitor, preferably linagliptin (optionally in combination with one or more other active agents) for use in the treatment of diabetic nephropathy, particularly diabetic nephropathy with an elevated serum creatinine and proteinuria (>300 mg/day) in patients with type 2 diabetes, such as e.g. including those patients having or being at-risk of a cardio- or cerebrovascular and/or renal (micro)vascular disease, event, complication or condition such as described herein (such as e.g. a patient at high vascular risk e.g. as defined herein); such as e.g. in those patients according to at least one of the embodiments 1 to 6 or 1 to 7 as described herein.

Further, the present invention relates to a certain DPP-4 inhibitor, preferably linagliptin (optionally in combination with one or more other active agents) for use in treating or lowering albuminuria or diabetic nephropathy on top of angiotensin-converting enzyme (ACE) inhibitor therapy and/or angiotensin II receptor blockade (ARB) therapy preferably in type 2 diabetes patients, such as e.g. including those patients having or being at-risk of a cardio- or cerebrovascular and/or renal (micro)vascular disease, event, complication or condition such as described herein (such as e.g. a patient at high vascular risk e.g. as defined herein); such as e.g. in those patients according to at least one of the embodiments 1 to 6 or 1 to 7 as described herein.

Further, the present invention relates to a certain DPP-4 inhibitor, preferably linagliptin (optionally in combination with one or more other active agents, such as e.g. including an ARB or ACE inhibitor, such as e.g. with or without additional standard background therapy such as e.g. with an ACEi or ARB) for use in preventing, reducing the risk or likelihood of or delaying the onset or slowing the progression of renal morbidity and/or mortality, preferably in type 2 diabetes patients, such as e.g. including those patients having or being at-risk of a cardio- or cerebrovascular and/or renal (micro)vascular disease, event, complication or condition such as described herein (such as e.g. a patient at high vascular risk e.g. as defined herein); such as e.g. in those patients according to at least one of the embodiments 1 to 6 or 1 to 7 as described herein.

Further, the present invention relates to a certain DPP-4 inhibitor, preferably linagliptin (optionally in combination with one or more other active agents) for use in treating, preventing, reducing the risk of or delaying the onset or progression of diabetic nephropathy, micro- or macro-albuminuria, chronic kidney disease (CKD), worsening of CKD, and/or acute renal failure, preferably in type 2 diabetes patients, such as e.g. including those patients having or being at-risk of a cardio- or cerebrovascular and/or renal (micro)vascular disease, event, complication or condition such as described herein (such as e.g. a patient at high vascular risk e.g. as defined herein); such as e.g. in those patients according to at least one of the embodiments 1 to 6 or 1 to 7 as described herein.

Further, the present invention relates to a certain DPP-4 inhibitor, preferably linagliptin (optionally in combination with one or more other active agents) for use in reducing the risk of or delaying the onset or the progression of micro- or macro-albuminuria, the onset of chronic kidney disease (CKD), the worsening of CKD, the onset of acute renal failure and/or of death, preferably in type 2 diabetes patients, such as e.g. including those patients having or being at-risk of a cardio- or cerebrovascular and/or renal (micro)vascular disease, event, complication or condition such as described herein (such as e.g. a patient at high vascular risk e.g. as defined herein); such as e.g. in those patients according to at least one of the embodiments 1 to 6 or 1 to 7 as described herein.

Further, the present invention relates to a certain DPP-4 inhibitor, preferably linagliptin ( optionally in combination with one or more other active agents, such as e.g. one or more antidiabetics, and/or optionally in combination with one or more further active agents, such as e.g. one or more antiplatelet agents, antihypertensive and/or lipid lowering agents) for use in preventing, reducing the risk of or delaying the onset or slowing the progression of renal morbidity and/or mortality, such as preventing, reducing or delaying the onset or progression of micro- or macro-albuminuria, the onset of chronic kidney disease (CKD), the worsening of CKD, and/or the onset of acute renal failure and/or of death, particularly in a human patient having diabetes, especially type 2 diabetes mellitus, and/or for use in treating, lowering, preventing, reducing the risk of, delaying the onset or slowing the progression of albuminuria (micro- or macro-albuminuria) or diabetic nephropathy, particularly in a human patient having diabetes, especially type 2 diabetes mellitus; such as e.g. including such patient having or being at-risk of a cardio- or cerebrovascular and/or renal (micro)vascular disease, event, complication or condition such as described herein (such as e.g. a patient at high vascular risk e.g. as defined herein); such as e.g. in such patient according to at least one of the embodiments 1 to 6 or 1 to 7 as described herein.

Further, the present invention relates to a certain DPP-4 inhibitor, preferably linagliptin (optionally in combination with one or more other active agents) for use in a method of treating, preventing, protecting against, reducing the risk of, delaying the occurrence or slowing the progression of:
a cardio- or cerebrovascular disease, complication or event, such as e.g. selected from cardiovascular (CV) death (including fatal stroke, fatal myocardial infarction and sudden death), non-fatal stroke, non-fatal myocardial infarction (MI) (silent MI may be excluded) and hospitalisation for unstable angina pectoris,
   and/or
a renal microvascular disease or complication, such as e.g. selected from nephropathy, (micro-or macro)albuminuria, chronic kidney disease (CKD), renal death, end-stage renal disease, renal impairment and loss in estimated glomerular filtration rate (e.g. eGFR ≥ 50% from baseline);
in patients (particularly human patients, who may suffer from diabetes, e.g. type 1 or type 2 diabetes or LADA, particularly type 2 diabetes) in need thereof, preferably in type 2 diabetes patients, such as e.g. including those patients having or being at-risk of a cardio- or cerebrovascular and/or renal (micro)vascular disease, event, complication or condition such as described herein (such as e.g. patients at high vascular risk e.g. as defined herein); such as e.g. in those patients according to at least one of the embodiments 1 to 6 or 1 to 7 as described herein; and/or e.g. including those patients with inadequate control of albuminuria despite therapy with an angiotensin-converting enzyme (ACE) inhibitor and/or an angiotensin II receptor blocker (ARB); particularly this method comprising administering the DPP-4 inhibitor in combination with the angiotensin-converting enzyme (ACE) inhibitor and/or the angiotensin II receptor blocker (ARB) to the patient.

Further, the present invention relates to a certain DPP-4 inhibitor, preferably linagliptin (optionally in combination with one or more other active agents, such as, e.g., another antidiabetic, and/or an ARB and/or an ACE inhibitor) for use in preventing, protecting against, reducing the risk of or delaying the onset or slowing the progression of renal and/or cardiovascular morbidity and/or mortality, preferably of both renal and cardiovascular morbidity and/or mortality, preferably in type 2 diabetes patients,
such as e.g. including those patients having or being at-risk of a cardio- or cerebrovascular and/or renal (micro)vascular disease, event, complication or condition such as described herein (such as e.g. patients at high vascular risk e.g. as defined herein); such as e.g. in those patients according to at least one of the embodiments 1 to 6 or 1 to 7 as described herein;
and/or e.g. including those patients with inadequate control of albuminuria despite therapy with an angiotensin-converting enzyme (ACE) inhibitor and/or an angiotensin II receptor blocker (ARB).

Further, the present invention relates to a certain DPP-4 inhibitor, preferably linagliptin (optionally in combination with one or more other active agents) for use in treating, preventing, reducing the risk of or delaying the onset or progression of nephropathy, micro-or macro-albuminuria, chronic kidney disease (CKD), worsening of CKD, renal impairment and/or acute renal failure, preferably in type 2 diabetes patients,
such as e.g. including those patients having or being at-risk of a cardio- or cerebrovascular and/or renal (micro)vascular disease, event, complication or condition such as described herein (such as e.g. patients at high vascular risk e.g. as defined herein); such as e.g. in those patients according to at least one of the embodiments 1 to 6 or 1 to 7 as described herein;
and/or including e.g. those patients with inadequate control of albuminuria despite therapy with an angiotensin-converting enzyme (ACE) inhibitor and/or an angiotensin II receptor blocker (ARB); particularly this method comprising administering the DPP-4 inhibitor in combination with the angiotensin-converting enzyme (ACE) inhibitor and/or the angiotensin II receptor blocker (ARB) to the patient.

Further, the present invention relates to a certain DPP-4 inhibitor, preferably linagliptin (optionally in combination with one or more other active agents) for use in reducing the risk of or delaying the onset or the progression of micro- or macro-albuminuria, the onset of chronic kidney disease (CKD), the worsening of CKD, the onset of acute renal failure and/or of death, preferably in type 2 diabetes patients,
such as e.g. including those patients having or being at-risk of a cardio- or cerebrovascular and/or renal (micro)vascular disease, event, complication or condition such as described herein (such as e.g. those patients at high vascular risk e.g. as defined herein); such as e.g. in those patients according to at least one of the embodiments 1 to 6 or 1 to 7 as described herein;
and/or including e.g. those patients with inadequate control of albuminuria despite therapy with an angiotensin-converting enzyme (ACE) inhibitor and/or an angiotensin II receptor blocker (ARB); particularly this method comprising administering the DPP-4 inhibitor in combination with the angiotensin-converting enzyme (ACE) inhibitor and/or the angiotensin II receptor blocker (ARB) to the patient.

Further, the present invention relates to a certain DPP-4 inhibitor, preferably linagliptin (optionally in combination with one or more other active agents, such as e.g. one or more antidiabetics, and/or optionally in combination with one or more further active agents, such as e.g. selected from antiplatelet agents, antihypertensive agents and/or lipid lowering agents) for use in preventing, protecting against, reducing the risk of or delaying the onset or slowing the progression of renal and/or cardiovascular morbidity and/or mortality, preferably of both renal and cardiovascular morbidity and/or mortality, particularly in a human patient with diabetes, especially type 2 diabetes mellitus,
such as e.g. including those patients having or being at-risk of a cardio- or cerebrovascular and/or renal (micro)vascular disease, event, complication or condition such as described herein (such as e.g. those patients at high vascular risk e.g. as defined herein);
such as, e.g., in those patients according to at least one of the embodiments 1 to 6 or 1 to 7 as described herein;
such as e.g. including patients on (ongoing) therapy with antiplatelet medication such as e.g. acetylsalicylic acid, and/or on (ongoing) therapy with an antihypertensive medication, such as e.g. an ACE inhibitor, ARB, beta-blocker, Calcium-antagonist or diuretic, and/or on (ongoing) therapy with a lipid lowering medication, such as e.g. a fibrate, niacin or statin; or a combination thereof.

In certain embodiments, duration of treatment with a certain DPP-4 inhibitor, particularly linagliptin (preferably 5 mg per day, administered orally, optionally in combination with one or more other active substances, e.g. such as those described herein) in the therapies according to the present invention (such as e.g. including or relating to primary or, particularly, secondary prevention of CV events) may be over a lengthy period (e.g. such as described herein), such as to optimize cardio- and/or renoprotective therapy and/or to improve cardiovascular and/or renal morbidity and/or mortality in a human patient, particularly human type 2 diabetes patient; such as e.g. (e.g. for secondary prevention of CV events) in a patient having or being at high risk of cardiovascular and/or renal microvascular disease, such as e.g. a patient at high vascular risk (such as described herein); such as e.g. a patient according to at least one of the embodiments 1 to 6 or 1 to 7 as described herein).

For example, a therapy according to the present invention may relate to at least 3 years duration of treatment with a certain DPP-4 inhibitor, particularly linagliptin (preferably 5 mg per day, administered orally, optionally in combination with one or more other active substances) for primary or secondary prevention of CV events, or to reduce the risk thereof.

For further example, a therapy according to the present invention may relate to at least 3 years duration of treatment with a certain DPP-4 inhibitor, particularly linagliptin (preferably 5 mg per day, administered orally, optionally in combination with one or more other active substances) for primary or secondary prevention of CV events (or to reduce the risk thereof) (e.g. at least 3-4 years or 5-6 years for secondary prevention, at least 3-6 years or 7-10 years for primary prevention), such as e.g. for decreasing, preventing, protecting against, delaying (e.g. occurrence or progression) and/or reducing the risk of: CV morbidity and/or (premature) CV mortality, such as e.g. one or more selected from: CV death (e.g. fatal myocardial infarction, fatal stroke, fatal heart failure, cardiogenic shock, or sudden cardiac death), non-fatal myocardial infarction, non-fatal stroke (e.g. (intracranial) hemorrhagic or non-hemorrhagic stroke, and/or silent or non-silent) and/or unstable angina pectoris (e.g. hospitalization for unstable angina pectoris), and/or, optionally, stable angina pectoris, transient ischemic attack, congestive heart failure, peripheral revascularization procedures and/or coronary revascularization procedures (such as e.g. hospitalization for any of such morbidity).

In an embodiment, a therapy according to the present invention may relate to at least 3-4 years duration of treatment with a certain DPP-4 inhibitor, particularly linagliptin (preferably 5 mg per day, administered orally, optionally in combination with one or more other active substances) for secondary prevention of CV events (or to reduce the risk thereof).

In an embodiment, a therapy according to the present invention may relate to at least 3-6 years duration of treatment with a certain DPP-4 inhibitor, particularly linagliptin (preferably 5 mg per day, administered orally, optionally in combination with one or more other active substances) for primary prevention of CV events (or to reduce the risk thereof).

In a further embodiment, a therapy according to the present invention may relate to at least 5-6 years duration of treatment with a certain DPP-4 inhibitor, particularly linagliptin (preferably 5 mg per day, administered orally, optionally in combination with one or more other active substances) for secondary prevention of CV events (or to reduce the risk thereof).

In a further embodiment, a therapy according to the present invention may relate to at least 7-10 years duration of treatment with a certain DPP-4 inhibitor, particularly linagliptin (preferably 5 mg per day, administered orally, optionally in combination with one or more other active substances) for primary prevention of CV events (or to reduce the risk thereof).

Primary prevention of CV events may relate, for example, to protection from (or reducing the risk of) developing the disease, such as in patients who are CV healthy. Secondary prevention of CV events may relate, for example, to delaying the onset, to slowing, or to protection from (or reducing the risk of) progression of the disease, such as in patients who are at-risk of CV events/disease or have CV disease.

A DPP-4 inhibitor within the meaning of the present invention includes, without being limited to, any of those DPP-4 inhibitors mentioned hereinabove and hereinbelow, preferably orally active DPP-4 inhibitors.

In a first embodiment (embodiment A), a DPP-4 inhibitor in the context of the present invention is any DPP-4 inhibitor of 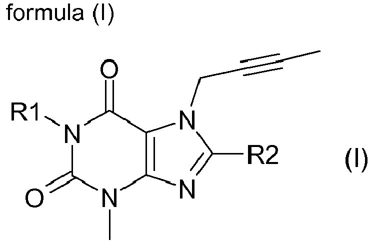 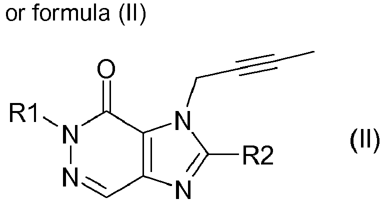 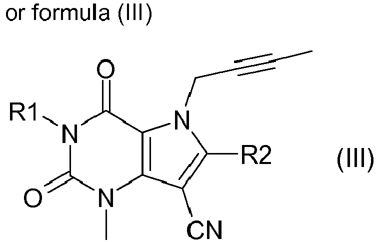 or 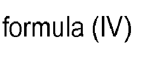 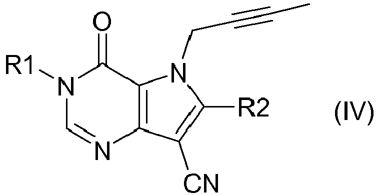 wherein **R1** denotes ([1,5]naphthyridin-2-yl)methyl, (quinazolin-2-yl)methyl, (quinoxalin-6-yl)methyl, (4-methyl-quinazolin-2-yl)methyl, 2-cyano-benzyl, (3-cyano-quinolin-2-yl)methyl, (3-cyano-pyridin-2-yl)methyl, (4-methyl-pyrimidin-2-yl)methyl, or (4,6-dimethyl-pyrimidin-2-yl)methyl and **R2** denotes 3-(*R*)-amino-piperidin-1-yl, (2-amino-2-methyl-propyl)-methylamino or (2-(*S*)-amino-propyl)-methylamino,
or its pharmaceutically acceptable salt.

Regarding the first embodiment (embodiment **A**), preferred DPP-4 inhibitors are any or all of the following compounds and their pharmaceutically acceptable salts:
- 1-[(4-methyl-quinazolin-2-yl)methyl]-3-methyl-7-(2-butyn-1-yl)-8-(3-(*R*)-amino-piperidin-1-yl)-xanthine (compare WO 2004/018468, example 2(142)): 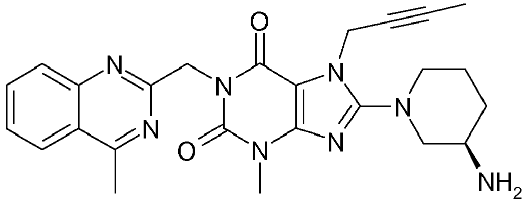
- 1-[([1,5]naphthyridin-2-yl)methyl]-3-methyl-7-(2-butyn-1-yl)-8-((*R*)-3-amino-piperidin-1-yl)-xanthine (compare WO 2004/018468, example 2(252)): 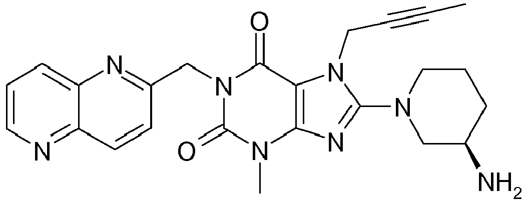
- 1-[(Quinazolin-2-yl)methyl]-3-methyl-7-(2-butyn-1-yl)-8-((*R*)-3-amino-piperidin-1-yl)-xanthine (compare WO 2004/018468, example 2(80)): 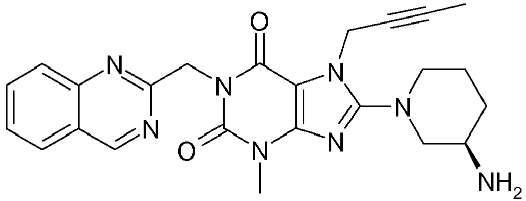
- 2-((R)-3-Amino-piperidin-1-yl)-3-(but-2-yinyl)-5-(4-methyl-quinazolin-2-ylmethyl)-3,5-dihydro-imidazo[4,5-d]pyridazin-4-one (compare WO 2004/050658, example 136): 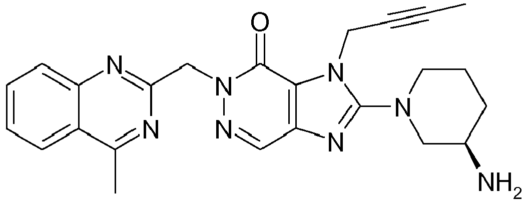
- 1-[(4-Methyl-quinazolin-2-yl)methyl]-3-methyl-7-(2-butyin-1-yl)-8-[(2-amino-2-methylpropyl)-methylamino]-xanthine (compare WO 2006/029769, example 2(1)): 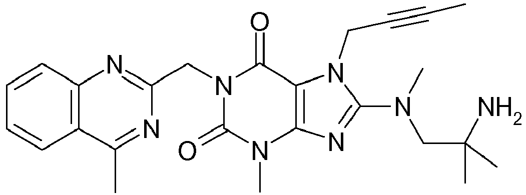
- 1-[(3-Cyano-quinolin-2-yl)methyl]-3-methyl-7-(2-butyn-1-yl)-8-((*R*)-3-amino-piperidin-1-yl)-xanthine (compare WO 2005/085246, example 1(30)): 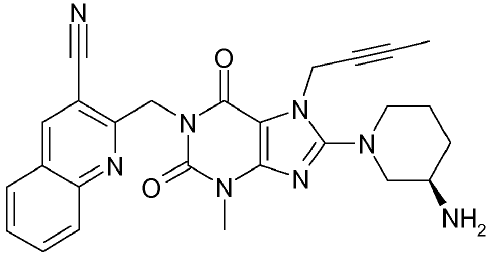
- 1-(2-Cyano-benzyl)-3-methyl-7-(2-butyn-1-yl)-8-((*R*)-3-amino-piperidin-1-yl)-xanthine (compare WO 2005/085246, example 1(39)): 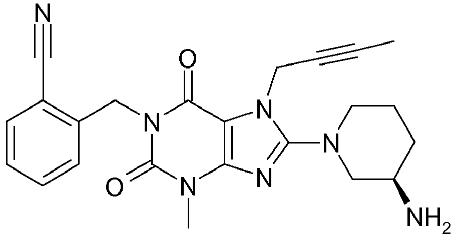
- 1-[(4-Methyl-quinazolin-2-yl)methyl]-3-methyl-7-(2-butyn-1-yl)-8-[(*S*)-(2-amino-propyl)-methylamino]-xanthine (compare WO 2006/029769, example 2(4)): 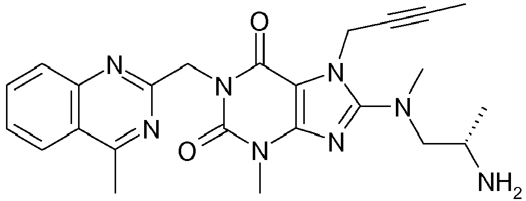
- 1-[(3-Cyano-pyridin-2-yl)methyl]-3-methyl-7-(2-butyn-1-yl)-8-((*R*)-3-amino-piperidin-1-yl)-xanthine (compare WO 2005/085246, example 1(52)): 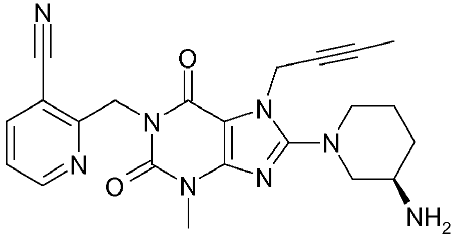
- 1-[(4-Methyl-pyrimidin-2-yl)methyl]-3-methyl-7-(2-butyn-1-yl)-8-((*R*)-3-amino-piperidin-1-yl)-xanthine (compare WO 2005/085246, example 1(81)): 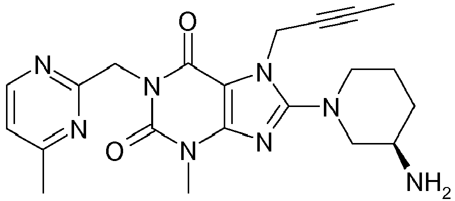
- 1-[(4,6-Dimethyl-pyrimidin-2-yl)methyl]-3-methyl-7-(2-butyn-1-yl)-8-((*R*)-3-amino-piperidin-1-yl)-xanthine (compare WO 2005/085246, example 1(82)): 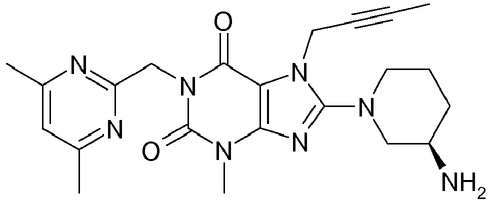
- 1-[(Quinoxalin-6-yl)methyl]-3-methyl-7-(2-butyn-1-yl)-8-((*R*)-3-amino-piperidin-1-yl)-xanthine (compare WO 2005/085246, example 1(83)):

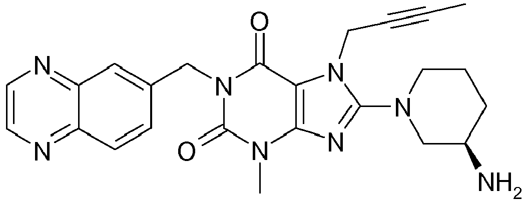

These DPP-4 inhibitors are distinguished from structurally comparable DPP-4 inhibitors, as they combine exceptional potency and a long-lasting effect with favourable pharmacological properties, receptor selectivity and a favourable side-effect profile or bring about unexpected therapeutic advantages or improvements when combined with other pharmaceutical active substances. Their preparation is disclosed in the publications mentioned.

In a second embodiment (embodiment **B**), a DPP-4 inhibitor in the context of the present invention is a DPP-4 inhibitor selected from the group consisting of
sitagliptin, vildagliptin, saxagliptin, alogliptin, gemigliptin, omarigliptin, evogliptin,
(2S)-1-{[2-(5-Methyl-2-phenyl-oxazol-4-yl)-ethylamino]-acetyl}-pyrrolidine-2-carbonitrile,
(2S)-1-{[1,1,-Dimethyl-3-(4-pyridin-3-yl-imidazol-1-yl)-propylamino]-acetyl}-pyrrolidine-2-carbonitrile,
(S)-1-((2S,3S,11bS)-2-Amino-9,10-dimethoxy-1,3,4,6,7,11b-hexahydro-2H-pyrido[2,1-a]isoquinolin-3-yl)-4-fluoromethyl-pyrrolidin-2-one,
(3,3-Difluoropyrrolidin-1 -yl)-((2S,4S)-4-(4-(pyrimidin-2-yl)piperazin-1 -yl)pyrrolidin-2-yl)methanone,
(1((3S,4S)-4-amino-1-(4-(3,3-difluoropyrrolidin-1-yl)-1,3,5-triazin-2-yl)pyrrolidin-3-yl)-5,5-difluoropiperidin-2-one,
(2S,4S)-1-{2-[(3S,1R)-3-(1H-1,2,4-Triazol-1-ylmethyl)cyclopentylamino]-acetyl}-4-fluoropyrrolidine-2-carbonitrile,
(R)-2-[6-(3-Amino-piperidin-1-yl)-3-methyl-2,4-dioxo-3,4-dihydro-2H-pyrimidin-1-ylmethyl]-4-fluoro-benzonitrile,
5-{(S)-2-[2-((S)-2-cyano-pyrrolidin-1-yl)-2-oxo-ethylamino]-propyl}-5-(1H-tetrazol-5-yl)-10,11-dihydro-5H-dibenzo[a,d]cycloheptene-2,8-dicarboxylic acid bis-dimethylamide,
3-{(2S,4S)-4-[4-(3-Methyl-1-phenyl-1H-pyrazol-5-yl)piperazin-1-yl]pyrrolidin-2-ylcarbonyl}thiazolidine,
[(2R)-1-{[(3R)-pyrrolidin-3-ylamino]acetyl}pyrrolidin-2-yl]boronic acid,
(2S,4S)-1-[2-[(4-ethoxycarbonylbicyclo[2.2.2]oct-1-yl)amino]acetyl]-4-fluoropyrrolidine-2-carbonitrile,
2-({6-[(3R)-3-amino-3-methylpiperidin-1-yl]-1,3-dimethyl-2,4-dioxo-1,2,3,4-tetrahydro-5H-pyrrolo[3,2-d]pyrimidin-5-yl}methyl)-4-fluorobenzonitrile,
6-[(3R)-3-amino-piperidin-1-yl]-5-(2-chloro-5-fluoro-benzyl)-1,3-dimethyl-1,5-dihydro-pyrrolo[3,2-d]pyrimidine-2,4-dione, and
(S)-2-methylpyrazolo[1,5-a]primidine-6-carboxylic acid {2-[(2-cyanopyrrolidin-1-yl)-2-oxoethylamino]-2-methylpropyl}amide,
or its pharmaceutically acceptable salt.

A more preferred DPP-4 inhibitor among the abovementioned DPP-4 inhibitors of embodiment **A** of this invention is 1-[(4-methyl-quinazolin-2-yl)methyl]-3-methyl-7-(2-butyn-1-yl)-8-(3-(*R*)-amino-piperidin-1-yl)-xanthine, particularly the free base thereof (which is also known as linagliptin or BI 1356).

The DPP-4 inhibitor of this invention may be selected from the group consisting of linagliptin, sitagliptin, vildagliptin, alogliptin, saxagliptin, teneligliptin, anagliptin and gemigliptin, or a pharmaceutically acceptable salt of one of the herein mentioned DPP-4 inhibitors, or a prodrug thereof.

A particularly preferred DPP-4 inhibitor to be emphasized within the present invention is linagliptin. The term "linagliptin" as employed herein refers to linagliptin or a pharmaceutically acceptable salt thereof, including hydrates and solvates thereof, and crystalline forms thereof, preferably linagliptin refers to 1-[(4-methyl-quinazolin-2-yl)methyl]-3-methyl-7-(2-butyn-1-yl)-8-(3-(*R*)-amino-piperidin-1-yl)-xanthine. Crystalline forms are described in WO 2007/128721. Methods for the manufacture of linagliptin are described in the patent applications WO 2004/018468 and WO 2006/048427 for example. Linagliptin is distinguished from structurally comparable DPP-4 inhibitors, as it combines exceptional potency and a long-lasting effect with favourable pharmacological properties, receptor selectivity and a favourable side-effect profile or bring about unexpected therapeutic advantages or improvements in mono- or dual or triple combination therapy.

For avoidance of any doubt, the disclosure of each of the foregoing and following documents cited above in connection with the specified DPP-4 inhibitors is specifically incorporated herein by reference in its entirety.

An embodiment of this invention refers to a DPP-4 inhibitor suitable for use in patients, wherein said patients further suffering from renal disease, renal dysfunction or renal impairment, particularly characterized in that said DPP-4 inhibitor is administered to said patients in the same dose levels as to patients with normal renal function, thus e.g. said DPP-4 inhibitor does not require downward dosing adjustment for impaired renal function.

For example, a DPP-4 inhibitor according to this invention (especially one which may be suited for patients with impaired renal function) may be such an oral DPP-4 inhibitor, which and whose active metabolites have preferably a relatively wide (e.g. about > 100 fold) therapeutic window and/or, especially, that are primarily eliminated via hepatic metabolism or biliary excretion (preferably without adding additional burden to the kidney).

In more detailed example, a DPP-4 inhibitor according to this invention (especially one which may be suited for patients with impaired renal function) may be such an orally administered DPP-4 inhibitor, which has a relatively wide (e.g. > 100 fold) therapeutic window (preferably a safety profile comparable to placebo) and/or which fulfils one or more of the following pharmacokinetic properties (preferably at its therapeutic oral dose levels):
- The DPP-4 inhibitor is substantially or mainly excreted via the liver (e.g. > 80 % or even > 90 % of the administered oral dose), and/or for which renal excretion represents no substantial or only a minor elimination pathway (e.g. < 10 %, preferably < 7 %, of the administered oral dose measured, for example, by following elimination of a radiolabelled carbon (¹⁴C) substance oral dose);
- The DPP-4 inhibitor is excreted mainly unchanged as parent drug (e.g. with a mean of > 70%, or > 80%, or, preferably, 90% of excreted radioactivity in urine and faeces after oral dosing of radiolabelled carbon (¹⁴C) substance), and/or which is eliminated to a non-substantial or only to a minor extent via metabolism (e.g. < 30%, or < 20%, or, preferably, 10%);
- The (main) metabolite(s) of the DPP-4 inhibitor is/are pharmacologically inactive. Such as e.g. the main metabolite does not bind to the target enzyme DPP-4 and, optionally, it is rapidly eliminated compared to the parent compound (e.g. with a terminal half-life of the metabolite of ≤ 20 h, or, preferably, ≤ about 16 h, such as e.g. 15.9 h).

In one embodiment, the (main) metabolite in plasma (which may be pharmacologically inactive) of a DPP-4 inhibitor having a 3-amino-piperidin-1-yl substituent is such a derivative where the amino group of the 3-amino-piperidin-1-yl moiety is replaced by a hydroxyl group to form the 3-hydroxy-piperidin-1-yl moiety (e.g. the 3-(S)-hydroxy-piperidin-1-yl moiety, which is formed by inversion of the configuration of the chiral center).

Further properties of a DPP-4 inhibitor according to this invention may be one or more of the following: Rapid attainment of steady state (e.g. reaching steady state plasma levels (> 90% of the steady state plasma concentration) between second and fifth day of treatment with therapeutic oral dose levels), little accumulation (e.g. with a mean accumulation ratio R_{A,AUC} ≤ 1.4 with therapeutic oral dose levels), and/or preserving a long-lasting effect on DPP-4 inhibition, preferably when used once-daily (e.g. with almost complete (> 90%) DPP-4 inhibition at therapeutic oral dose levels, > 80% inhibition over a 24h interval after once-daily intake of therapeutic oral drug dose), significant decrease in 2h postprandial blood glucose excursions by ≥ 80 % (already on first day of therapy) at therapeutic dose levels, and cumulative amount of unchanged parent compound excreted in urine on first day being below 1% of the administered dose and increasing to not more than about 3-6% in steady state.

Thus, for example, a DPP-4 inhibitor according to this invention may be characterized in that said DPP-4 inhibitor has a primarily non-renal route of excretion, i.e. said DPP-4 inhibitor is excreted to a non-substantial or only to a minor extent (e.g. < 10 %, preferably < 7 %, e.g. about 5 %, of administered oral dose, preferably of oral therapeutic dose) via the kidney (measured, for example, by following elimination of a radiolabelled carbon (¹⁴C) substance oral dose).

Further, a DPP-4 inhibitor according to this invention may be characterized in that said DPP-4 inhibitor is excreted substantially or mainly via the liver, bile or faeces (measured, for example, by following elimination of a radiolabelled carbon (¹⁴C) substance oral dose).

Further, a DPP-4 inhibitor according to this invention may be characterized in that
said DPP-4 inhibitor is excreted mainly unchanged as parent drug (e.g. with a mean of > 70%, or > 80%, or, preferably, 90 % of excreted radioactivity in urine and faeces after oral dosing of radiolabelled carbon (¹⁴C) substance),
said DPP-4 inhibitor is eliminated to a non-substantial or only to a minor extent via metabolism, and/or
the main metabolite of said DPP-4 inhibitor is pharmacologically inactive or has a relatively wide therapeutic window.

Further, a DPP-4 inhibitor according to this invention may be characterized in that
said DPP-4 inhibitor does not significantly impair glomerular and/or tubular function of a type 2 diabetes patient with chronic renal insufficiency (e.g. mild, moderate or severe renal impairment or end stage renal disease), and/or
said DPP-4 inhibitor trough levels in the blood plasma of type 2 diabetes patients with mild or moderate renal impairment are comparable to the levels in patients with normal renal function, and/or
said DPP-4 inhibitor does not require to be dose-adjusted in a type 2 diabetes patient with impaired renal function (e.g. mild, moderate or severe renal impairment or end stage renal disease, preferably regardless of the stage of renal impairment).

Further, a DPP-4 inhibitor according to this invention may be characterized in that
said DPP-4 inhibitor provides its minimally effective dose at that dose that results in >50% inhibition of DPP-4 activity at trough (24 h after last dose) in >80% of patients, and/or
said DPP-4 inhibitor provides its fully therapeutic dose at that dose that results in >80% inhibition of DPP-4 activity at trough (24 h after last dose) in >80% of patients.

Further, a DPP-4 inhibitor according to this invention may be characterized in that being suitable for use in type 2 diabetes patients who are with diagnosed renal disease, impairment or complication and/or who are at-risk of developing renal disease, impairment or complications, e.g. patients with or at-risk of diabetic nephropathy (including chronic and progressive renal insufficiency, albuminuria, proteinuria, fluid retention in the body (edema) and/or hypertension).

Unless otherwise noted, according to this invention it is to be understood that the definitions of the active agents (including the DPP-4 inhibitors) mentioned hereinabove and herein below may also contemplate their pharmaceutically acceptable salts, and prodrugs, hydrates, solvates and polymorphic forms thereof. Particularly the terms of the therapeutic agents given herein refer to the respective active drugs. With respect to salts, hydrates and polymorphic forms thereof, particular reference is made to those which are referred to herein.

Within this invention it is to be understood that the combinations, compositions or combined uses according to this invention may envisage the simultaneous, sequential or separate administration of the active components or ingredients.

In this context, "combination" or "combined" within the meaning of this invention may include, without being limited, fixed and non-fixed (e.g. free) forms (including kits) and uses, such as e.g. the simultaneous, sequential or separate use of the components or ingredients.

The combined administration of this invention may take place by administering the active components or ingredients together, such as e.g. by administering them simultaneously in one single or in two separate formulations or dosage forms. Alternatively, the administration may take place by administering the active components or ingredients sequentially, such as e.g. successively in two separate formulations or dosage forms.

For the combination therapy of this invention the active components or ingredients may be administered separately (which implies that they are formulated separately) or formulated altogether (which implies that they are formulated in the same preparation or in the same dosage form). Hence, the administration of one element of the combination of the present invention may be prior to, concurrent to, or subsequent to the administration of the other element of the combination.

Unless otherwise noted, combination therapy may refer to first line, second line or third line therapy, or initial or add-on combination therapy or replacement therapy.

Unless otherwise noted, monotherapy may refer to first line therapy (e.g. therapy of patients with insufficient glycemic control by diet and exercise alone, such as e.g. drug-naive patients, typically patients early after diagnosis and/or who have not been previously treated with an antidiabetic agent, and/or patients ineligible for metformin therapy such as e.g. patients for whom metformin therapy is contraindicated, such as e.g. due to renal impairment, or inappropriate, such as e.g. due to intolerance).

Unless otherwise noted, add-on combination therapy may refer to second line or third line therapy (e.g. therapy of patients with insufficient glycemic control despite (diet and exercise plus) therapy with one or two conventional antidiabetic agents, typically patients who are pre-treated with one or two antidiabetic agents, such as e.g. patients with such existing antidiabetic background medication).

Unless otherwise noted, initial combination therapy may refer to first line therapy (e.g. therapy of patients with insufficient glycemic control by diet and exercise alone, such as e.g. drug-naive patients, typically patients early after diagnosis and/or who have not been previously treated with an antidiabetic agent).

With respect to embodiment A, the methods of synthesis for the DPP-4 inhibitors according to embodiment **A** of this invention are known to the skilled person. Advantageously, the DPP-4 inhibitors according to embodiment **A** of this invention can be prepared using synthetic methods as described in the literature. Thus, for example, purine derivatives of formula (I) can be obtained as described in WO 2002/068420, WO 2004/018468, WO 2005/085246, WO 2006/029769 or WO 2006/048427, the disclosures of which are incorporated herein. Purine derivatives of formula (II) can be obtained as described, for example, in WO 2004/050658 or WO 2005/110999, the disclosures of which are incorporated herein.

Purine derivatives of formula (III) and (IV) can be obtained as described, for example, in WO 2006/068163, WO 2007/071738 or WO 2008/017670, the disclosures of which are incorporated herein. The preparation of those DPP-4 inhibitors, which are specifically mentioned hereinabove, is disclosed in the publications mentioned in connection therewith. Polymorphous crystal modifications and formulations of particular DPP-4 inhibitors are disclosed in WO 2007/128721 and WO 2007/128724, respectively, the disclosures of which are incorporated herein in their entireties. Formulations of particular DPP-4 inhibitors with metformin or other combination partners are described in WO 2009/121945, the disclosure of which is incorporated herein in its entirety.

Typical dosage strengths of the dual fixed combination (tablet) of linagliptin / metformin IR (immediate release) are 2.5/500 mg, 2.5/850 mg and 2.5/1000 mg, which may be administered 1-3 times a day, particularly twice a day.

Typical dosage strengths of the dual fixed combination (tablet) of linagliptin / metformin XR (extended release) are 5/500 mg, 5/1000 mg and 5/1500 mg (each one tablet) or 2.5/500 mg, 2.5/750 mg and 2.5/1000 mg (each two tablets), which may be administered 1-2 times a day, particularly once a day, preferably to be taken in the evening with meal.

In certain embodiments, the dual fixed combination (tablet) of linagliptin / metformin XR (extended release) (e.g. 5/1000 mg, 2.5/1000 mg or 2.5/750 mg dosage strength) is administered under fed conditions or under fasted conditions (such as e.g. once daily).

The present invention further provides a DPP-4 inhibitor as defined herein for use in (add-on or initial) combination therapy with metformin (e.g. in a total daily amount from 500 to 2000 mg metformin hydrochloride (or up to 2500 mg metformin hydrochloride per day), such as e.g. 500 mg, 850 mg or 1000 mg once or twice daily).

For example, the present invention further relates to a DPP-4 inhibitor as defined herein (particularly linagliptin, e.g. in a daily dose of 5 mg, preferably administered once daily) for use in (add-on) combination therapy with metformin (e.g. administered once or twice daily), e.g. in a total daily amount from 500 to 2000 mg (or up to 2500 mg) metformin hydrochloride (e.g. 1000 mg to 2000 mg, or 1500 mg to 2000 mg, such as e.g. 500 mg, 1000 mg, 1500 mg, 2000 mg or 2500 mg daily amount), e.g. administered as 500 mg, 750 mg, 850 mg or 1000 mg once or twice daily, particularly for use in type 2 diabetes patients with inadequate glycemic control despite metformin therapy (e.g. second or third line therapy).

For example, the present invention further relates to a DPP-4 inhibitor as defined herein (particularly linagliptin, e.g. in a daily dose of 5 mg, preferably administered once daily) for use in (initial) combination therapy with metformin (e.g. administered once or twice daily), e.g. in a total daily amount from 500 to 2000 mg (or up to 2500 mg) metformin hydrochloride (e.g. 1000 mg to 2000 mg, or 1500 mg to 2000 mg, such e.g. 500 mg, 1000 mg, 1500 mg, 2000 mg or 2500 mg daily amount), e.g. administered as 500 mg, 750 mg, 850 mg or 1000 mg once or twice daily, particularly for use in (drug-naive,) type 2 diabetes patients with insufficient glycemic control (e.g. first line therapy).

For further example, the present invention relates to a DPP-4 inhibitor as defined herein (particularly linagliptin, e.g. in an oral daily dose of 5 mg, preferably administered once daily) co-administered with metformin, e.g. in a total daily amount of 1000 mg metformin hydrochloride, preferably administered once daily (preferably at evening time), for use in (drug-naive, newly diagnosed) type 2 diabetes patients with insufficient glycemic control on diet and exercise alone (e.g. first line therapy, such as patients on diet and exercise regimen who are not previously treated with an oral antidiabetic therapy or insulin).

For further example, the present invention relates to a DPP-4 inhibitor as defined herein (particularly linagliptin, e.g. in an oral daily dose of 5 mg, preferably administered once daily) co-administered with metformin, e.g. in a total daily amount of 1500 mg to 2000 mg metformin hydrochloride, preferably in immediate release form, preferably administered once daily, for use in (drug-naive, newly diagnosed) type 2 diabetes patients with insufficient glycemic control on diet and exercise alone (e.g. HbA1c levels of 8.5% to 12.0%) (e.g., first line therapy, such as patients on diet and exercise regimen who are not previously treated with an oral antidiabetic therapy or insulin or GLP-1 receptor agonist).

With respect to embodiment **B,** the methods of synthesis for the DPP-4 inhibitors of embodiment **B** are described in the scientific literature and/ or in published patent documents.

The active compounds of this invention may be administered by various ways, for example by oral, buccal, sublingual, enterical, parenteral (e.g., transdermal, intramuscular or subcutaneous), inhalative (e.g., liquid or powder inhalation, aerosol), pulmonary, intranasal (e.g. spray), intraperitoneal, vaginal, rectal, or topical routes of administration and may be formulated, alone or together, in suitable dosage unit formulations containing conventional non-toxic pharmaceutically acceptable carriers, adjuvants and vehicles appropriate for each route of administration.

In an embodiment, the DPP-4 inhibitor according to the invention is preferably administered orally.

Suitable doses and dosage forms of the DPP-4 inhibitors may be determined by a person skilled in the art and may include those described herein or in the relevant references.

For pharmaceutical application in warm-blooded vertebrates, particularly humans, the compounds of this invention are usually used in dosages from 0.001 to 100 mg/kg body weight, preferably at 0.01-15 mg/kg or 0.1-15 mg/kg, in each case 1 to 4 times a day. For this purpose, the compounds, optionally combined with other active substances, may be incorporated together with one or more inert conventional carriers and/or diluents, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethylene glycol, propylene glycol, cetylstearyl alcohol, carboxymethylcellulose or fatty substances such as hard fat or suitable mixtures thereof into conventional galenic preparations such as plain or coated tablets, capsules, powders, suspensions or suppositories.

The pharmaceutical compositions according to this invention comprising the DPP-4 inhibitors as defined herein are thus prepared by the skilled person using pharmaceutically acceptable formulation excipients as described in the art and appropriate for the desired route of administration. Examples of such excipients may include, without being restricted to, diluents, binders, carriers, fillers, lubricants, flow promoters, crystallisation retardants, disintegrants, solubilizers, colorants, pH regulators, surfactants and/or emulsifiers.

Oral formulations or dosage forms of the DPP-4 inhibitor of this invention may be prepared according to known techniques.

A pharmaceutical composition or dosage form (e.g. oral tablet) of a DPP-4 inhibitor according to embodiment **A** of the invention may typically contain as excipients (in addition to an active ingredient), for example: one or more diluents, a binder, a disintegrant, and a lubricant, preferably each as disclosed herein-below. In an embodiment, the disintegrant may be optional.

Examples of suitable diluents for compounds according to embodiment **A** include cellulose powder, calcium hydrogen phosphate, erythritol, low substituted hydroxypropyl cellulose, mannitol, pregelatinized starch or xylitol.

Examples of suitable lubricants for compounds according to embodiment **A** include talc, polyethyleneglycol, calcium behenate, calcium stearate, hydrogenated castor oil or magnesium stearate.

Examples of suitable binders for compounds according to embodiment **A** include copovidone (copolymerisates of vinylpyrrolidon with other vinylderivates), hydroxypropyl methylcellulose (HPMC), hydroxypropylcellulose (HPC), polyvinylpyrrolidon (povidone), pregelatinized starch, or low-substituted hydroxypropylcellulose (L-HPC).

Examples of suitable disintegrants for compounds according to embodiment **A** include corn starch or crospovidone.

Suitable methods of preparing (oral) preparations or dosage forms of the DPP-4 inhibitors according to embodiment **A** of the invention are
- direct tabletting of the active substance in powder mixtures with suitable tabletting excipients;
- granulation with suitable excipients and subsequent mixing with suitable excipients and subsequent tabletting as well as film coating; or
- packing of powder mixtures or granules into capsules.

Suitable granulation methods are
- wet granulation in the intensive mixer followed by fluidised bed drying;
- one-pot granulation;
- fluidised bed granulation; or
- dry granulation (e.g. by roller compaction) with suitable excipients and subsequent tabletting or packing into capsules.

An exemplary composition (e.g. tablet core) of a DPP-4 inhibitor according to embodiment **A** of the invention comprises the first diluent mannitol, pregelatinized starch as a second diluent with additional binder properties, the binder copovidone, the disintegrant corn starch, and magnesium stearate as lubricant; wherein copovidone and/or corn starch may be optional.

A tablet of a DPP-4 inhibitor according to embodiment **A** of the invention may be film coated, preferably the film coat comprises hydroxypropylmethylcellulose (HPMC), polyethylene glycol (PEG), talc, titanium dioxide and iron oxide (e.g. red and/or yellow).

For further details on dosage forms, formulations and administration of DPP-4 inhibitors of this invention, reference is made to scientific literature and/or published patent documents, particularly to those cited herein.

The pharmaceutical compositions (or formulations) may be packaged in a variety of ways. Generally, an article for distribution includes one or more containers that contain the one or more pharmaceutical compositions in an appropriate form. Tablets are typically packed in an appropriate primary package for easy handling, distribution and storage and for assurance of proper stability of the composition at prolonged contact with the environment during storage. Primary containers for tablets may be bottles or blister packs.

A suitable bottle, e.g. for a pharmaceutical composition or combination (tablet) comprising a DPP-4 inhibitor according to embodiment **A** of the invention, may be made from glass or polymer (preferably polypropylene (PP) or high density polyethylene (HD-PE)) and sealed with a screw cap. The screw cap may be provided with a child resistant safety closure (e.g. press-and-twist closure) for preventing or hampering access to the contents by children. If required (e.g. in regions with high humidity), by the additional use of a desiccant (such as e.g. bentonite clay, molecular sieves, or, preferably, silica gel) the shelf life of the packaged composition can be prolonged.

A suitable blister pack, e.g. for a pharmaceutical composition or combination (tablet) comprising a DPP-4 inhibitor according to embodiment **A** of the invention, comprises or is formed of a top foil (which is breachable by the tablets) and a bottom part (which contains pockets for the tablets). The top foil may contain a metallic foil, particularly aluminium or aluminium alloy foil (e.g. having a thickness of 20µm to 45µm, preferably 20µm to 25µm) that is coated with a heat-sealing polymer layer on its inner side (sealing side). The bottom part may contain a multi-layer polymer foil (such as e.g. poly(vinyl chloride) (PVC) coated with poly(vinylidene choride) (PVDC); or a PVC foil laminated with poly(chlorotriflouroethylene) (PCTFE)) or a multi-layer polymer-metal-polymer foil (such as e.g. a cold-formable laminated PVC/aluminium/polyamide composition). Examples of blister packs may include alu/alu, alu/PVC/polyvinylacetate copolymer-acrylate or alu/PVC/PCTFE/PVC blisters.

To ensure a long storage period especially under hot and wet climate conditions an additional overwrap or pouch made of a multi-layer polymer-metal-polymer foil (e.g. a laminated polyethylene/aluminium/polyester composition) may be used for the blister packs. Supplementary desiccant (such as e.g. bentonite clay, molecular sieves, or, preferably, silica gel) in this pouch package may prolong the shelf life even more under such harsh conditions.

The article may further comprise a label or package insert, which refer to instructions customarily included in commercial packages of therapeutic products, that may contain information about the indications, usage, dosage, administration, contraindications and/or warnings concerning the use of such therapeutic products. In one embodiment, the label or package inserts indicates that the composition can be used for any of the purposes described herein.

With respect to the first embodiment (embodiment **A),** the dosage typically required of the DPP-4 inhibitors mentioned herein in embodiment **A** when administered intravenously is 0.1 mg to 10 mg, preferably 0.25 mg to 5 mg, and when administered orally is 0.5 mg to 100 mg, preferably 2.5 mg to 50 mg or 0.5 mg to 10 mg, more preferably 2.5 mg to 10 mg or 1 mg to 5 mg, in each case 1 to 4 times a day. Thus, e.g. the amount of 1-[(4-methyl-quinazolin-2-yl)methyl]-3-methyl-7-(2-butyn-1-yl)-8-(3-(R)-amino-piperidin-1-yl)-xanthine when administered orally is 0.5 mg to 10 mg per patient per day, preferably 2.5 mg to 10 mg or 1 mg to 5 mg per patient per day.

A dosage form prepared with a pharmaceutical composition comprising a DPP-4 inhibitor mentioned herein in embodiment **A** contain the active ingredient in a dosage range of 0.1-100 mg. Thus, e.g. particular oral dosage strengths of 1-[(4-methyl-quinazolin-2-yl)methyl]-3-methyl-7-(2-butyn-1-yl)-8-(3-(R)-amino-piperidin-1-yl)-xanthine are 0.5 mg, 1 mg, 2.5 mg, 5 mg and 10 mg.

A special embodiment of the DPP-4 inhibitors of this invention refers to those orally administered DPP-4 inhibitors which are therapeutically efficacious at low dose levels, e.g. at oral dose levels < 100 mg or < 70 mg per patient per day, preferably < 50 mg, more preferably < 30 mg or < 20 mg, even more preferably from 1 mg to 10 mg, particularly from 1 mg to 5 mg (more particularly 5 mg), per patient per day (if required, divided into 1 to 4 single doses, particularly 1 or 2 single doses, which may be of the same size, preferentially, administered orally once- or twice daily (more preferentially once-daily), advantageously, administered at any time of day, with or without food. Thus, for example, the daily oral amount 5 mg BI 1356 can be given in an once daily dosing regimen (i.e. 5 mg BI 1356 once daily) or in a twice daily dosing regimen (i.e. 2.5 mg BI 1356 twice daily), at any time of day, with or without food.

The dosage of the active ingredients in the combinations and compositions in accordance with the present invention may be varied, although the amount of the active ingredients shall be such that a suitable dosage form is obtained. Hence, the selected dosage and the selected dosage form shall depend on the desired therapeutic effect, the route of administration and the duration of the treatment. Dosage ranges for the combination may be from the maximal tolerated dose for the single agent to lower doses.

A particularly preferred DPP-4 inhibitor to be emphasized within the meaning of this invention is 1-[(4-methyl-quinazolin-2-yl)methyl]-3-methyl-7-(2-butyn-1-yl)-8-(3-(R)-amino-piperidin-1-yl)-xanthine (also known as BI 1356 or linagliptin). BI 1356 exhibits high potency, 24h duration of action, and a wide therapeutic window. In patients with type 2 diabetes receiving multiple oral doses of 1, 2.5, 5 or 10 mg of BI 1356 once daily for 12 days, BI 1356 shows favourable pharmacodynamic and pharmacokinetic profile (see e.g. Table 3 below) with rapid attainment of steady state (e.g. reaching steady state plasma levels (> 90% of the pre-dose plasma concentration on Day 13) between second and fifth day of treatment in all dose groups), little accumulation (e.g. with a mean accumulation ratio R_{A,AUC} ≤ 1.4 with doses above 1 mg) and preserving a long-lasting effect on DPP-4 inhibition (e.g. with almost complete (> 90%) DPP-4 inhibition at the 5 mg and 10 mg dose levels, i.e. 92.3 and 97.3% inhibition at steady state, respectively, and > 80% inhibition over a 24h interval after drug intake), as well as significant decrease in 2h postprandial blood glucose excursions by ≥ 80 % (already on Day 1) in doses ≥ 2.5 mg, and with the cumulative amount of unchanged parent compound excreted in urine on Day 1 being below 1% of the administered dose and increasing to not more than about 3-6% on Day 12 (renal clearance CL_{R,ss} is from about 14 to about 70 mL/min for the administered oral doses, e.g. for the 5 mg dose renal clearance is about 70 ml/min). In people with type 2 diabetes BI 1356 shows a placebo-like safety and tolerability. With low doses of about ≥ 5 mg, BI 1356 acts as a true once-daily oral drug with a full 24 h duration of DPP-4 inhibition. At therapeutic oral dose levels, BI 1356 is mainly excreted via the liver and only to a minor extent (about < 7% of the administered oral dose) via the kidney. BI 1356 is primarily excreted unchanged via the bile. The fraction of BI 1356 eliminated via the kidneys increases only very slightly over time and with increasing dose, so that there will likely be no need to modify the dose of BI 1356 based on the patients' renal function. The non-renal elimination of BI 1356 in combination with its low accumulation potential and broad safety margin may be of significant benefit in a patient population that has a high prevalence of renal insufficiency and diabetic nephropathy.

**Table 3: Geometric mean (gMean) and geometric coefficient of variation (gCV) of pharmacokinetic parameters of BI 1356 at steady state (Day 12)**

| **Parameter** | **1 mg** | **2.5 mg** | **5 mg** | **10 mg** |
|---|---|---|---|---|
| | **gMean (gCV)** | **gMean (gCV)** | **gMean (gCV)** | **gMean (gCV)** |
| AUC₀₋₂₄ [nmol·h/L] | 40.2 (39.7) | 85.3 (22.7) | 118 (16.0) | 161 (15.7) |
| AUC_{T,ss} [nmol·h/L] | 81.7 (28.3) | 117 (16.3) | 158 (10.1) | 190 (17.4) |
| Cₘₐₓ [nmol/L] | 3.13 (43.2) | 5.25 (24.5) | 8.32 (42.4) | 9.69 (29.8) |
| C_{max,ss} | 4.53 (29.0) | 6.58 (23.0) | 11.1 (21.7) | 13.6 (29.6) |
| [nmol/L] | | | | |
| tₘₐₓ* [h] | 1.50 [1.00 - 3.00] | 2.00 [1.00 - 3.00] | 1.75 [0.92 - 6.02] | 2.00 [1.50 - 6.00] |
| t_{max,ss}* [h] | 1.48 [1.00 - 3.00] | 1.42 [1.00 - 3.00] | 1.53 [1.00 - 3.00] | 1.34 [0.50 - 3.00] |
| T_{½,ss} [h] | 121 (21.3) | 113 (10.2) | 131 (17.4) | 130 (11.7) |
| Accumulation t_{½,} [h] | 23.9 (44.0) | 12.5 (18.2) | 11.4 (37.4) | 8.59 (81.2) |
| R_{A,Cmax} | 1.44 (25.6) | 1.25 (10.6) | 1.33 (30.0) | 1.40 (47.7) |
| R_{A,AUC} | 2.03 (30.7) | 1.37 (8.2) | 1.33 (15.0) | 1.18 (23.4) |
| fe₀₋₂₄ [%] | NC | 0.139 (51.2) | 0.453 (125) | 0.919 (115) |
| fe_{τ,ss} [%] | 3.34 (38.3) | 3.06 (45.1) | 6.27 (42.2) | 3.22 (34.2) |
| CL_{R,ss} [mL/min] | 14.0 (24.2) | 23.1 (39.3) | 70 (35.0) | 59.5 (22.5) |

| | | | | |
|---|---|---|---|---|
| * median and range [min-max] NC not calculated as most values below lower limit of quantification | | | | |

As different metabolic functional disorders often occur simultaneously, it is quite often indicated to combine a number of different active principles with one another. Thus, depending on the functional disorders diagnosed, improved treatment outcomes may be obtained if a DPP-4 inhibitor is combined with one or more active substances customary for the respective disorders, such as e.g. one or more active substances selected from among the other antidiabetic substances, especially active substances that lower the blood sugar level or the lipid level in the blood, raise the HDL level in the blood, lower blood pressure or are indicated in the treatment of atherosclerosis or obesity.

The DPP-4 inhibitors mentioned above - besides their use in mono-therapy - may also be used in conjunction with one or more other active substances, by means of which improved treatment results can be obtained. Such a combined treatment may be given as a free combination of the substances or in the form of a fixed combination, for example in a tablet or capsule. Pharmaceutical formulations of the combination partner needed for this may either be obtained commercially as pharmaceutical compositions or may be formulated by the skilled man using conventional methods. The active substances which may be obtained commercially as pharmaceutical compositions are described in numerous places in the prior art, for example in the list of drugs that appears annually, the "Rote Liste ^{®}" of the federal association of the pharmaceutical industry, or in the annually updated compilation of manufacturers' information on prescription drugs known as the "Physicians' Desk Reference".

Examples of antidiabetic combination partners are metformin; sulphonylureas such as glibenclamide, tolbutamide, glimepiride, glipizide, gliquidon, glibornuride and gliclazide; nateglinide; repaglinide; mitiglinide; thiazolidinediones such as rosiglitazone and pioglitazone; PPAR gamma modulators such as metaglidases; PPAR-gamma agonists such as e.g. rivoglitazone, mitoglitazone, INT-131 and balaglitazone; PPAR-gamma antagonists; PPAR-gamma/alpha modulators such as tesaglitazar, muraglitazar, aleglitazar, indeglitazar and KRP297; PPAR-gamma/alpha/delta modulators such as e.g. lobeglitazone; AMPK-activators such as AICAR; acetyl-CoA carboxylase (ACC1 and ACC2) inhibitors; diacylglycerol-acetyltransferase (DGAT) inhibitors; pancreatic beta cell GCRP agonists such as GPR119 agonists (SMT3-receptor-agonists), such as the GPR119 agonists 5-ethyl-2-{4-[4-(4-tetrazol-1-yl-phenoxymethyl)-thiazol-2-yl]-piperidin-1-yl}-pyrimidine or 5-[1-(3-isopropyl-[1,2,4]oxadiazol-5-yl)-piperidin-4-ylmethoxy]-2-(4-methanesulfonyl-phenyl)-pyridine; 11β-HSD-inhibitors; FGF19 agonists or analogues; alpha-glucosidase blockers such as acarbose, voglibose and miglitol; alpha2-antagonists; insulin and insulin analogues such as human insulin, insulin lispro, insulin glusilin, r-DNA-insulinaspart, NPH insulin, insulin detemir, insulin degludec, insulin tregopil, insulin zinc suspension and insulin glargin; Gastric inhibitory Peptide (GIP); amylin and amylin analogues (e.g. pramlintide or davalintide); GLP-1 and GLP-1 analogues such as Exendin-4, e.g. exenatide, exenatide LAR, liraglutide, taspoglutide, lixisenatide (AVE-0010), LY-2428757 (a PEGylated version of GLP-1), dulaglutide (LY-2189265), semaglutide or albiglutide; SGLT2-inhibitors such as e.g. dapagliflozin, sergliflozin (KGT-1251), atigliflozin, canagliflozin, ipragliflozin, luseogliflozin or tofogliflozin; inhibitors of protein tyrosine-phosphatase (e.g. trodusquemine); inhibitors of glucose-6-phosphatase; fructose-1,6-bisphosphatase modulators; glycogen phosphorylase modulators; glucagon receptor antagonists; phosphoenolpyruvatecarboxykinase (PEPCK) inhibitors; pyruvate dehydrogenasekinase (PDK) inhibitors; inhibitors of tyrosine-kinases (50 mg to 600 mg) such as PDGF-receptor-kinase (cf. EP-A-564409, WO 98/35958, US 5093330, WO 2004/005281, and WO 2006/041976) or of serine/threonine kinases; glucokinase/regulatory protein modulators incl. glucokinase activators; glycogen synthase kinase inhibitors; inhibitors of the SH2-domain-containing inositol 5-phosphatase type 2 (SHIP2) ; IKK inhibitors such as high-dose salicylate; JNK1 inhibitors; protein kinase C-theta inhibitors; beta 3 agonists such as ritobegron, YM 178, solabegron, talibegron, N-5984, GRC-1087, rafabegron, FMP825; aldosereductase inhibitors such as AS 3201, zenarestat, fidarestat, epalrestat, ranirestat, NZ-314, CP-744809, and CT-112; SGLT-1 or SGLT-2 inhibitors; KV 1.3 channel inhibitors; GPR40 modulators such as e.g. [(3S)-6-({2',6'-dimethyl-4'-[3-(methylsulfonyl)propoxy]biphenyl-3-yl}methoxy)-2,3-dihydro-1-benzofuran-3-yl]acetic acid; SCD-1 inhibitors; CCR-2 antagonists; dopamine receptor agonists (bromocriptine mesylate [Cycloset]); 4-(3-(2,6-dimethylbenzyloxy)phenyl)-4-oxobutanoic acid; sirtuin stimulants; and other DPP IV inhibitors.

Metformin is usually given in doses varying from about 500 mg to 2000 mg up to 2500 mg per day using various dosing regimens from about 100 mg to 500 mg or 200 mg to 850 mg (1-3 times a day), or about 300 mg to 1000 mg once or twice a day, or delayed-release metformin in doses of about 100 mg to 1000 mg or preferably 500 mg to 1000 mg once or twice a day or about 500 mg to 2000 mg once a day. Particular dosage strengths may be 250, 500, 625, 750, 850 and 1000 mg of metformin hydrochloride.

A dosage of pioglitazone is usually of about 1-10 mg, 15 mg, 30 mg, or 45 mg once a day.

Rosiglitazone is usually given in doses from 4 to 8 mg once (or divided twice) a day (typical dosage strengths are 2, 4 and 8 mg).

Glibenclamide (glyburide) is usually given in doses from 2.5-5 to 20 mg once (or divided twice) a day (typical dosage strengths are 1.25, 2.5 and 5 mg), or micronized glibenclamide in doses from 0.75-3 to 12 mg once (or divided twice) a day (typical dosage strengths are 1.5, 3, 4.5 and 6 mg).

Glipizide is usually given in doses from 2.5 to 10-20 mg once (or up to 40 mg divided twice) a day (typical dosage strengths are 5 and 10 mg), or extended-release glibenclamide in doses from 5 to 10 mg (up to 20 mg) once a day (typical dosage strengths are 2.5, 5 and 10 mg).

Glimepiride is usually given in doses from 1-2 to 4 mg (up to 8 mg) once a day (typical dosage strengths are 1, 2 and 4 mg).

A dual combination of glibenclamide/metformin is usually given in doses from 1.25/250 once daily to 10/1000 mg twice daily. (typical dosage strengths are 1.25/250, 2.5/500 and 5/500 mg).

A dual combination of glipizide/metformin is usually given in doses from 2.5/250 to 10/1000 mg twice daily (typical dosage strengths are 2.5/250, 2.5/500 and 5/500 mg).

A dual combination of glimepiride/metformin is usually given in doses from 1/250 to 4/1000 mg twice daily.

A dual combination of rosiglitazone/glimepiride is usually given in doses from 4/1 once or twice daily to 4/2 mg twice daily (typical dosage strengths are 4/1, 4/2, 4/4, 8/2 and 8/4 mg). A dual combination of pioglitazone/glimepiride is usually given in doses from 30/2 to 30/4 mg once daily (typical dosage strengths are 30/4 and 45/4 mg).

A dual combination of rosiglitazone/metformin is usually given in doses from 1/500 to 4/1000 mg twice daily (typical dosage strengths are 1/500, 2/500, 4/500, 2/1000 and 4/1000 mg).

A dual combination of pioglitazone/metformin is usually given in doses from 15/500 once or twice daily to 15/850 mg thrice daily (typical dosage strengths are 15/500 and 15/850 mg).

The non-sulphonylurea insulin secretagogue nateglinide is usually given in doses from 60 to 120 mg with meals (up to 360 mg/day, typical dosage strengths are 60 and 120 mg); repaglinide is usually given in doses from 0.5 to 4 mg with meals (up to 16 mg/day, typical dosage strengths are 0.5, 1 and 2 mg). A dual combination of repaglinide/metformin is available in dosage strengths of 1/500 and 2/850 mg.

Acarbose is usually given in doses from 25 to 100 mg with meals. Miglitol is usually given in doses from 25 to 100 mg with meals.

Examples of combination partners that lower the lipid level in the blood are HMG-CoA-reductase inhibitors such as simvastatin, atorvastatin, lovastatin, fluvastatin, pravastatin, pitavastatin and rosuvastatin; fibrates such as bezafibrate, fenofibrate, clofibrate, gemfibrozil, etofibrate and etofyllinclofibrate; nicotinic acid and the derivatives thereof such as acipimox; PPAR-alpha agonists; PPAR-delta agonists such as e.g. {4-[(R)-2-ethoxy-3-(4-trifluoromethyl-phenoxy)-propylsulfanyl]-2-methyl-phenoxy}-acetic acid; PPAR- alpha/delta agonists; inhibitors of acyl-coenzyme A:cholesterolacyltransferase (ACAT; EC 2.3.1.26) such as avasimibe; cholesterol resorption inhibitors such as ezetimib; substances that bind to bile acid, such as cholestyramine, colestipol and colesevelam; inhibitors of bile acid transport; HDL modulating active substances such as D4F, reverse D4F, LXR modulating active substances and FXR modulating active substances; CETP inhibitors such as torcetrapib, JTT-705 (dalcetrapib) or compound 12 from WO 2007/005572 (anacetrapib); LDL receptor modulators; MTP inhibitors (e.g. lomitapide); and ApoB100 antisense RNA.

A dosage of atorvastatin is usually from 1 mg to 40 mg or 10 mg to 80 mg once a day.

Examples of combination partners that lower blood pressure are beta-blockers such as atenolol, bisoprolol, celiprolol, metoprolol and carvedilol; diuretics such as hydrochlorothiazide, chlortalidon, xipamide, furosemide, piretanide, torasemide, spironolactone, eplerenone, amiloride and triamterene; calcium channel blockers such as amlodipine, nifedipine, nitrendipine, nisoldipine, nicardipine, felodipine, lacidipine, lercanipidine, manidipine, isradipine, nilvadipine, verapamil, gallopamil and diltiazem; ACE inhibitors such as ramipril, lisinopril, cilazapril, quinapril, captopril, enalapril, benazepril, perindopril, fosinopril and trandolapril; as well as angiotensin II receptor blockers (ARBs) such as telmisartan, candesartan, valsartan, losartan, irbesartan, olmesartan, azilsartan and eprosartan.

A dosage of telmisartan is usually from 20 mg to 320 mg or 40 mg to 160 mg per day.

Examples of combination partners which increase the HDL level in the blood are Cholesteryl Ester Transfer Protein (CETP) inhibitors; inhibitors of endothelial lipase; regulators of ABC1; LXRalpha antagonists; LXRbeta agonists; PPAR-delta agonists; LXRalpha/beta regulators, and substances that increase the expression and/or plasma concentration of apolipoprotein A-I.

Examples of combination partners for the treatment of obesity are sibutramine; tetrahydrolipstatin (orlistat); alizyme (cetilistat); dexfenfluramine; axokine; cannabinoid receptor 1 antagonists such as the CB1 antagonist rimonobant; MCH-1 receptor antagonists; MC4 receptor agonists; NPY5 as well as NPY2 antagonists (e.g. velneperit); beta3-AR agonists such as SB-418790 and AD-9677; 5HT2c receptor agonists such as APD 356 (lorcaserin); myostatin inhibitors; Acrp30 and adiponectin; steroyl CoA desaturase (SCD1) inhibitors; fatty acid synthase (FAS) inhibitors; CCK receptor agonists; Ghrelin receptor modulators; Pyy 3-36; orexin receptor antagonists; and tesofensine; as well as the dual combinations bupropion/naltrexone, bupropion/zonisamide, topiramate/phentermine and pramlintide/metreleptin.

Examples of combination partners for the treatment of atherosclerosis are phospholipase A2 inhibitors; inhibitors of tyrosine-kinases (50 mg to 600 mg) such as PDGF-receptor-kinase (cf. EP-A-564409, WO 98/35958, US 5093330, WO 2004/005281, and WO 2006/041976); oxLDL antibodies and oxLDL vaccines; apoA-1 Milano; ASA; and VCAM-1 inhibitors.

Further, the certain DPP-4 inhibitor of this invention may be used in combination with a substrate of DPP-4 (particularly with an anti-inflammatory substrate of DPP-4), which may be other than GLP-1, for the purposes according to the present invention, such substrates of DPP-4 include, for example - without being limited to, one or more of the following:
Incretins:
   Glucagon-like peptide (GLP)-1
   Glucose-dependent insulinotropic peptide (GIP)
Neuroactive:
   Substance P
   Neuropeptide Y (NPY)
   Peptide YY
Energy homeostasis:
   GLP-2
   Prolactin
   Pituitary adenylate cyclase activating peptide (PACAP)
Other hormones:
   PACAP 27
   Human chorionic gonadotrophin alpha chain
   Growth hormone releasing factor (GHRF)
   Luteinizing hormone alpha chain
   Insulin-like growth factor (IGF-1)
   CCL8/eotaxin
   CCL22/macrophage-derived chemokine
   CXCL9/interferon-gamma-induced monokine
Chemokines:
   CXCL10/interferon-gamma-induced protein-10
   CXCL11/interferon-inducible T cell a chemoattractant
   CCL3L1/macrophage inflammatory protein 1alpha isoform
   LD78beta
   CXCL12/stromal-derived factor 1 alpha and beta
Other:
   Enkephalins, gastrin-releasing peptide, vasostatin-1,
   peptide histidine methionine, thyrotropin alpha

Further or in addition, the certain DPP-4 inhibitor of this invention may be used in combination with one or more active substances which are indicated in the treatment of nephropathy, such as selected from diuretics, ACE inhibitors and/or ARBs.

Further or in addition, the certain DPP-4 inhibitor of this invention may be used in combination with one or more active substances which are indicated in the treatment or prevention of cardiovascular diseases or events (e.g. major cardiovascular events).

Moreover, optionally in addition, the certain DPP-4 inhibitor of this invention may be used in combination with one or more antiplatelet agents, such as e.g. (low-dose) aspirin (acetylsalicylic acid), a selective COX-2 or nonselective COX-1/COX-2 inhibitor, or a ADP receptor inhibitor, such as a thienopyridine (e.g. clopidogrel or prasugrel), elinogrel or ticagrelor, or a thrombin receptor antagonist such as vorapaxar.

Yet moreover, optionally in addition, the certain DPP-4 inhibitor of this invention may be used in combination with one or more anticoagulant agents, such as e.g. heparin, a coumarin (such as warfarin or phenprocoumon), a pentasaccharide inhibitor of Factor Xa (e.g. fondaparinux), or a direct thrombin inhibitor (such as e.g. dabigatran), or a Faktor Xa inhibitor (such as e.g. rivaroxaban or apixaban or edoxaban or otamixaban).

Still yet moreover, optionally in addition, the certain DPP-4 inhibitor of this invention may be used in combination with one or more agents for the treatment of heart failure (such as e.g. those mentioned in WO 2007/128761).

The present invention is not to be limited in scope by the specific embodiments described herein. Various modifications of the invention in addition to those described herein may become apparent to those skilled in the art from the present disclosure. Such modifications are intended to fall within the scope of the appended claims.

All patent applications cited herein are hereby incorporated by reference in their entireties.

### Reduction in loss of podocytes; Expression of podocalyxin as a marker of podocyte integrity:

The expression of podocalyxin is analysed by immunohistochemistry using a podocalyxin specific antibody. Kidney sections from male diabetic db/db mice (10 weeks old at start and treated for 3 months) in the following groups are analysed:
Diabetic control (n=10), linagliptin 3 mg/kg (n=8), enalapril 20 mg/kg (n=10) and
heterozygous control mice (n=8).
All of the evaluations of the glomerular staining intensities are done semiquantitatively by two different experts of kidney pathology blinded for the slides.

The predefined scoring gradient is 0, 1, 2, and 3. 0 means no expression, whereas 3 is given when expression is highest. Group means are compared with a non-parametric test. P values less than 0.05 are considered significant.

Figure 1 shows the expression of podocalyxin as a marker for podocyte integrity in linagliptin-, enalapril- or vehicle-treated diabetic db/db mice and in healthy control mice.

This prove of concept study in db/db mice (abstract see below) indicates that DPP-4 inhibition might offer an new therapeutic approach for the treatment of proteinuric diseases associated with podocyte loss. This study clearly demonstrates that the DPP-4 inhibitor linagliptin significantly reduces the loss of podocytes in a blood glucose independent manner in db/db mice (Fig. 1). Podocyte loss is determined by podocalyxin staining. Podocalyxin, a sialoglycoprotein, is thought to be the major constituent of the glycocalyx of podocytes. It is a member of the CD34 family of transmembrane sialomucin. It coats the secondary foot processes of the podocytes. It is negatively charged and thus functions to keep adjacent foot processes separated, thereby keeping the urinary filtration barrier open. This function is further supported by knockout studies in mice which reveal an essential role in podocyte morphogenesis.

### Renoprotective effects of Linagliptin; Protection of podocytes:

Diabetic nephropathy is the main cause of end-stage renal disease. This study investigated the effects of linagliptin on diabetic nephropathy in severe insulin-resistant and old db/db mice as a model for diabetic nephropathy. Male diabetic db/db mice (10 weeks) were divided into 3 groups and treated for 12 weeks with vehicle (n=10), linagliptin 3 mg/kg/day (n=8), or the angiotensin converting enzyme (ACE) inhibitor enalapril 20 mg/kg/day (n=10). Heterozygous db/+ mice treated with vehicle were used as controls (n=8). Levels of glucose, triglycerides, insulin, cystatin C and creatinine were analysed in serum and urine samples at baseline and monthly thereafter. Body weight, urinary albumin excretion and OGTT were monitored periodically.

Renal histology (glomerulosclerosis, tubulointerstitial fibrosis) and expression of the sialoglycoprotein podocalyxin (a marker of podocyte integrity in the glomeruli, marker for glomerular damage), glucagon-like peptide 1 receptor (GLP-1R), alpha-smooth muscle actin and type I collagen were evaluated at the end of the study.

### Results:

At 22 weeks, db/db mice showed significantly (p<0.01) higher levels of fasting plasma glucose, insulin, and triglycerides, and increased body weight compared with healthy db/+ mice. Linagliptin and enalapril had limited effects on fasted or post-prandial glucose levels. However, histology analysis showed that tubulointerstitial fibrosis and glomerular mesangial matrix expansion were reduced almost to control levels in both treatment groups compared with diabetic vehicle (p<0.05 for both). Urinary albumin excretion rates and tubulointerstitial fibrosis were significantly decreased in db/db mice treated with linagliptin compared with those treated with enalapril (both p<0.05).

Podocalyxin expression in db/db vehicle treated mice was significantly reduced compared with db/+ controls (1.59±0.2 vs 2.65±0.1; p<0.001). Mice treated with linagliptin and enalapril had significantly higher podocalyxin expression compared with diabetic mice (2.3±0.2 and 2.4±0.2, respectively; p<0.05 for both).

The expression pattern of α-smooth muscle actin was also determined in kidneys as a marker of mesangial cell damage. Linagliptin treatment normalized the expression of α-smooth muscle actin-positive myofibroblasts in the interstitium and glomeruli of diabetic db/db mice. Similar results were obtained for type I collagen deposition.

Immunohistochemical staining of kidney sections revealed a decrease in GLP-1R expression in the cortical glomeruli of db/db mice (1.67 ± 0.07) compared with healthy control mice (2.15 ± 0.1; P < 0.01). Linagliptin treatment significantly increased the expression of GLP-1R in the glomeruli of db/db mice (1.90 ± 0.04; P < 0.05) compared to vehicle-treated diabetic db/db mice.

In conclusion, this study suggests that linagliptin protects podocytes from injury and may therefore be efficacious in the treatment, prevention or delay in progression of diabetic nephropathy independent of its effect on glucose homeostasis. Further, this study suggests that linagliptin is useful for treating, preventing or delaying progression of glomerulosclerosis and/or tubulointerstitial fibrosis, or glomerular and/or tubulointerstitial injury. Further, this study suggests that linagliptin is useful for renoprotection through inhibition of podocyte damage and myofibroblast transformation (reduction of α-SMA expression).

The renoprotective effect of linagliptin in this model seems to be as effective as treatment with an ACE inhibitor, the current gold standard for treatment of diabetic nephropathy.

### Linagliptin for use in lowering albuminuria on top of recommended standard treatment for diabetic nephropathy:

Despite optimal therapy, people with type 2 diabetes (T2D) remain at high risk for kidney damage, manifest as albuminuria, and many develop progressive renal failure. Further, despite optimal therapy with inhibitors of the renin-angiotensin-aldosterone system (RAAS), patients with type 2 diabetes mellitus (T2DM) remain at an increased risk of progressive renal failure and cardiovascular disease, for which albuminuria has emerged as a predictive biomarker. Linagliptin, a DPP-4 inhibitor, has previously shown evidence of albumin lowering on top of telmisartan in mice. We explored the clinical effect of linagliptin on albuminuria in T2D patients with early diabetic nephropathy. Four randomized, double-blind, 24-week, placebo-controlled trials of linagliptin (5 mg qd) on no, mono, or dual oral glucose-lowering background therapy (such as e.g. linagliptin monotherapy, linagliptin add-on to metformin or linagliptin add-on to metformin plus a sulphonylurea, or linagliptin plus metformin initial combination) had data available for urinary albumin-to-creatinine ratio (UACR) and were pooled for analysis (n=2472). Participants were included in this analysis if they had: i) 30≤UACR≤3000 mg/g creatinine; ii) stable treatment with ACE/ARBs ≥4 weeks prior and during the trial; and iii) eGFR >30 ml/min/1.73 m². The endpoint was the percentage change in geometric mean UACR. In this analysis, 492 (19.9%) patients met UACR and eGFR thresholds of whom 46% received stable ACE/ARB therapy (linagliptin n=168; placebo n=59). Mean baseline A1C and median UACR were 8.2% vs 8.5% and 76 vs 78 mg/g creatinine for the linagliptin and placebo groups, respectively. After 24 weeks, placebo-corrected changes in A1C and FPG were -0.71% and -26 mg/dl, respectively (both p<.0001). Linagliptin significantly lowered adjusted UACR by 33% (95%CI 22 to 42%; p<.05) with a between group difference vs placebo of -29% (-3 to -48%; p<.05). Overall, kidney function and blood pressure were unchanged although more patients on placebo received new anti-hypertensive drugs (17% vs 11% with linagliptin). Sensitivity analyses in patients not previously treated with RAS blockade (n=265) found similar results. Linagliptin may have kidney-protective properties beyond glucose-lowering effects. This protective effect may be independent of race. Linagliptin may be useful for treating or lowering albuminuria on top of standard of care of angiotensin-converting enzyme (ACE) inhibition or angiotensin II receptor blockade (ARB) in T2DM patients with early diabetic nephropathy.

### Linagliptin for use in treatment of albuminuria in patients with type 2 diabetes and diabetic nephropathy:

Background and aims: Diabetes mellitus has become the most common single cause of end-stage renal disease and a high proportion of individuals with type 2 diabetes (T2D) are found to have microalbuminuria and overt nephropathy shortly after the diagnosis of their diabetes. Linagliptin, a DPP-4 inhibitor, has recently demonstrated glycaemic efficacy and safety in T2D patients at advanced stages of kidney disease. Here the clinical effect of linagliptin on albuminuria in T2D patients with early diabetic nephropathy is reported.

Materials and methods: Seven randomised, double-blind, placebo-controlled trials (duration 24-52 weeks) of linagliptin (5 mg q.d.) as monotherapy or add-on to various glucose-lowering background therapies had data available for urinary albumin-to-creatinine ratio (UACR) and were eligible for this analysis (n=4113). Data after 24 weeks of treatment were generated to allow pooling and two sets were defined: 1) Diabetic nephropathy in earlier stages of T2D (with and without oral glucose-lowering background therapies, such as e.g. linagliptin monotherapy, linagliptin add-on to metformin or linagliptin add-on to metformin plus a sulphonylurea, or linagliptin plus metformin initial combination): participants from four 24-week pivotal phase III trials if they had persistent albuminuria, defined as 30≤UACR≤3000 mg/g (eGFR >30 ml/min/1.73 m²) and stable treatment with an angiotensin-converting enzyme inhibitor (ACEi) or angiotensin II receptor blocker (ARB) at baseline (ongoing treatment with ACEi or ARB); 2) Diabetic nephropathy in elderly patients (various glucose-lowering background therapies including insulin, such as e.g. linagliptin monotherapy, linagliptin add-on to metformin or linagliptin add-on to metformin plus a sulphonylurea, or linagliptin plus metformin initial combination, or linagliptin in combination with basal insulin): patients from all seven trials, fulfilling UACR criteria 30≤UACR≤3000 mg/g (eGFR >30 ml/min/1.73 m²) and aged ≥65 years (with or without ongoing treatment with ACEi or ARB). The endpoint in both sets was the percentage change in geometric mean UACR after 24 weeks.

Results: For set #1, 492 out of 2472 patients met UACR criteria of whom 46% received stable ACEi/ARB therapy (linagliptin, n=168; placebo, n=59). For set #2, 1331 patients were aged ≥65 years of whom 377 (28%) met UACR criteria (linagliptin, n=232; placebo, n=145). Mean baseline HbA1c and median UACR were 8.3% and 76 mg/g overall for set #1, and 8.1% (overall), 77 mg/g (linagliptin) and 86 mg/g (placebo) for set #2. In set 1, placebo-corrected changes in HbA1c and fasting plasma glucose were -0.71% and -1.4 mmol/L (-26 mg/dL), respectively (both P<0.0001). Linagliptin significantly lowered adjusted UACR by 33% (95% Cl: 22%, 42%; P<0.05) with a between-group difference versus placebo of -29% (95% Cl: -3%, -48%; P<0.05). In set 2, linagliptin also significantly lowered adjusted UACR by 30% (95% Cl: 13%, 43%; P<0.05) with a trend towards a reduction versus placebo of - 25% (95% Cl: -47%, +6%). In all seven studies, blood pressure and renal function were not affected to a clinically meaningful extent by either treatment.

Conclusions: In studies up to 52 weeks, linagliptin lowered albuminuria beyond what may be expected by its glucose-lowering effects. Changes in albuminuria were seen more rapidly (e.g. with the overall UACR effect occurring as early as 12 weeks treatment duration) than would be expected based on structural changes. A decrease in albuminuria suggests a long-term renal benefit.

Further, linagliptin (5 mg qd) lowers (micro)albuminuria in vulnerable diabetic nephropathy patients (with or without additional standard background therapy such as e.g. with an ACEi or ARB) such as who are aged ≥ 65 years typically having longer diabetes duration (> 5 years), renal impairment (such as mild (60 to <90 eGFR ml/min/1.73 m²) or moderate (30 to <60 eGFR ml/min/1.73 m²) renal impairment) and/or higher baseline UACR (such as advanced stages of micro- or macroalbuminuria).

In some instances, the diabetic nephropathy patients amenable to the therapy of this invention may be on hypertension and/or lipid lowering medication at baseline, such as e.g. on (ongoing) therapy with an ACE inhibitor, ARB, beta-blocker, Calcium-anatgonist or diuretic, or combination thereof, and/or on (ongoing) therapy with a fibrate, niacin or statin, or combination thereof.

### Renal Safety and Outcomes with Linagliptin: Meta-Analysis in 5466 Patients with Type 2 Diabetes:

Long-term glycemic control in diabetes is associated with reduced risk of renal microvascular complications. Linagliptin has shown nephroprotective effects in animal models and significantly reduced albuminuria in type 2 diabetes (T2D) associated nephropathy. As these effects were independent of short-term glycemic improvements, it was speculated that linagliptin may have nephroprotective effects. The aim of this study was to evaluate renal safety/outcomes with linagliptin in phase 3, randomized, double-blind, placebo-controlled trials (≥ 12 wks). Predefined events from 13 trials were analysed using a composite primary endpoint: new onset/occurrence of a) micro- (first documented UACR ≥30 mg/g) or b) macro-(first documented UACR ≥300 mg/g) albuminuria, c) CKD (serum creatinine increase ≥250 µmol/L), d) worsening of CKD (loss in eGFR >50% vs baseline), e) acute renal failure (ARF, standardized MedDRA query) and f) death (any cause). Of 5466 patients included (mean baseline HbA1c: 8.2% and eGFR: 91.4 ml/min/1.73m2), 3505 received linagliptin 5mg qd and 1961 placebo; cumulative exposure (person yrs) was 1756 and 1057, respectively. Events occurred in 448 (12.8%) patients receiving linagliptin vs 306 (15.6%) for placebo. The hazard ratio (HR) for the composite endpoint for linagliptin vs. placebo was 0.84 (95% Cl 0.72-0.97, p<.05) and was not significantly altered by race, but was lower in patients <65 vs >65 yrs (HR: 0.77 vs 1.04). RRs were consistently reduced for individual renal endpoints: micro- (-15%) and macroalbuminuria (-12%), new onset (-56%) or worsening of CKD (-24%), ARF (-7%), and death (-23%). In this large meta-analysis, renal safety and outcomes were significantly improved in patients with T2D treated with linagliptin (5 mg/day, e.g. once daily; e.g. as monotherapy or in combination with other antidiabetic agents (e.g. metformin, sulphonylurea, insulin, metformin+ sulphonylurea, metformin+insulin) in patients with inadequately controlled type 2 diabetes). These data support a direct nephroprotective effect of linagliptin. Linagliptin may be useful for preventing, reducing or delaying the onset or slowing the progression of micro- or macro-albuminuria, the onset of chronic kidney disease (CKD), the worsening of CKD, the onset of acute renal failure and/or of death. Thus, linagliptin may be useful for preventing, reducing the risk of or delaying the onset or slowing the progression of renal morbidity and/or mortality, preferably in T2DM patients. Particularly, linagliptin may be useful for preventing, reducing the risk of or delaying the onset/occurrence or slowing the progression of CKD, preferably in T2DM patients.

In some instances, the (at-risk) patients (particularly type 2 diabetes patients) amenable to the renoprotection or risk-reduction of this invention (e.g. prevention, reduction or delay of the onset or progression of micro- or macro-albuminuria, the onset of chronic kidney disease (CKD), the worsening of CKD, the onset of acute renal failure and/or of death) may have renal/cardiovascular history and/or medications (optionally in addition to antidiabetic medications), such as diabetic nephropathy, macrovascular disease (e.g. coronary artery disease, peripheral artery disease, cerebrovascular disease, hypertension), microvascular disease (e.g. diabetic nephropathy, neuropathy, retinopathy), coronary artery disease, cerebrovascular disease, peripheral artery disease, hypertension, ex-smoker or current smoker, and/or on acetylsalicylic acid, antihypertensive and/or lipid lowering medication, such as e.g. on (ongoing) therapy with acetylsalicylic acid, an ACE inhibitor, ARB, beta-blocker, Calcium-anatgonist or diuretic, or combination thereof, and/or on (ongoing) therapy with a fibrate, niacin or statin, or combination thereof.

In some further instances, the (at-risk) patients (particularly type 2 diabetes patients) amenable to the renoprotection or risk-reduction of this invention (e.g. prevention, reduction or delay of the onset or progression of micro- or macro-albuminuria, the onset of chronic kidney disease (CKD), the worsening of CKD, the onset of acute renal failure and/or of death) may be patients with increased risk of renal events (such as e.g. patients with an eGFR < 60 mL/min or, particularly < 30 mL/min per 1.73 m²), micro- or macroalbuminuria, and/or elderly patients (such as e.g. > 70 years).

### Examples

In order that this invention be more fully understood, the herein-given examples are set forth. Further embodiments, features or aspects of the present invention may become apparent from the examples. The examples serve to illustrate, by way of example, the principles of the invention without restricting it.

### Treatment of patients with type 2 diabetes mellitus at high cardiovascular and renal microvascular risk:

The long term impact on cardiovascular and renal (microvascular) safety, morbidity and/or mortality and relevant efficacy parameters (e.g. HbA1c, fasting plasma glucose, treatment sustainability) of treatment with linagliptin in a relevant population of patients with type 2 diabetes mellitus (such as e.g. at high vascular risk) can be investigated as follows:
Type 2 diabetes patient with insufficient glycemic control (naive or pre-treated with any antidiabetic background medication, excluding treatment with GLP-1 receptor agonists, DPP-4 inhibitors or SGLT-2 inhibitors if ≥ consecutive 7 days, e.g. having HbA1c 6.5-10%), and high risk of cardiovascular events, e.g. defined by:
albuminuria (micro or macro) and previous macrovascular disease: e.g. defined according to Condition I as indicated below;
   and/or
impaired renal function: e.g. as defined according to Condition II as indicated below;

Condition I:
   albuminuria (such as e.g. urine albumin creatinine ratio (UACR) ≥ 30 mg/g creatinine or ≥ 30 mg/l (milligram albumin per liter of urine) or ≥ 30 µg/min (microgram albumin per minute) or ≥ 30 mg/24 h (milligram albumin per 24 hours)) and
   previous macrovascular disease, such as e.g. defined as one or more of a) to f):
      a) previous myocardial infarction (e.g. > 2 months),
      b) advanced coronary artery disease, such as e.g. defined by any one of the following:
         - ≥ 50% narrowing of the luminal diameter in 2 or more major coronary arteries (e.g. LAD (Left Anterior Descending), CX (Circumflex) or RCA (Right Coronary Artery)) by coronary angiography or CT angiography,
         - left main stem coronary artery with ≥ 50% narrowing of the luminal diameter,
         - prior percutaneous or surgical revascularization of ≥ 2 major coronary arteries (e.g. ≥ 2 months),
         - combination of prior percutaneous or surgical revascularization, such as e.g. of 1 major coronary artery (e.g. ≥ 2 months) and ≥ 50% narrowing of the luminal diameter by coronary angiography or CT angiography of at least 1 additional major coronary artery,
      c) high-risk single-vessel coronary artery disease, such as e.g. defined as the presence of ≥ 50% narrowing of the luminal diameter of one major coronary artery (e.g. by coronary angiography or CT angiography in patients not revascularised) and at least one of the following:
         - a positive non invasive stress test, such as e.g. confirmed by either:
            - a positive ECG exercise tolerance test in patients without left bundle branch block, Wolff-Parkinson-White syndrome, left ventricular hypertrophy with repolarization abnormality, or paced ventricular rhythm, atrial fibrillation in case of abnormal ST-T segments,
            - a positive stress echocardiogram showing induced regional systolic wall motion abnormalities,
            - a positive nuclear myocardial perfusion imaging stress test showing stress induced reversible perfusion abnormality,
            - patient discharged from hospital with a documented diagnosis of unstable angina pectoris (e.g. ≥ 2 - 12 months),
      d) previous ischemic or haemorrhagic stroke (e.g. > 3 months),
      e) presence of carotid artery disease (e.g. symptomatic or not), such as e.g. documented by either:
         - imaging techniques with at least one lesion estimated to be ≥50% narrowing of the luminal diameter,
         - prior percutaneous or surgical carotid revascularization,
      f) presence of peripheral artery disease, such as e.g. documented by either:
         - previous limb angioplasty, stenting or bypass surgery,
         - previous limb or foot amputation due to macrocirculatory insufficiency,
         - angiographic evidence of peripheral artery stenosis ≥ 50% narrowing of the luminal diameter in at least one limb (e.g. definition of peripheral artery: common iliac artery, internal iliac artery, external iliac artery, femoral artery, popliteal artery),
Condition II:
   impaired renal function (e.g. with or without CV co-morbidities), such as e.g. defined by:
   - impaired renal function (e.g. as defined by MDRD formula) with an estimated glomerular filtration rate (eGFR) 15-45 mL/min/1.73 m² with any urine albumin creatinine ratio (UACR), and/or
   - impaired renal function (e.g. as defined by MDRD formula) with an with an estimated glomerular filtration rate (eGFR) ≥ 45-75 mL/min/1.73 m² with an urine albumin creatinine ratio (UACR) > 200 mg/g creatinine or > 200 mg/l (milligram albumin per liter of urine) or > 200 µg/min (microgram albumin per minute) or > 200 mg/24 h (milligram albumin per 24 hours);
are treated over a lengthy period (e.g. for 4-5 years, or preferably at least 48 months) with linagliptin (preferably 5 mg per day, administered orally, preferably in form of a tablet, optionally in combination with one or more other active substances, e.g. such as those described herein) and compared with patients who have been treated with placebo (as add-on therapy on top of standard of care).

Evidence of the therapeutic success compared with patients who have been treated with placebo can be found in non-inferiority or superiority compared to placebo, e.g. in the (longer) time taken to first occurrence of cardio- or cerebrovascular events, e.g. time to first occurrence of any of the following components of the composite CV endpoint: cardiovascular death (including fatal stroke, fatal myocardial infarction and sudden death), non-fatal myocardial infarction (excluding silent myocardial infarction), non-fatal stroke, and (optional) hospitalisation e.g. for unstable angina pectoris; and/or
in the (longer) time taken to first occurrence of renal microvascular events, e.g. time to first occurrence of any of the following components of the composite renal endpoint: renal death, sustained end-stage renal disease, and sustained decrease of 50% or more in eGFR.

Further therapeutic success can be found in the (smaller) number of or in the (longer) time taken to first occurrence of any of: cardiovascular death, (non)-fatal myocardial infarction, silent MI, (non)-fatal stroke, hospitalisation for unstable angina pectoris, hospitalisation for coronary revascularization, hospitalisation for peripheral revascularization, hospitalisation for congestive heart failure, all cause mortality, renal death, sustained end-stage renal disease, loss in eGFR, new incidence of macroalbuminuria, progression in albuminuria, progression in CKD, need for anti-retinopathy therapy; or improvement in albuminuria, renal function, CKD; or improvement in cognitive function or prevention of/protection against accelerated cognitive decline.

Cognitive functions can be assessed by standardized tests as measure of cognitive functioning such as e.g. by using the Mini-Mental State Examination (MMSE), the Trail Making Test (TMT) and/or the Verbal Fluency Test (VFT).

Additional therapeutic success (compared to placebo) can be found in greater change from baseline in HbA1c and/or FPG.

Further additional therapeutic success can be found in greater proportion of patients on study treatment at study end maintain glycemic control (e.g. HbA1c </= 7%).

Further additional therapeutic success can be found in greater proportion of patients on study treatment who at study end maintain glycemic control without need for additional antidiabetic medication (during treatment) to obtain HbA1c </= 7%.

Further additional therapeutic success can be found in lower proportion of patients on study treatment initiated on insulin or treated with insulin or in lower dose of insulin dose used. Further additional therapeutic success can be found in lower change from baseline in body weight or greater proportion of patients with ≤ 2% weight gain or lower proportion of patients with ≥ 2% weight gain at study end.

## Claims

1. Linagliptin, or a pharmaceutically acceptable salt thereof, optionally in combination with one or more other active agents, for use in therapy of patients having or being at-risk of cardiovascular and/or renal microvascular disease, such as e.g. patients at high vascular risk.

2. Linagliptin, or a pharmaceutically acceptable salt thereof, optionally in combination with one or more other active agents, for use according to claim 1, wherein the therapy is both cardioprotective and renoprotective therapy.

3. Linagliptin, optionally in combination with one or more other active agents, for use according to claim 1 or 2, wherein the therapy includes preventing, protecting against, delaying the occurrence of, delaying the progression of and/or reducing the risk of at least one selected from: CV morbidity, premature CV mortality, renal morbidity and premature renal mortality.

4. Linagliptin, optionally in combination with one or more other active agents, for use according to claim 1, 2 or 3, wherein the therapy includes:
preventing, protecting against, reducing the risk of and/or delaying the occurrence of:
a cardio- or cerebrovascular disease or event, such as e.g. selected from the group consisting of: cardiovascular (CV) death (including fatal stroke, fatal myocardial infarction, fatal heart failure, cardiogenic shock and/or sudden death), non-fatal stroke (hemorrhagic or non-hemorrhagic) and non-fatal myocardial infarction (MI) (with or without silent MI) and, optionally, hospitalisation (e.g. for unstable angina pectoris, stable angina pectoris, transient ischemic attack, coronary revascularization procedures, peripheral revascularization or congestive heart failure),
and/or
preventing, protecting against, reducing the risk of, delaying the progression of and/or delaying the occurrence of:
a renal microvascular disease, such as e.g. selected from the group consisting of:
albuminuria (e.g. micro- or macro-albuminuria), chronic kidney disease (CKD), renal impairment, renal death, end-stage renal disease and loss in estimated glomerular filtration rate (e.g. eGFR ≥ 50% from baseline).

5. Linagliptin, optionally in combination with one or more other active agents, for use according to claim 1, 2, 3 or 4, wherein the therapy includes a combined method of:
preventing a cardio- or cerebrovascular disease or event selected from: cardiovascular (CV) death (including fatal stroke, fatal myocardial infarction and sudden death), non-fatal stroke, non-fatal myocardial infarction (MI) (silent MI may be excluded) and, optionally,
hospitalisation for unstable angina pectoris,
and
preventing a renal microvascular disease or event selected from: renal death, end-stage renal disease and loss in estimated glomerular filtration rate (e.g. eGFR ≥ 50% from baseline).

6. Linagliptin, optionally in combination with one or more other active agents, for use according to any one of claims 1 to 5, wherein the therapy includes preventing, protecting against, delaying the occurrence of, delaying the progression of and/or reducing the risk of accelerated cognitive decline or impairment, dementia, and/or depressive, mood or anxiety disorders.

7. Linagliptin, optionally in combination with one or more other active agents, for use according to any one of claims 1 to 6, wherein the therapy includes treatment of diabetes, especially type 2 diabetes mellitus, and/or diabetic nephropathy and/or (micro- or macro-)albuminuria.

8. Linagliptin, optionally in combination with one or more other active agents, for use according to any one of claims 1 to 7, wherein the patients have
(a) a microvascular disease (e.g. retinopathy, neuropathy, or renal microvascular disease such as nephropathy, albuminuria (e.g. micro- or macro-albuminuria), proteinuria, chronic kidney disease (e.g. CKD stage 1, 2, 3, 4 or 5) and/or renal impairment (e.g. mild, moderate or severe renal impairment or end-stage renal disease (ESRD)),
and/or
(b) a (previous) macrovascular (e.g. cardio- or cerebrovascular) disease (such as e.g. myocardial infarction, coronary artery disease, (ischemic or haemorrhagic) stroke, carotid artery disease and/or peripheral artery disease).

9. Linagliptin, optionally in combination with one or more other active agents, for use according to any one of claims 1 to 8, wherein the patients have:
nephropathy or chronic kidney disease (e.g. CKD stage 1, 2, 3, 4 or 5, particularly CKD 3 to 5), including e.g. albuminuria (e.g. micro- or macro-albuminuria, proteinuria, particularly with macro-albuminuria) and/or renal impairment (e.g. mild, moderate or severe renal impairment or end-stage renal disease (ESRD), particularly of moderate, severe or ESRD stage and/or particularly with albuminuria, more particularly with macro-albuminuria);
with or without a (previous) macrovascular (e.g. cardio- or cerebrovascular) disease (such as e.g. myocardial infarction, coronary artery disease, (ischemic or haemorrhagic) stroke, carotid artery disease and/or peripheral artery disease).

10. Linagliptin, optionally in combination with one or more other active agents, for use according to any one of claims 1 to 9, wherein the patients have:
both
albuminuria (e.g. micro- or macro-albuminuria)
and
previous macrovascular (e.g. cardio- or cerebrovascular) disease (such as e.g. myocardial infarction, coronary artery disease, (ischemic or haemorrhagic) stroke, carotid artery disease and/or peripheral artery disease);
and/or
either
(mild or moderate) renal impairment (e.g. CKD stage 1, 2 or 3, such as CKD stage 1, 2 (mild) or 3a (mild-moderate), preferably eGFR ≥ 45-75 mL/min/1.73 m²) with macro-albuminuria,
or
(moderate or severe) renal impairment (e.g. CKD stage 3 or 4, such as CKD stage 3b (moderate-severe) or 4 (severe), preferably eGFR 15-45 mL/min/1.73 m²), with or without any albuminuria (such as e.g. with or without micro- or macro-albuminuria).

11. Linagliptin, optionally in combination with one or more other active agents, for use according to any one of claims 1 to 10, wherein the patients have:
(i) albuminuria (micro or macro) (such as e.g. urine albumin creatinine ratio (UACR) ≥ 30 mg/g creatinine or ≥ 30 mg/l (milligram albumin per liter of urine) or ≥ 30 µg/min (microgram albumin per minute) or ≥ 30 mg/24 h (milligram albumin per 24 hours)) and
previous macrovascular disease, such as e.g. defined as one or more of a) to f):
a) previous myocardial infarction,
b) advanced coronary artery disease,
c) high-risk single-vessel coronary artery disease,
d) previous ischemic or haemorrhagic stroke,
e) presence of carotid artery disease,
f) presence of peripheral artery disease;
and/or
(ii) impaired renal function (e.g. with or without CV co-morbidities), such as e.g. defined by:
• impaired renal function with an eGFR 15-45 mL/min/1.73 m² with any urine albumin creatinine ratio (UACR), or
• impaired renal function with an eGFR ≥ 45-75 mL/min/1.73 m² with an urine albumin creatinine ratio (UACR) > 200 mg/g creatinine or > 200 mg/l (milligram albumin per liter of urine) or > 200 µg/min (microgram albumin per minute) or > 200 mg/24 h (milligram albumin per 24 hours).

12. Linagliptin, optionally in combination with one or more other active agents, for use according to any one of claims 1 to 11, wherein the patients are diabetic patients, particularly type 2 diabetes patients.

13. Linagliptin, in combination with one or more other active agents, for use according to any one of claims 1 to 12, wherein the one or more other active agents are selected from: other antidiabetic substances, active substances that lower the blood sugar level, active substances that lower the lipid level in the blood, active substances that raise the HDL level in the blood, active substances that lower blood pressure, active substances that are indicated in the treatment of atherosclerosis or obesity, and/or active substances which are indicated in the treatment or prevention of major CV events and/or antiplatelet agents and/or anticoagulants;
and/or wherein the one or more other active agents are selected from: other antidiabetic agents and/or antihypertensive agents;
and/or wherein the other active agents include antidiabetic agents selected from metformin, repaglinide, nateglinide, sulphonylureas, pioglitazone, alpha-glucosidase blockers and insulins;
particularly
Linagliptin, in combination with metformin, a sulphonylurea and/or an insulin, for use according to any one of claims 1 to 12, or
Linagliptin, in combination with metformin, for use according to any one of claims 1 to 12, or
Linagliptin, in combination with an insulin, for use according to any one of claims 1 to 12.

14. Linagliptin, in combination with a diuretic, an angiotensin-converting enzyme (ACE) inhibitor and/or an angiotensin II receptor blocker (ARB), such as e.g. telmisartan, for use according to any one of claims 1 to 13.

15. Linagliptin, optionally in combination with one or more other active agents, for use according to any one of the claims 1 to 14, wherein the patients are non-diabetic or diabetic patients, such as e.g. LADA or, preferably, type 2 diabetes patients.

16. Linagliptin, optionally in combination with one or more other active agents, for use according to any one of the claims 1 to 15, wherein the patients are diabetes (preferably type 2 diabetes) patients, who are naive or pre-treated with one or more conventional antidiabetic agents, such as e.g. patients with insufficient glycemic control by diet and exercise alone, or patients with insufficient glycemic control by diet and exercise plus despite therapy with one or more conventional antidiabetic agents.

17. Linagliptin, optionally in combination with one or more other active agents, for use according to any one of the claims 1 to 16, wherein the patients are (pre-treated) diabetes (preferably type 2 diabetes) patients with insufficient glycemic control despite therapy with one or two conventional antidiabetic agents selected from the group consisting of metformin, thiazolidinediones (particularly pioglitazone), sulphonylureas, glinides, α-glucosidase inhibitors (e.g. acarbose, voglibose), and insulin or insulin analogues, preferably wherein linagliptin is used in add-on combination therapy with said one or two conventional antidiabetic agents.

18. Linagliptin, optionally in combination with one or more other active agents, for use according to any one of the claims 1 to 16, wherein the patients are (naive) diabetes (preferably type 2 diabetes) patients with insufficient glycemic control by diet and exercise alone, preferably wherein linagliptin is used in monotherapy, or in initial combination therapy with a conventional antidiabetic agent selected from the group consisting of metformin, thiazolidinediones (particularly pioglitazone), sulphonylureas, glinides, α-glucosidase inhibitors (e.g. acarbose, voglibose), and insulin or insulin analogues.

19. Linagliptin, optionally in combination with one or more other active agents, for use according to any one of the claims 1 to 18, wherein the patients are on metformin background therapy.

20. Linagliptin, optionally in combination with one or more other active agents, for use according to any one of the claims 1 to 19, wherein the patient is a diabetes (particularly type 2 diabetes) patient whose diabetes is in advanced stage and/or whose diabetes is associated with renal disease or renal impairment.

21. Linagliptin, optionally in combination with one or more other active agents, for use according to any one of claims 1 to 20, wherein the patients are treated with linagliptin, optionally in combination with one or more other active agents, over a lengthy period, which is at least 1-6 years, >/= 2 years, or 3-7 years such as 3-4 years, 3-5 years, 3-6 years, 4-5 years, 4-6 years, 5-6 years or 5-7 years, preferably at least 48 months, more preferably at least 3 years.
